# EUROPEAN PATENT APPLICATION

(11) **EP 2 390 254 A1**
(43) Date of publication of application: **30.11.2011**
(21) Application number: 10733551.5
(22) Date of filing: 22.01.2010
(51) Int. Cl.: C07D 487/04, A61K 31/519, A61P 3/00, A61P 3/04, A61P 3/06, A61P 3/10, A61P 9/00, A61P 9/10, A61P 9/12, A61P 43/00

(54) **NOVEL PYRROLOÝ2,3-d¨PYRIMIDINE COMPOUND**

(30) Priority: 22.01.2009 JP 2009011809
(71) Applicant: Mitsubishi Tanabe Pharma Corporation, Osaka-shi Osaka 541-8505 (JP)
(72) Inventor: TSUBOI, Yasunori, Osaka-shi Osaka 541-8505 (JP); SHIRAI, Kimihiro, Osaka-shi Osaka 541-8505 (JP)
(74) Representative: Wiedemann, Peter
(86) International application number: PCT/JP2010/050767
(87) International publication number: WO 2010/084944

(57) **Abstract**

Disclosed is a novel pyrrolo[2,3-d]pyrimidine compound represented by formula [I] or a pharmacologically acceptable salt thereof, which has a GPR119 receptor agonistic activity and is useful for a pharmaceutical. In formula [I], E represents a group represented by formula: -NH-, or the like; ring A represents a 6-membered aromatic ring which may contain 1 to 2 nitrogen atoms as heteroatoms (the aromatic ring may be substituted by a halogen atom, a group represented by formula: -CONR^{a}R^{b}, or the like; R^{a} and R^{b} are the same or different and independently represent hydrogen, alkyl, hydroxyalkyl, or the like); R¹ represents an acyl group or the like; and R² represents a halogen atom or a cyano group.

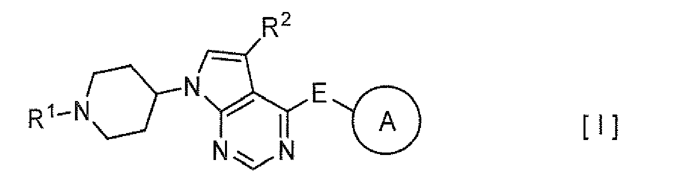

## Description

### TECHNICAL FIELD

The present invention relates to a novel pyrrolo[2,3-d]pyrimidine compound or a pharmaceutically acceptable salt thereof which shows an excellent GPR119 receptor agonistic activity and is useful as a medicament.

### BACKGROUND ART

It has been reported that a G protein-coupled receptor (GPCR) GPR119 is highly expressed in pancreatic insulin-producing β cells and intestinal cells and is activated by a compound such as oleoylethanolamide (OEA) which belongs to a natural long-chain fatty acid amide, PSN632408 which belongs to a low-molecular synthetic ligand, etc., as well as that the activation of the receptor has caused an observation of inhibitory effects on food-intake and body weight-gain in high-fat diet fed rats (Nonpatent Document 1).

Further, recent studies of physiological roles of GPR119 using a selective low-molecular agonist (AR231453) of the following formula has revealed that glucose-dependent insulin release is enhanced in pancreatic β cells via cAMP increases (adenylate cyclase activation) by the activation of the receptor, and glucose homeostasis may be improved thereby (Nonpatent Document 2).

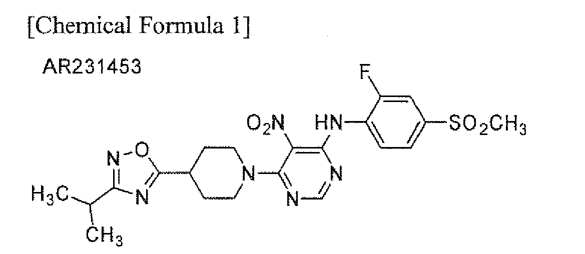

Additionally, it has been believed that the receptor modulates glucose homeostasis via enhancement of release of incretins (glucagon-like peptide-1/GLP-1 and glucose-dependent insulinotropic peptide/GIP) which are so-called endogenous antidiabetic hormone (Nonpatent Document 3). Furthermore, low-molecular GPR119 agonists may be expected to have direct and/or indirect pancreatic protective effects (antiapoptotic effects and/or growth-stimulating effects on islet cells) via incretin hormones. In view of the above knowledge, GPR119 has been focused as an attractive therapeutic target on metabolic diseases including diabetes and obesity.

Currently, bipiperidinyl compound (Patent Document 1), 1H-pyrazolo[3,4-d]pyrimidin-4-yloxypiperidine compound (Patent Document 2), 6,7-dihydro-5H-pyrrolo[2,3-d]pyrimidin-4-yloxy-1-piperidine compound, 2,3-dihydro-1-indol-4-yloxy-1-piperidine compound (Patent Document 3), 4-(benzo[b][1,4]oxazn-4(3H)-yl)piperdine compound (Patent Document 4), [1,2,3]triazolo[4,5-c]pyrimidine (Patent Document 5), etc. have been known as a GPR119 agonist except for the above OEA, PSN632408 and AR231453, but it has not been reported yet that pyrrolo[2,3-d]pyrimidine compounds of the present invention have an agonistic activity against GPR119.

- [Patent Document 1]: WO 2008/076243 pamphlet
- [Patent Document 2]: WO 2005/007658 pamphlet
- [Patent Document 3]: WO 2008/008895 pamphlet
- [Patent Document 4]: WO 2008/137435 pamphlet
- [Patent Document 5]: WO 2008/137436 pamphlet

- [Nonpatent Document 1]: Cell Metabolism 3:167-175 (2006)
- [Nonpatent Document 2]: Endocrinology 149 (5):2035-2037 (2008)
- [Nonpatent Document 3]: Endocrinology 149 (5):2038-2047 (2008)

### DISCLOSURE OF INVENTION

### PROBLEMS TO BE RESOLVED BY INVENTION

The present invention is directed to provide a novel pyrrolo[2,3-d]pyrimidine compound which shows an excellent GPR119 receptor agonistic activity and is useful as a medicament.

### MEANS OF SOLVING PROBLEMS

The present invention relates to a compound of the following general formula [I]: 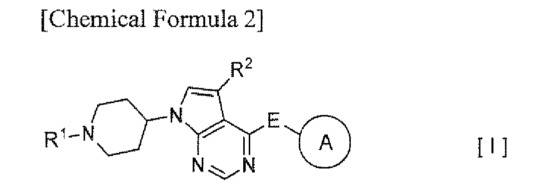 wherein E is a group of formula: -NH-, -O-, -C(=O)-, -CH(OH)- or -CF₂-,
Ring A is 6-membered aromatic ring optionally containing 1 to 2 nitrogen atoms as heteroatoms wherein the 6-membered aromatic ring may be optionally substituted by I to 3 groups selected from a) a halogen atom, b) cyano, c) alkylsulfonyl, d) alkyl optionally substituted by 1 to 3 halogen atoms, e) a group of formula: -CONR^{a}R^{b} and f) 5 to 6-membered heteroaryl containing the same or different 1 to 4 heteroatoms selected from the group consisting of nitrogen, oxygen and sulfur atoms,
R^{a} and R^{b} are the same or different and each hydrogen, alkyl, monohydroxyalkyl or alkoxyalkyl, or both combine each other together with the adjacent nitrogen atom to form 3 to 7-membered nitrogen-containing aliphatic heterocycle which may further contain heteroatoms selected from oxygen and sulfur atoms and may be optionally substituted by 1 to 2 hydroxyl,
R¹ is
a) acyl of R¹¹OCO- wherein R¹¹ is alkyl optionally substituted by 1 to 3 halogen atoms or cyanoalkyl,
b) 5 to 6-membered heteroaryl which contains the same or different 1 to 4 heteroatoms selected from the group consisting of nitrogen, oxygen and sulfur atoms wherein the heteroaryl may be optionally substituted by 1 to 3 groups selected from a halogen atom, alkyl optionally substituted by 1 to 3 halogen atoms, cycloalkyl, alkoxyalkyl, cycloalkylalkyl, alkoxy optionally substituted by 1 to 3 halogen atoms, alkoxycarbonyl and a group of formula: -CONR^{c}R^{d} wherein both R^{c} and R^{d} combine each other to form 3 to 7-membered nitrogen-containing aliphatic heterocycle optionally substituted by 1 to 2 halogen atoms, or
c) aryl (or nitrogen-containing heteroaryl)-alkyl,
R² is a halogen atom, cyano or alkoxycarbonyl; or a pharmaceutically acceptable salt thereof.

The present invention also relates to a pharmaceutical composition comprising as the active ingredient the above compound [I] or a pharmaceutically acceptable salt thereof. Further, the present invention relates to a GPR119 modulator comprising as the active ingredient the above compound [I] or a pharmaceutically acceptable salt thereof, particularly a GPR119 agonist.

### EFFECT OF INVENTION

The present compound is a compound with an excellent modulating effect including an agonistic effect on GPR119 activity, which is characterized by showing few adverse effects and has high safety as a medicament. For example, as seen in the following Experiments, the present compound is useful as a GPR119 agonist due to its excellent cAMP production-enhancing effect on human GPR119-expressed CHO cells in an assay system of said cells. Further, the present compound, a low-molecular GPR119 agonist, may be expected to have direct and/or indirect pancreatic protective effects (antiapoptotic effects and/or growth-stimulating effects on islet cells) via incretin hormones.

### BEST MODE FOR CARRYING OUT THE INVENTION

In the present compound [I], 6-membered aromatic ring of Ring A which may optionally contain 1 to 2 nitrogen atoms as heteroatoms includes benzene, pyridine or pyrimidine ring. Among them, benzene or pyridine ring is preferable.

In case that a substituent on Ring A is a group of formula: -CONR^{a}R^{b} and R^{a} and R^{b} combine each other together with the adjacent nitrogen atom to form 3 to 7-membered nitrogen-containing aliphatic heterocycle, the 3 to 7-membered nitrogen-containing aliphatic heterocycle includes azetidyl, azacyclopropyl, pyrrolidinyl, piperidino, piperazino, morpholino, thiomorpholino, thiopyrrolidinyl, azacycloheptyl, etc. Among them, pyrrolidinyl, piperidino or thiomorpholino is preferable.

In case that a substituent on Ring A is 5 to 6-membered heteroaryl containing the same or different 1 to 4 heteroatoms selected from the group consisting of nitrogen, oxygen and sulfur atoms, the 5 to 6-membered heteroaryl includes pyrrolyl, furyl, thienyl, imidazolyl, pyrazolyl, triazolyl, oxazolyl, isoxazolyl, thiazolyl, isothiazolyl, oxadiazolyl, thiadiazolyl, tetrazolyl, pyridyl, pyrimidinyl, pyrazinyl, pyridazinyl, triazinyl, etc. Among them, nitrogen-containing 5-membered heteroaryl such as tetrazolyl or triazinyl is preferable.

In case that R¹ is 5 to 6-membered heteroaryl containing the same or different 1 to 4 heteroatoms selected from the group consisting of nitrogen, oxygen and sulfur atoms, the heteroaryl includes pyrrolyl, imidazolyl, pyrazolyl, triazolyl, tetrazolyl, isothiazolyl, isoxazolyl, thiazolyl, oxadiazolyl, furazanyl, thiadiazolyl, thienyl, furyl, pyridyl, pyrimidinyl, pyrazinyl, pyridazinyl, triazinyl, etc. Among them, oxadiazolyl, thiadiazolyl, tetrazolyl, pyridyl or pyrimidinyl is preferable.

In case that R¹ is aryl-alkyl, the aryl ring moiety of the group includes benzene ring. Also, in case that R¹ is nitrogen-containing heteroaryl-alkyl, the nitrogen-containing heteroaryl ring moiety of the group includes 5 to 6-membered nitrogen-containing heteroaryl ring containing 1 to 2 nitrogen atoms as heteroatoms, more specifically pyrrole, imidazole, oxazole, pyridine or pyrimidine, etc. Among them, pyridine ring is preferable.

In case that a group of formula: -NR^{c}R^{d} is 3 to 7-membered nitrogen-containing aliphatic heterocycle, the 3 to 7-membered nitrogen-containing aliphatic heterocycle includes azetidyl, azacyclopropyl, pyrrolidinyl, piperidino, piperazino, morpholino, azacycloheptyl, etc.

### Among them, azetidyl is preferable.

The present invention encompasses the following embodiments as one embodiment in the general formula [I]:
(1) A compound, wherein E is a group of formula: -NH-, Ring A is (i) benzene ring substituted by 1 to 3 groups selected from (a) a halogen atom, (b) cyano, (c) alkylsulfonyl, (d) a group of formula: -CONR^{a}R^{b} and (e) 5-membered heteroaryl which contains the same or different 1 to 3 heteroatoms selected from the group consisting of nitrogen, oxygen and sulfur atoms, or (ii) pyridine ring substituted by 1 to 2 groups selected from the group consisting of
   (a) a halogen atom and (b) a group of formula: -CONR^{a}R^{b}, R^{a} and R^{b} are the same or different and each hydrogen, alkyl, monohydroxyalkyl or alkoxyalkyl, or both R^{a} and R^{b} combine each other together with the adjacent nitrogen atom to form 4 to 6-membered nitrogen-containing aliphatic heterocycle which may further contain heteroatoms selected from oxygen and sulfur atoms and may be optionally substituted by 1 to 2 hydroxyl, R¹ is a) acyl group of R¹¹OCO-, b) 5 to 6-membered heteroaryl which contains the same or different 1 to 4 heteroatoms selected from nitrogen, sulfur and oxygen atoms and is substituted by a halogen atom, alkyl, alkyl substituted by 1 to 3 halogen atoms, cyanoalkyl, cycloalkyl, alkoxy optionally substituted by 1 to 3 halogen atoms, alkoxycarbonyl or a group of formula: -CONR^{c}R^{d}, or c) nitrogen-containing 6-membered heteroaryl-alkyl;

(2) A compound, wherein E is a group of formula: -O-, Ring A is benzene ring substituted by 1 to 3 groups selected from a) a halogen atom, b) cyano, c) alkylsulfonyl, d) a group of formula: -CONR^{a}R^{b} and e) 5 to 6-membered heteroaryl containing 1 to 4 nitrogen atoms, R^{a} and R^{b} are the same or different and each hydrogen, alkyl or monohydroxyalkyl, R¹ is a) acyl group of R¹¹OCO- or b) 5 to 6-membered heteroaryl which contains 1 to 3 heteroatoms selected from nitrogen and oxygen atoms and is substituted by alkyl;

(3) A compound, wherein E is a group of formula: -C(=O)-, Ring A is benzene ring substituted by 1 to 3 groups selected from a) a halogen atom and b) alkylsulfonyl, R¹ is acyl group of R¹¹OCO-;

(4) A compound, wherein E is a group of formula: -CH(OH)-, Ring A is benzene ring substituted by 1 to 3 groups selected from a) a halogen atom and b) alkylsulfonyl, R¹ is acyl group of R¹¹OCO-;

(5) A compound, wherein E is a group of formula: -CF₂-, Ring A is benzene ring substituted by 1 to 3 groups selected from a) a halogen atom and b) alkylsulfonyl, R¹ is acyl group of R¹¹OCO-;

Among the compounds of the present invention, a preferable compound includes a compound of the general formula [I], wherein E is a group of formula: -NH- or -O-, Ring A is benzene ring substituted by 1 to 3 groups selected from the group consisting of a) a halogen atom, b) cyano, c) alkylsulfonyl, d) a group of formula: -CONR^{a}R^{b}, wherein R^{a} and R^{b} are the same or different and each hydrogen, alkyl or monohydroxyalkyl, or both R^{a} and R^{b} combine each other together with the adjacent nitrogen atom to form 5 to 6-membered aliphatic nitrogen-containing heterocycle in which the heterocycle may further contain sulfur atom as heteroatoms and may be optionally substituted by 1 to 2 hydroxyl, and e) 5 to 6-membered heteroaryl which contains the same or different 1 to 3 heteroatoms selected from the group consisting of nitrogen, oxygen and sulfur atoms, R¹ is a) alkoxycarbonyl or b) 5 to 6-membered heteroaryl which contains 1 to 3 heteroatoms selected from nitrogen and oxygen atoms and is substituted by a halogen atom, alkyl, dihalogenoalkyl, trihalogenoalkyl, cycloalkyl, alkoxy or dihalogenoalkoxy, and R² is a halogen atom. Among them, more preferable compound includes a compound wherein E is a group of formula: -NH-.

Among the compounds of the present invention, even more preferable compound includes a compound of the following formula [I-A]: 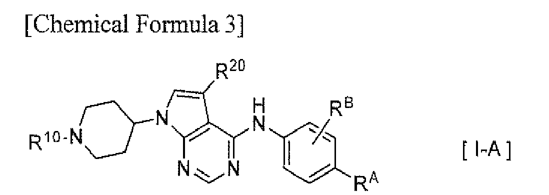 wherein R^{A} is a) a group of -CONR^{e}R^{f} wherein R^{e} and R^{f} are the same or different and each hydrogen, alkyl or monohydroxyalkyl or both combine each other together with the adjacent nitrogen atom to form 5 to 6-membered aliphatic nitrogen-containing heterocycle which may further contain sulfur atom as heteroatoms and may be optionally substituted by 1 to 2 hydroxyl, or b) 5-membered heteroaryl containing 1 to 3 nitrogen atoms as heteroatoms, R^{B} is a halogen atom, R¹⁰ is a) alkoxycarbonyl or b) 5 to 6-membered heteroaryl which contains 1 to 3 heteroatoms selected from nitrogen and oxygen atoms and is substituted by a halogen atom, alkyl, cycloalkyl, trihalogenoalkyl or alkoxy, R²⁰ is a halogen atom, or a pharmaceutically acceptable salt thereof.

Among the compounds of the present invention, particularly preferable compound includes a compound selected from the group consisting of:
4-[7-[1-(5-ethylpyrimidin-2-yl)piperidin-4-yl]-5-fluoro-7H-pyrrolo[2,3-d]pyrimidin-4-ylamino]-3-fluoro-N,N-dimethylbenzamide;
4-[7-[1-(5-ethylpyrimidin-2-yl)piperidin-4-yl]-5-fluoro-7H-pyrrolo[2,3-d]pyrimidin-4-ylamino]-3-chloro-N,N-dimethylbenzamide;
4-[7-[1-(5-ethylpyrimidin-2-yl)piperidin-4-yl]-5-fluoro-7H-pyrrolo[2,3-d]pyrimidn-4-ylamino]-3-fluoro-N-(2-hydroxyethyl)-N-methylbenzamide;
[4-[7-[1-(5-ethylpyrimidin-2-yl)piperidin-4-yl]-5-fluoro-7H-pyrrolo[2,3-d]pyrimidin-4-ylamino]-3-fluorophenyl]((R)-3-hydroxypyrrolidin-1-yl)methanone;
[4-[7-[1-(5-ethylpyrimidin-2-yl)piperidin-4-yl]-5-fluoro-7H-pyrrolo[2,3-d]pyrimidin-4-ylamino]-3-fluorophenyl]((S)-3-hydroxypyrrolidin-1-yl)methanone;
4-[7-[1-(5-ethylpyrimidin-2-yl)piperidin-4-yl]-5-fluoro-7H-pyrrolo[2,3-d]pyrimidin-4-ylamino]-3-fluoro-N-(3-hydroxypropyl)-N-methylbenzamide;
3-fluoro-4-[5-fluoro-7-[1-(5-isopropyl-[1,2,4]oxadiazol-3-yl)piperidin-4-yl]-7H-pyrrolo[2,3-d]pyrimidin-4-ylamino]-N-(2-hydroxyethyl)-N-methylbenzamide;
[4-[7-[1-(5-propylpyrimidin-2-yl)piperidin-4-yl]-5-fluoro-7H-pyrrolo[2,3-d]pyrimidin-4-ylamino]-3-fluorophenyl]((R)-3-hydroxypyrrolidin-1-yl)methanone;
4-[7-[1-(5-isopropylpyrimidin-2-yl)piperidin-4-yl]-5-fluoro-7H-pyrrolo[2,3-d]pyrimidin-4-ylamino]-3-fluoro-N-(2-hydroxyethyl)-N-methylbenzamide;
[4-[7-[1-(5-cyclopropylpyrimidin-2-yl)piperidin-4-yl]-5-fluoro-7H-pyrrolo[2,3-d]pyrimidin-4-ylamino]-3-fluorophenyl]((R)-3-hydroxypyrrolidin-l-yl)methanone;
4-[7-[1-(5-cyclopropylpyrimidin-2-yl)piperidin-4-yl]-5-fluoro-7H-pyrrolo[2,3-d]pyrimidin-4-ylamino]-3-fluoro-N-(2-hydroxyethyl)-N-methylbenzamide;
[4-[7-[1-(5-isopropylpyrimidin-2-yl)piperidin-4-yl]-5-fluoro-7H-pyrrolo[2,3-d]pyrimidin-4-ylamino]-3-fluorophenyl]((R)-3-hydroxypiperidin-1-yl)methanone;
4-[7-[1-(5-chloropyrimidin-2-yl)piperidin-4-yl]-5-fluoro-7H-pyrrolo[2,3-d]pyrimidin-4-ylamino]-3-fluoro-N-(2-hydroxyethyl)-N-methylbenzarmide;
4-[7-[1-(5-isopropoxypyrimidin-2-yl)piperidin-4-yl]-5-fluoro-7H-pyrrolo[2,3-d]pyrimidin-4-ylaminoi-3-fluoro-N-(2-hydroxyethyl)-N-methylbenzamide;
4-[7-[1-(5-isopropoxypyrimidin-2-yl)piperidin-4-yl]-5-fluoro-7H-pyrrolo[2,3-d]pyrimidin-4-ylamino]-3-fluoro-N-(3-hydroxypropyl)-N-methylbenzamide;
3-fluoro-4-[5-fluoro-7-[1-(3-isopropyl-[1,2,4]oxadiazol-5-yl)piperidin-4-yl]-7H-pyrrolo[2,3-d]pyrimidin-4-ylamino]-phenyl]-pyrrolidin-l-yl-methanone;
isopropyl 4-[5-fluoro-4-[2-fluoro-4-(pyrrolidine-1-carbonyl)-phenylamino]-pyrrolo[2,3-d]pyrimidin-7-yl]piperidine-1-carboxylate;
[3-fluoro-4-[5-fluoro-7-[1-(5-trifluoromethyl-[1,2,4]oxadiazol-3-yl)piperidin-4-yl]-7H-pyrrolo[2,3-d]pyrimidin-4-ylamino]-phenyl]((R)-3-hydroxypyrrolidin-1-yl)methanone; and
[4-[7-[1-(5-ethylpyrimidin-2-yl)piperidin-4-yl]-5-fluoro-7H-pyrrolo[2,3-d]pyrimidin-4-ylamino]-3-chlorophenyl]((R)-3-hydroxypyrrolidin-1-yl)methanone, or a pharmaceutically acceptable salt thereof.

The compound [I] of the present invention having asymmetric carbon atoms within its molecule may exist as multiple stereoisomers thereof including diastereoisomers and optical isomers based on the asymmetric carbon atoms. The present invention encompasses any one of the stereoisomers of the present compound, or a mixture thereof.

The compound [I] of the present invention has an excellent agonistic activity against GPR119 receptor, and hence, it is useful for the prevention and/or treatment of various diseases or conditions which may be expected to be improved by the modulation of the receptor activity, e.g., metabolic diseases including obesity, hyperglycemia, diabetes (including insulin-dependent diabetes, non-insulin dependent type-2 diabetes, or intermediate diabetes thereof) and a complication thereof, metabolic syndrome, glucose intolerance, hyperinsulinemia, hyperlipidemia, hypercholesterolemia, hypertriglyceridemia and abnormal lipid metabolism, or cardiovascular diseases including arterial sclerosis, hypertension, coronary disease, cardiac infarction, etc.

The compound [I] of the present invention or a pharmaceutically acceptable salt thereof is characterized by low toxicity and high safety as a medicament.

The compound [I] of the present invention may be used both in a free form and in a form of a pharmaceutically acceptable salt thereof in a pharmaceutical use. The pharmaceutically acceptable salt includes an inorganic acid salt such as hydrochloride, sulfate, phosphate or hydrobromide, or an organic acid salt such as acetate, trifluoroacetate, fumarate, oxalate, citrate, methanesulfonate, benzenesulfonate, tosylate or maleate, etc.

The compound [I] of the present invention or a pharmaceutically acceptable salt thereof includes an intramolecular salt and an adduct thereof, and a solvate or hydrate thereof, etc.

The compound [I] of the present invention or a pharmaceutically acceptable salt thereof may be orally or parenterally administered, and may be used as a conventional pharmaceutical formulation such as tablet, granule, capsule, powder, injection, inhalation, etc.

Doses of the compound [I] of the present invention or a pharmaceutically acceptable salt thereof vary depending on the administration method, ages, body weights or conditions of patients, but are preferably about 0.001 to 100 mg/kg, particularly about 0.01 to 10 mg/kg per day for injection and about 0.01 to 1000 mg/kg, particularly about 0.1 to 100 mg/kg per day for oral preparation.

The compound [I] of the present invention or a pharmaceutically acceptable salt thereof may be used alone or in combination with one or more other drugs depending on therapeutically targeted diseases. Such drugs include the following agents.

(a) antihypertensive agent: angiotensin-converting enzyme inhibitor (including enalapril maleate, imidapril hydrochloride), angiotensin Π receptor antagonist (including losartan potassium, candesartan cilexetil), β blocker (including atenolol, bisoprolol fumarate), α/β blocker (including carvedilol, labetalol hydrochloride), calcium antagonist (including amlodipine besylate, dilthiazem hydrochloride), α₁ blocker (including doxazosin mesylate, prazosin hydrochloride), central α₂ agonist or other centrally acting drugs (including clonidine hydrochloride, reserpine), vasodilating agent (including hydralazine hydrochloride, minoxidil), etc.;

(b) diuretic agent: thiazide diuretic agent (including chlorothiazide, hydrochlorothiazide), loop diuretic agent (including bumetanide, furosemide), potassium-sparing diuretic agent (including amiloride hydrochloride, triamterene);

(c) heart failure drug: nitrate drug (including nitroglycerin), digitalis preparation (including digoxin, digitoxin), catecholamines (including dobutamine hydrochloride, denopamine), endotherine antagonist (including bosentan), phosphodiesterase inhibitor (including milrinone lactate, amrinone), neutral endopeptidase inhibitor (including fasidotril), atrial uretic peptide, etc.;

(d) antiarrhythmic agent: Na channel blocker (including procaine amide hydrochloride, flecainide acetate), K channel blocker (including amiodarone hydrochloride), Ca channel blocker (including verapamil hydrochloride), etc.;

(e) medicament for hyperlipidemia: HMG-CoA reductase inhibitor (including pravastatin sodium, atorvastatin calcium, fluvastatin sodium), fibrate derivatives (including bezafibrate, clofibrate), squalene synthetase inhibitor, etc.;

(f) antithrombotic agent: blood coagulation inhibitor (including warfarin sodium, heparin sodium), thrombolytic agent (including urokinase, t-PA), antiplatelet agent (including aspirin, ticlopidine hydrochloride);

(g) a therapeutic agent for diabetes/diabetic complication: insulin, DPP4 inhibitor (including vildagliptin, sitagliptin), α-glucosidase inhibitor (including voglibose, acarbose, miglitol, emiglitate), biguanide (including metformin hydrochloride, buformin, phenformin), insulin resistance-improving agent (including pioglitazone, troglitazone, rosiglitazone), insulin secretagogue (including sulfonylurea compounds such as tolbutamide, glibenclamide, gliclazide, glyclopyramide, chlorpropamide, glimepiride, glybuzide, glybuzole, tolazamide, acetohexamide), amylin antagonist (including pramlintide), aldose reductase inhibitor (including epalrestat, tolrestat, zenarestat, fidarestat, minalrestat, zopolrestat), neurotrophic factor (including NGF), AGE inhibitor (including pimagedine, pyratoxatine), a neurotrophic factor production-promoting agent, etc.;

(h) antiobesity agent: central antiobesity agent (including mazindol, fenfluramine, sibutramine), pancreatic lipase inhibitor (including orlistat), β₃ agonist (including SB-226552, BMS-196085), peptidic anorexiant (including leptin), cholecystokinin receptor agonist (including lintitript), etc.;

(i) nonsteroidal anti-inflammatory agent: acetaminophen, ibuprofen, etc.;

(j) chemotherapeutic agent: metabolic antagonist (including 5-fluorouracil, methotrexate), anticancer agent (including vincristine, taxol, cisplatin), etc.;

(k) immuno-modulating agent: immunosuppressant (including cyclosporine, tacrolimus), immunopotentiating agent (including Krestin, Lentinan), cytokines (including interleukin 1, interferon), cyclooxygenase inhibitor (including indomethacin, celecoxib), anti-TNFα antibody (including infliximab), etc.

A dosage form when the compound [I] of the present invention is used in combination with other agents includes (1) a single dosage form (a fixed combination) containing the compound [I] and other agents, and (2) a concomitant administration of a drug containing the compound [I] with a drug containing other agents. In the concomitant administration (2), each drug may be administered in different administration routes and times.

The compound [I] wherein E is a group of formula: -NH- may be prepared according to the following Scheme 1 or 2, for example.

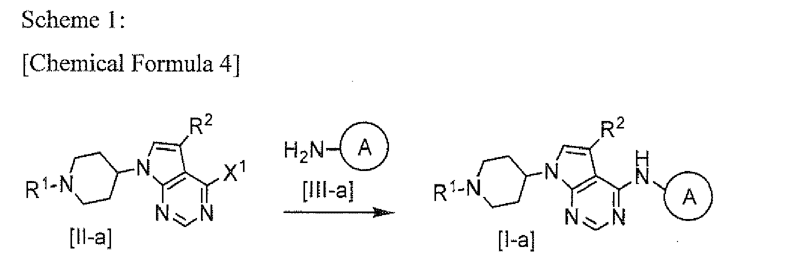

In the above scheme, X¹ is a halogen atom, and other symbols have the same meanings as defined above.

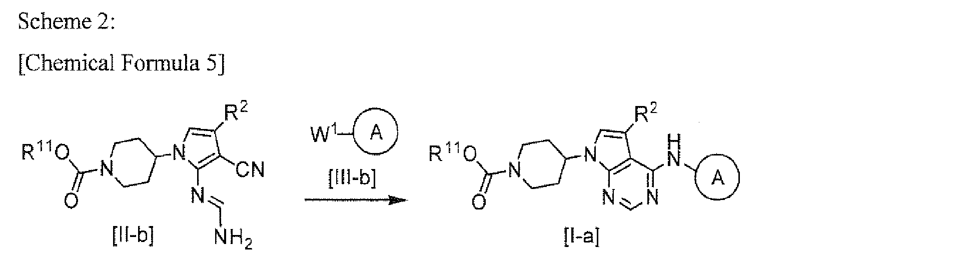

In the above scheme, W¹ is a halogen atom, and other symbols have the same meanings as defined above.

The reaction of compound [II-a] with amine compound [III-a] may be carried out in a solvent in the presence of a palladium catalyst and a base and in the presence or absence of a ligand. The solvent may be any inert solvents which do not affect the reaction, and includes ethers such as dioxane, aromatic hydrocarbons such as toluene, amides such as N,N-dimethylformamide, water, etc. The palladium catalyst includes palladium acetate, tris(dibenzylideneacetone)dipalladium, dichlorobis(triphenylphosphine)palladium, tetrakis(triphenylphosphine)palladium, [1,1'-bis(diphenylphosphino)ferrocene]palladium dichloride, etc. The ligand includes 2-(di-tert-butylphosphino)biphenyl, triphenylphosphine, 2-(di-tert-butylphosphino)-1,1'-binaphthyl, 2-dicyclohexylphosphino-2'-(N,N-dimethylamino)biphenyl, etc. The base includes sodium tert-butoxide, potassium tert-butoxide, sodium phenoxide, potassium carbonate, cesium carbonate, potassium phosphate, sodium hydrogencarbonate, lithium chloride, triethylamine, etc. A usage of the compound [III-a] is 0.9 to 3.0 equivalents, preferably 1.0 to 1.2 equivalents to compound [II-a]. A usage of the palladium catalyst is 0.01 to 0.3 equivalents, preferably 0.01 to 0.1 equivalents to compound [II-a] or compound [III-a]. A usage of the base is 1.0 to 5.0 equivalents, preferably 2.0 to 3.0 equivalents to compound [II-a] or compound [III-a]. A usage of the ligand is 0.01 to 0.3 equivalents, preferably 0.01 to 0.1 equivalents to compound [II-a] or compound [III-a]. The reaction may be carried out at 0°C to 200°C, preferably 100°C to 150°C.

The reaction of compound [II-a] with amine compound [III-a] may be also carried out in a solvent (including alcohols such as isopropanol) in the presence of an acid catalyst (including hydrochloric acid). A usage of the acid catalyst may be 0.01 to 1.0 equivalents to compound [II-a].

The reaction of compound [II-b] with compound [III-b] may be carried out in the similar manner to the above reaction of compound [II-a] with amine compound [III-a].

The compound [I] wherein E is a group of formula: -O- may be prepared according to the following Scheme 3.

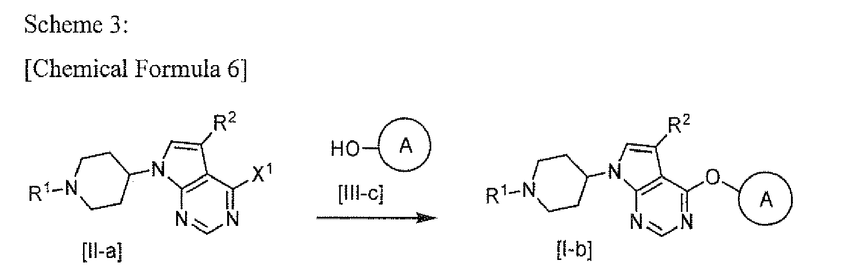

In the above scheme, symbols have the same meanings as defined above.

The reaction of compound [II-a] with compound [III-c] may be carried out in a solvent in the presence of a base. The solvent may be any inert solvents which do not affect the reaction, and includes ethers such as dimethylsulfoxide, tetrahydrofuran, amides such as N,N-dimethylformamide, ketones such as acetone, etc. The base includes potassium carbonate, cesium carbonate, sodium carbonate, sodium hydride, etc. A usage of compound [III-c] is 0.9 to 3.0 equivalents, preferably 1.0 to 1.5 equivalents, to compound [II-a]. A usage of the base is 1.0 to 5.0 equivalents, preferably 1.5 to 3.0 equivalents to compound [II-a] or compound [III-c]. The reaction may be carried out at 0°C to 200°C, preferably 60°C to 100°C.

The compound [I] wherein E is a group of formula: -C(=O)- or -CH(OH)- may be prepared according to the following Scheme 4.

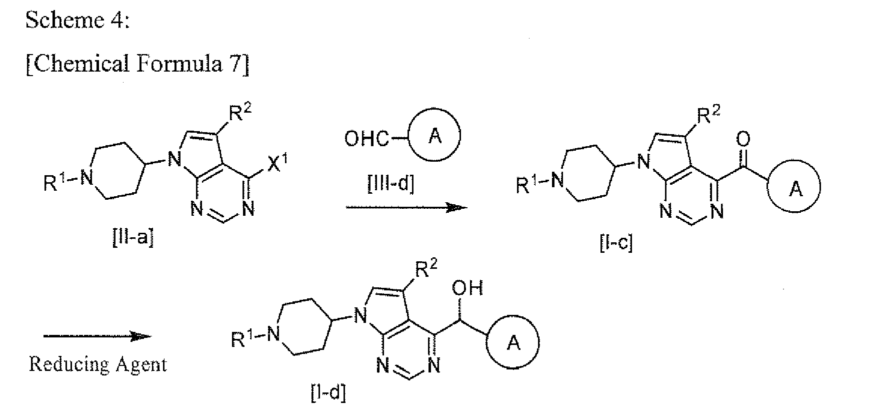

In the above scheme, symbols have the same meanings as defined above.

The reaction of compound [II-a] with compound [III-d] may be carried out in a solvent in the presence of an activating agent and a base. The solvent may be any inert solvents which do not affect the reaction, and includes ethers such as dioxane. The activating agent includes N,N-dímethylimidazolinium iodide, N,N-dimethylbenzoimidazolinium iodide, etc. The base includes sodium hydride, potassium tert-butoxide, etc. A usage of compound [III-d] is 1.0 to 10.0 equivalents, preferably 1.5 to 2.5 equivalents, to compound [II-a]. A usage of the activating agent is 0.05 to 5 equivalents, preferably 0.5 to 1.5 equivalents, to compound [II-a] or compound [III-d]. A usage of the base is 1.0 to 10.0 equivalents, preferably 2.0 to 3.0 equivalents, to compound [II-a] or compound [III-d]. The reaction may be carried out at -100 to 100°C, preferably -40 to 20°C.

The compound [I-c] may be reduced in a solvent in the presence of a reducing agent. The solvent may be any inert solvents which do not affect the reaction, and includes alcohols such as methanol, ethers such as tetrahydrofuran, etc. The reducing agent includes sodium borohydride, sodium cyanoborohydride, etc. A usage of the reducing agent is 0.25 to 10 equivalents, preferably 2.0 to 3.0 equivalents, to compound [I-c]. The reaction may be carried out at -40 to 80°C, preferably 0 to 30°C.

The compound [I] wherein R¹ is an acyl group of formula: R¹¹OCO- may be also prepared according to the following Scheme 5.

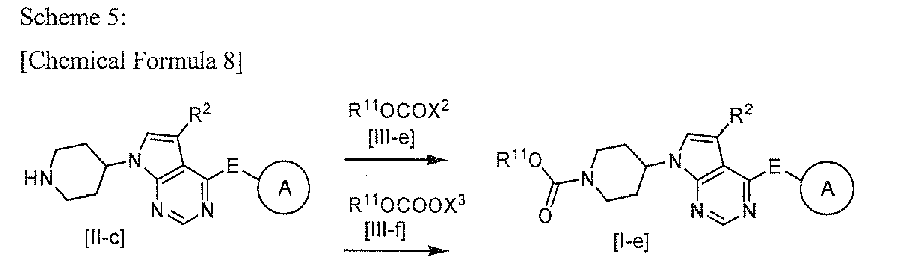

In the above scheme, X² is a halogen atom, X³ is p-nitrophenyl, and other symbols have the same meanings as defined above.

The reaction of compound [II-c] or a salt thereof (including a mineral acid salt such as hydrochloride) with compound [III-e] may be carried out in a solvent in the presence of a base. The solvent may be any inert solvents which do not affect the reaction, and includes halogenated aliphatic hydrocarbons such as dichloromethane, ethers such as tetrahydrofuran, aromatic hydrocarbons such as toluene, etc. The base includes triethylamine, diisopropylethylamine, pyridine, etc. A usage of compound [III-e] is 0.9 to 3.0 equivalents, preferably 1.0 to 1.5 equivalents, to compound [II-c]. A usage of the base is 1.0 to 5.0 equivalents, preferably 1.5 to 2.0 equivalents, to compound [II-c] or compound [III-e]. The reaction may be carried out at 0°C to 100°C, preferably 0°C to room temperature.

The reaction of compound [II-c] or a salt thereof (including a mineral acid salt such as hydrochloride) with compound [III-f] may be carried out in the similar manner to the above reaction of compound [II-c] with compound [III-e].

The compound [I] wherein R¹ is a cyclic group of formula: 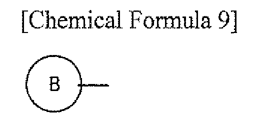 wherein Ring B is 5 to 6-membered heteroaryl containing the same or different 1 to 4 heteroatoms selected from the group consisting of nitrogen, oxygen and sulfur atoms in which the heteroaryl may be optionally substituted by 1 to 3 groups selected from alkyl, alkoxy and alkoxycarbonyl which are optionally substituted by 1 to 3 halogen atoms may be also prepared according to the following Scheme 6, for example.

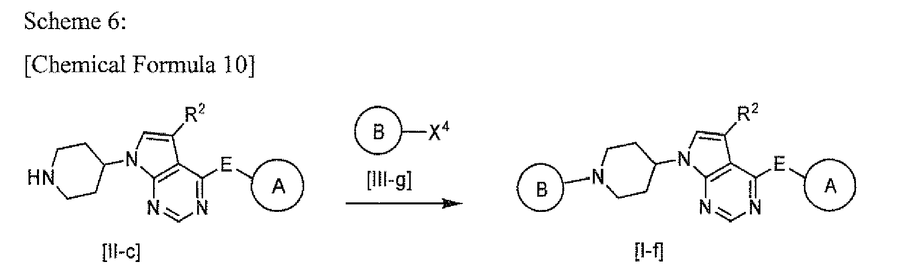

In the above scheme, X⁴ is a halogen atom or methanesulfonyl, and other symbols have the same meaningsas defined above.

The reaction of compound [II-c] or a salt thereof (including a mineral acid salt such as hydrochloride) with compound [III-g] may be carried out in a solvent in the presence or absence of a base. The solvent may be any inert solvents which do not affect the reaction, and includes amides such as dimethylformamide, ethers such as tetrahydrofuran, etc. The base includes diisopropylethylamine, triethylamine, pyridine, potassium carbonate, etc. A usage of compound [III-g] is 1.0 to 10 equivalents, preferably 1.5 to 3.0 equivalents, to compound [II-c]. A usage of the base is 1.0 to 5.0 equivalents, preferably 1.5 to 3.0 equivalents, to compound [II-c] or compound [III-g] . The reaction may be carried out at 0°C to 150°C, preferably room temperature to 80°C.

The reaction of compound [II-c] or a salt thereof with compound [III-g] may be also carried out in a solvent in the presence of a palladium catalyst and a base and in the presence or absence of an activating agent. The solvent, the palladium catalyst and the base illustrated in Scheme 1 (reaction of compound [II-a] with amine compound [III-a]) may be used in the reaction. The activating agent includes 1,3-bis(2,6-diisopropylphenyl)-4,5-dihydroimidazolium tetrafluoroborate, etc. A usage of compound [III-g] is 1.0 to 3.0 equivalents, preferably 1.0 to 1.5 equivalents, to compound [II-c]. A usage of the palladium catalyst is 0.01 to 0.3 equivalents, preferably 0.01 to 0.1 equivalents, to compound [II-c] or compound [III-g]. A usage of the base is 1.0 to 5.0 equivalents, preferably 2.0 to 4.0 equivalents, to compound [II-c] or compound [III-g]. The reaction may be carried out at 0 to 200°C, preferably 100 to 150°C.

The compound [I] wherein R¹ is a group of formula: 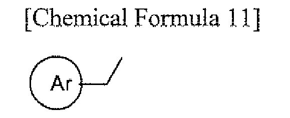 wherein Ar is aryl (or nitrogen-containing heteroaryl) may be also prepared according to the following Scheme 7, for example.

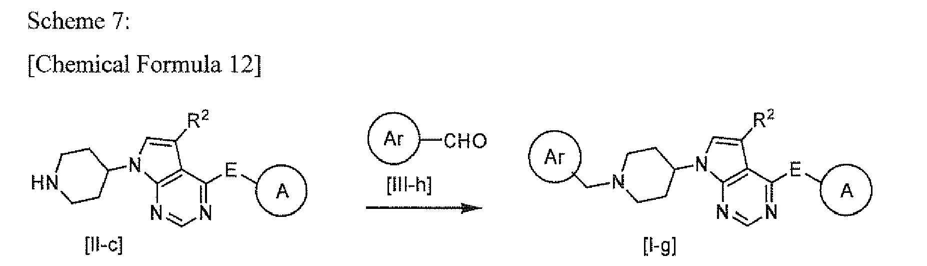

In the above scheme, symbols have the same meanings as defined above.

The reaction of compound [II-c] or a salt thereof (including a mineral acid salt such as hydrochloride) with aldehyde compound [III-h] may be carried out in a solvent in the presence of a reducing agent and a base or an acid. The solvent may be any inert solvents which do not affect the reaction, and includes halogenated aliphatic hydrocarbons such as dichloromethane, ethers such as tetrahydrofuran, alcohols such as methanol, etc. The reducing agent includes sodium triacetoxyborohydride, sodium cyanoborohydride, etc. The base includes potassium acetate, etc. The acid includes acetic acid, etc. A usage of compound [III-h] is 1.0 to 10.0 equivalents, preferably 1.5 to 2.0 equivalents, to compound [II-c]. A usage of the reducing agent is 1.0 to 10.0 equivalents, preferably 1.5 to 2.0 equivalents, to compound [II-c] or compound [III-h]. A usage of the base or the acid is 1.0 to 10.0 equivalents, preferably 1.5 to 2.0 equivalents, to compound [II-c] or compound [III-h]. The reaction may be carried out at - 40 to 80°C, preferably 0 to 30°C.

The compound [I] wherein Ring A is 6-membered aromatic cyclic group of the following formula: 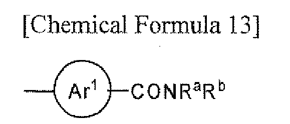 wherein the 6-membered aromatic ring Ar¹ may contain 1 to 2 nitrogen atoms as heteroatoms and may be optionally substituted by 1 to 2 groups selected from a halogen atom and cyano as well as a group of formula: -CONR^{a}R^{b} may be also prepared according to the following Scheme 8, for example.

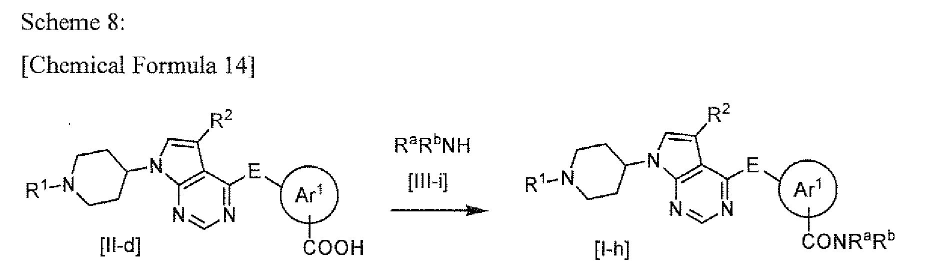

In the above scheme, symbols have the same meanings as defined above.

The reaction of compound [II-d] or a salt thereof (including a mineral acid salt such as hydrochloride) with amine compound [III-i] or a salt thereof (including a mineral acid salt such as hydrochloride) may be carried out in a solvent in the presence of a condensing agent and in the presence or absence of a base and an activating agent. The solvent may be any inert solvents which do not affect the reaction, and includes halogenated aliphatic hydrocarbons such as dichloromethane, amides such as N,N-dimethylformamide, ethers such as tetrahydrofuran, water, etc. The condensing agent includes 1-methyl-3-(3-dimethylaminopropyl)carbodiimide hydrochloride (WSC•HCl), N,N'-dicyclohexylcarbodiimide, diethyl cyanophosphonate, etc. The activating agent includes N-hydroxybenzotriazole monohydrate, N-hydroxysuccinimide, etc. The base includes triethylamine, diisopropylethylamine, pyridine, etc. A usage of compound [III-i] is 1.0 to 5.0 equivalents, preferably 1.0 to 1.5 equivalents, to compound [II-d]. A usage of the condensing agent is 1.0 to 5.0 equivalents, preferably 1.0 to 1.5 equivalents, to compound [II-d] or compound [III-i]. A usage of the activating agent is 1.0 to 5.0 equivalents, preferably 1.0 to 1.5 equivalents, to compound [II-d] or compound [III-i]. A usage of the base is 1.0 to 2.0 equivalents, preferably 1.0 to 1.2 equivalents, to compound [II-d] or compound [III-i]. The reaction may be carried out at 0°C to 100°C, preferably 0°C to 40°C.

The compound [I] wherein R² is cyano may be also prepared according to the following Scheme 9, for example.

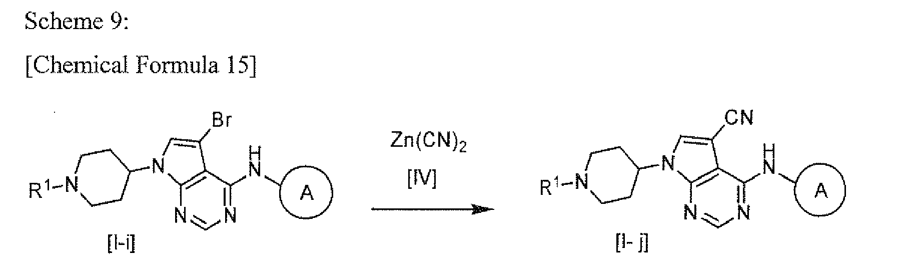

In the above scheme, symbols have the same meanings as defined above.

The reaction of compound [I-i] with zinc cyanide (compound [IV]) may be carried out in a solvent in the presence of a palladium catalyst. The solvent may be any inert solvents which do not affect the reaction, and includes amides such as dimethylformamide, aromatic hydrocarbons such as toluene, ethers such as 1,2-dimethoxyethane, etc. The palladium catalyst includes tetrakis(triphenylphosphine)palladium, [1,1'-bis(diphenylphosphino)ferrocene]palladium dichloride, tris(dibenzylideneacetone)dipalladium, etc. A usage of compound [IV] is 0.5 to 2.0 equivalents, preferably 0.6 to 1.0 equivalents, to compound [I-i]. A usage of the palladium catalyst is 0.01 to 0.5 equivalents, preferably 0.05 to 0.1 equivalents, to compound [I-i] or compound [IV]. The reaction may be carried out at room temperature to 200°C, preferably 60 to 100°C.

The compound [I] wherein E is a group of formula: -CF₂- may be prepared according to the following Scheme 10, for example.

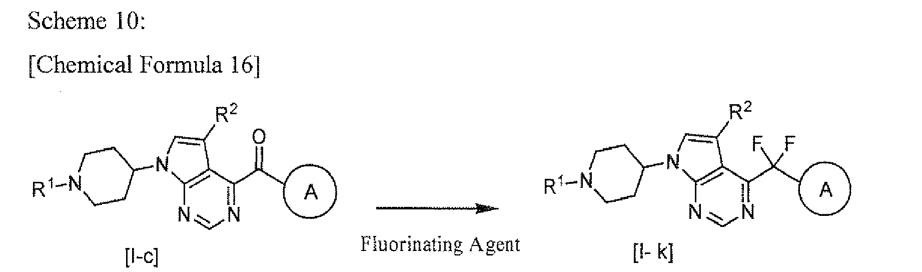

In the above scheme, symbols have the same meanings as defined above.

The reaction of compound [I-c] with a fluorinating agent may be carried out in a solvent. The solvent may be any inert solvents which do not affect the reaction, and includes halogenated aliphatic hydrocarbons such as dichloromethane, aromatic hydrocarbons such as benzene, etc. The fluorinating agent includes N,N-diethylaminosulfur trifluoride (DAST), N,N-di-(2-methoxy)ethylaminosulfur trifluoride (DEOXO-FLUOR), etc. A usage of the fluorinating agent is 1.0 to 20.0 equivalents, preferably 2.0 to 4.0 equivalents, to compound [I-c]. The reaction may be carried out at -40 to 100°C, preferably 40 to 60°C.

Among compound [I], a compound of the following formula: 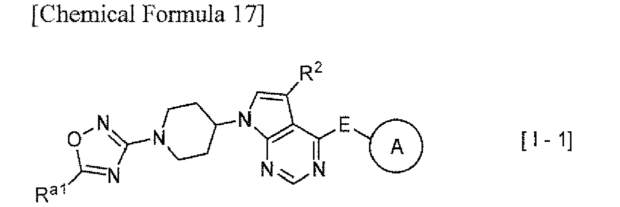 wherein R^{a1} is alkyl optionally substituted by 1 to 3 halogen atoms, cycloalkyl, alkoxyalkyl or cycloalkylalkyl and other symbols have the same meanings as defined above may be also prepared by reacting a compound of the following formula: 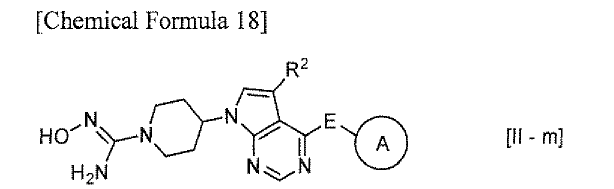 wherein symbols have the same meanings as defined above with carboxylic acid chloride: R^{a1}-COCl [a]
wherein symbols have the same meanings as defined above in a solvent (including aromatic hydrocarbons such as toluene, halogenated aliphatic hydrocarbons such as dichloromethane) in the presence of a base (including an organic amine such as triethylamine).

The compound [I] wherein R¹ comprises a group of formula: -CONR^{c}R^{d} and both R^{c} and R^{d} combine each other to form 3 to 7-membered nitrogen-containing aliphatic heterocycle optionally substituted by 1 to 2 halogen atoms may be also prepared by reacting a corresponding compound wherein R¹ comprises -COOH with a cyclic amine compound of formula:

HNR^{c}R^{d} [b]

wherein symbols have the same meanings as defined above or a salt thereof in a solvent (including halogenated aliphatic hydrocarbons such as dichloromethane) in the presence of a condensing agent (including water-soluble carbodiimide) in the presence or absence of a base (including an organic amine such as triethylamine) and an activating agent (including 1-hydroxybenzotriazole).

Among compound [I], a compound of formula [I-m]: 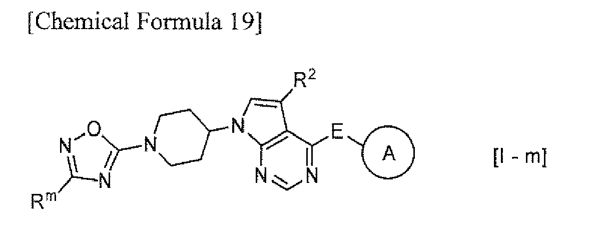 wherein R^{m} is alkyl optionally substituted by 1 to 3 halogen atoms, cycloalkyl, alkoxyalkyl or cycloalkylalkyl and other symbols have the same meanings as defined above may be also prepared by reacting a compound of the following formula [cc]: 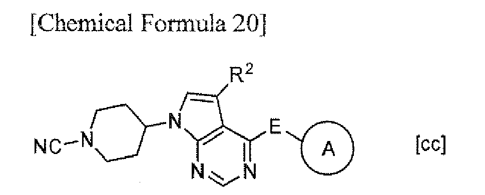 wherein symbols have the same meanings as defined above with a compound of the following formula [III-m]: 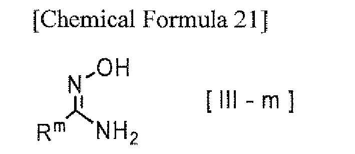 wherein symbols have the same meanings as defined above in a solvent (including amides such as dimethylformamide, nitriles such as acetonitrile, aromatic hydrocarbons such as toluene) in the presence of an acid catalyst (including a protonic acid such as p-toluenesulfonic acid, Lewis acid such as zinc chloride, or a mixture thereof). A usage of the acid catalyst may be 0.001 to 1.0 equivalents to compound [cc].

Synthetic intermediates of the present invention compound [II-a], compound [II-b], compound [II-c], compound [cc] and compound [II-d] may be prepared according to the following Scheme, for example.

The compound [II-a] wherein R² is halogen (i.e., compound [II-a1]) may be prepared according to the following Scheme 11.

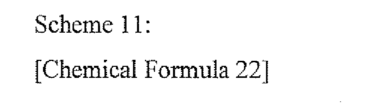

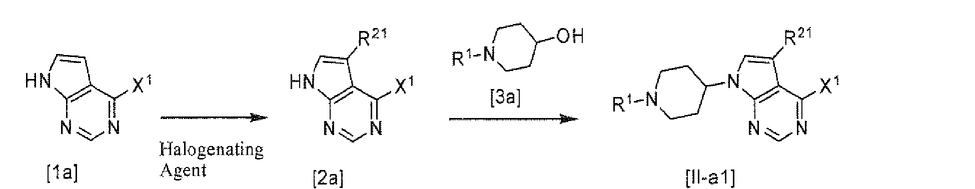

In the above scheme, R²¹ is a halogen atom, X¹ is a halogen atom, and other symbols have the same meanings as defined above.

The reaction of compound [1a] with a halogenating agent may be carried out in a solvent in the presence or absence of an acid. The solvent may be any inert solvents which do not affect the reaction, and includes nitriles such as acetonitrile, halogenated aliphatic hydrocarbons such as dichloromethane, etc. The halogenating agent includes N-fluoro-N'-(chloromethyl)triethylenediamine bis(tetrafluoroborate), N-chlorosuccinimide, N-bromosuccinimide, N-iodosuccinimide, etc. The acid includes acetic acid, etc.

The reaction of compound [2a] with compound [3a] may be carrid out in a solvent in the presence of an additive including tris-substituted phosphine such as triphenylphosphine and diethyl azodicarboxylate. The solvent may be any inert solvents which do not affect the reaction, and includes ethers such as tetrahydrofuran, aromatic hydrocarbons such as toluene, etc.

The compound [II-a] wherein R² is cyano (compound [II-a2]) may be prepared by reacting compound [II-a1] with zinc cyanide (compound [IV]). The reaction may be carried out in the similar manner to the above reaction of compound [I-i] with compound [IV] (Scheme 9).

Compound [II-b] may be prepared according to the following Scheme 12.

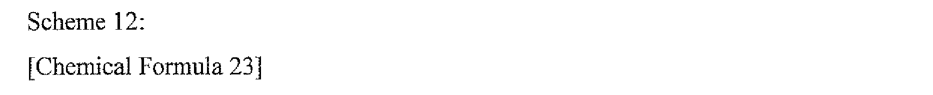

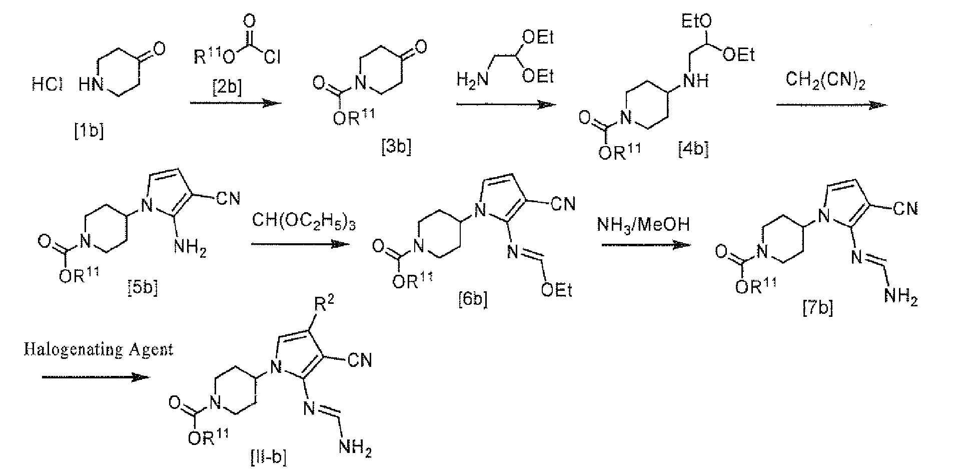

In the above scheme, symbols have the same meanings as defined above.

The reaction of compound [1b] or a salt thereof (including hydrochloride) with compound [2b] may be carried out in a solvent such as dichloromethane in the presence of a base such as triethylamine.

The reaction of compound [3b] with aminoacetaldehyde diethyl acetal may be carried out in a solvent such as dichloromethane in the presence of an acid catalyst such as acetic acid, a base such as triethylamine and boron hydride compound such as sodium triacetoxyborohydride.

The reaction of compound [4b] with malononitrile may be carried out in a solvent such as dichloromethane in the presence of an additive such as p-toluenesulfonic acid.

The reaction of compound [5b] with triethyl orthoformate may be carried out in a solvent such as acetonitrile in the presence of an acid catalyst such as acetic acid.

The conversion of compound [6b] into compound [7b] may be carried out by treating compound [6b] with ammonia in a solvent such as methanol.

The reaction of compound [7b] with a halogenating agent may be carried out in a solvent such as acetonitrile. The halogenating agent includes N-chlorosuccinimide, N-bromosuccinimide, N-iodosuccinimide, bromine, etc.

Compound [II-c] may be prepared according to the following Scheme 13.

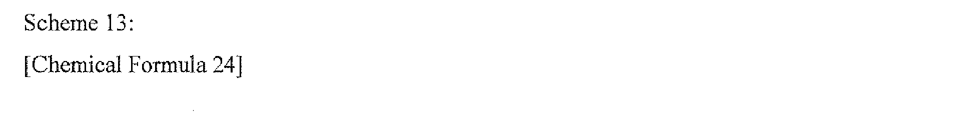

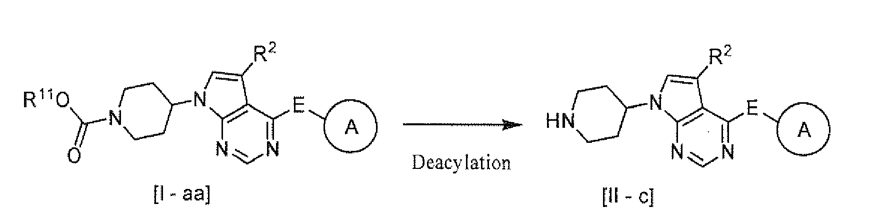

In the above scheme, symbols have the same meanings as defined above.

The deacylation of compound [I-aa] may be carried out according to a conventional method depending on types of acyl groups. For example, an acyl group may be removed from a compound [I-aa] wherein R¹¹ is tert-butoxycarbonyl by treating with hydrochloric acid/dioxane.

Compound [II-d] may be prepared according to the following Scheme 14.

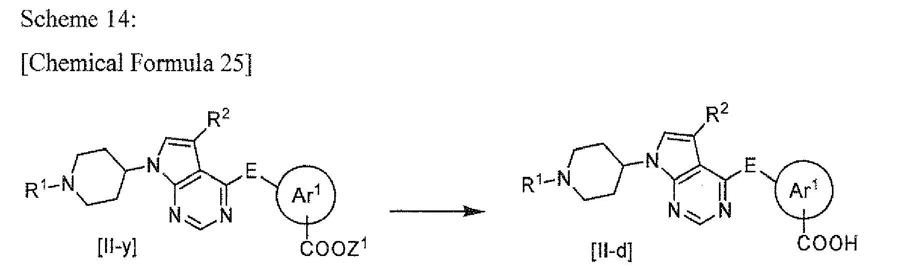

In the above scheme, Z¹ is a protective group of carboxyl, and other symbols have the same meanings as defined above.

Z¹ of compound [II-y] includes alkyl such as tert-butyl, aralkyl such as benzyl, etc. The removal of the protective group from compound [II-y] may be carried out according to a conventional method. For example, the removal of the protective group from compound [II-y] wherein Z¹ is tert-butyl may be carried out by treating the compound with hydrochloric acid/dioxane, etc. in a solvent or neat.

The intermediate compound of the present invention of the following formula: 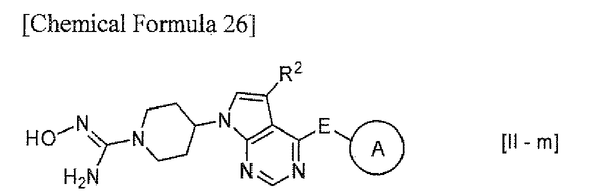 wherein symbols have the same meanings as defined above may be prepared by reacting a compound of the following formula [II-c]: 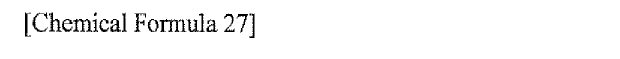 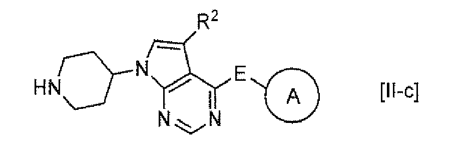 wherein symbols have the same meanings as defined above with cyanogen halide (e.g., cyanogen bromide) in a solvent (including alcohols such as ethanol, ethers such as tetrahydrofuran) in the presence of a base (including sodium hydrogencarbonate) to give a compound of the following formula [cc]: 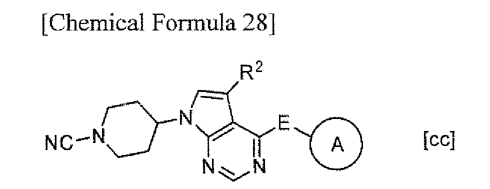 wherein symbols have the same meanings as defined above, then reacting Compound [cc] with hydroxylamine in a solvent (including alcohols such as isopropanol).

Herein, "a halogen atom" refers to fluorine atom, chlorine atom, iodine atom or bromine atom, "alkyl" or "alkoxy" refers to C₁₋₈, preferably C₁₋₆, straight- or branched-chain alkyl or alkoxy, and "cycloalkyl" refers to C₃₋₈, preferably C₃₋₆, cycloalkyl. Also, "alkylene" refers to C₁₋₈, preferably C₁₋₆, straight- or branched-chain alkylene, and "alkanoyl" refers to C₂-₈, preferably C₂₋₆, straight- or branched-chain alkanoyl.

Abbreviations used herein refer to the following meanings, unless otherwise specified.
- Ac:: acetyl
- Boc:: tert-butoxycarbonyl
- DMF:: dimethylformamide
- DMSO:: dimethylsulfoxide
- Me:: methyl
- Et:: ethyl
- i-Pr:: isopropyl
- i-Bu:: isobutyl
- t-Bu or tert-Bu:: tert-butyl
- MOMO:: methoxymethoxy
- Ph:: phenyl
- Bzl:: benzyl
- TFA:: trifluoroacetic acid
- CDI:: 1,1'-carbonyldiimidazole
- HOBt:: 1 -hydroxybenzotriazole
- DIAD:: diisopropyl azodicarboxylate
- dppf:: diphenylphosphinoferrocene
- PPh₃:: triphenylphosphine
- HPLC:: high-performance liquid chromatography

### EXAMPLES

### Example 1

Preparation of tert-butyl 4-[4-(4-dimethylcarbamoyl-2-fluorophenylamino)-5-fluoropyrrolo[2,3-d]pyrimidin-7-yl]piperidine-1-carboxylate 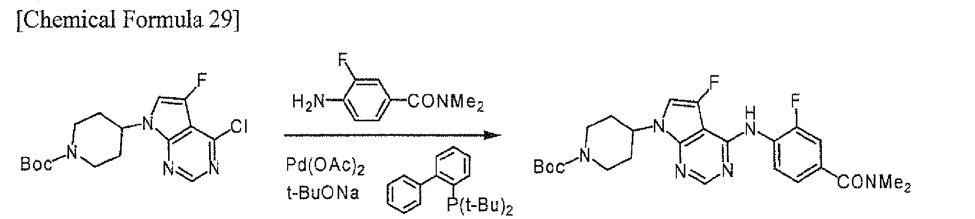 To a solution of tert-butyl 4-(4-chloro-5-fluoropyrrolo[2,3-d]pyrimidin-7-yl)piperidine-1-carboxylate (obtained in Reference Example 1; 75 mg) in 1,4-dioxane (1.5 mL) were added 4-amino-3-fluoro-N,N-dimethylbenzamide (obtained in Reference Example 2; 42.4 mg), palladium acetate (0.5 mg), 2-(di-tert-butylphosphino)biphenyl (0.7 mg) and sodium tert-butoxide (50.8 mg), and the mixture was stirred in a microwave reactor (Initiator, manufactured by Biotage Inc.) at 120°C for 20 minutes. To the reaction mixture was added ethyl acetate, and the organic layer was washed with water and then concentrated. The resulting residue was purified by column chromatography on NH silica-gel (Chromatorex; Fuji i Silysia Chemical Ltd., solvent; hexane/ethyl acetate = 80/20 to 55/45) to give the titled compound (39.0 mg) as a powder (yield 37%).
MS(APCI)m/z; 501 [M+H]⁺.

### Example 2

Preparation of isopropyl 4-[5-chloro-4-(3-chloro-5-dimethylcarbamoylpyridin-2-ylamino)pyrrolo[2,3-d]pyrimidin-7-yl]piperidine-1-carbxylate
[Chemical Formula 30] 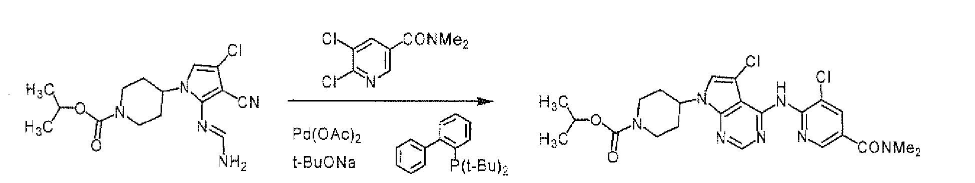 To a solution of isopropyl 4-(4-chloro-3-cyano-2-formimidoylaminopyrrol-1-yl)piperidine-1-carboxylate (obtained in Reference Example 25; 101 mg) in 1,4-dioxane (5 mL) were added 5,6-dichloro-N,N-dimethylnicotinamide (obtained in Reference Example 28; 99 mg), palladium acetate (7 mg), 2-(di-tert-butylphosphino)biphenyl (12 mg) and sodium tert-butoxide (72 mg), and the mixture was stirred in a microwave reactor (Initiator, manufactured by Biotage Inc.) at 120°c for 60 minutes. The mixture was poured into water and extracted with ethyl acetate three times. The organic layer was dried over magnesium sulfate and then concentrated. The resulting residue was purified by column chromatography on silica gel (solvent; chloroform/methanol = 100/0 to 90/10) to give the titled compound (15.5 mg) as a powder (yield 14%).
MS(APCI)m/z; 520/522[M+H]⁺.

### Example 3

Preparation of tert-butyl 4-[5-fluoro-4-(2-fluoro-4-mesylphenoxy)pyrrolo[2,3-d]pyrimidin-7-yl]piperidine-1-carboxylate 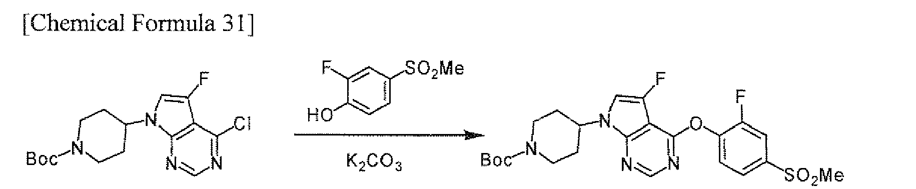 To a solution of tert-butyl 4-(4-chloro-5-fluoropyrrolo[2,3-d]pyrimidin-7-yl)-piperidine-1-carboxylate (obtained in Reference Example 1; 50 mg) in dimethylsulfoxide (1.5 mL) were added 2-fluoro-4-mesylphenol (obtained in Reference Example 27; 32 mg) and potassium carbonate (58 mg), and the mixture was stirred at 100°C for 1 hour. The reaction mixture was cooled to room temperature, and thereto was added water and the mxiture was extracted with ethyl acetate. The organic layer was concentrated, and the resulting residue was purified by column chromatography on NH silica-gel (Chromatorex; Fuji Silysia Chemical Ltd., solvent; hexane/ethyl acetate = 80/20 to 50/50) to give the titled compound (51.7 mg) as a powder (yield 72%).
MS(APCI)m/z; 509[M+H]⁺.

### Example 4

Preparation of isopropyl 4-[4-(4-dimethylcarbamoyl-2-fluorophenylamino)-5-fluoropyrrolo[2,3-d]pyrimidin-7-yl]piperidine-1-carboxylate 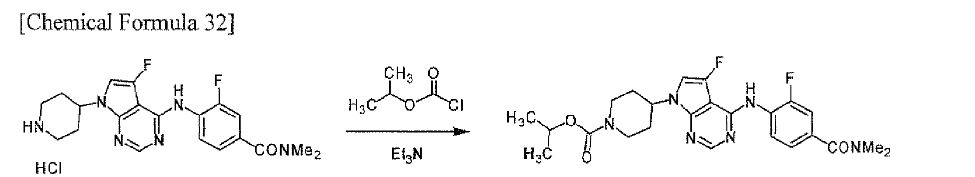 To a solution of 3-fluoro-4-[5-fluoro-7-(piperidin-4-yl)-7H-pyrrolo[2,3-d]pyimidin-4-ylamino]-N,N-dimethylbenzamide hydrochloride (obtained in Reference Example 4; 50 mg) in dichloromethane (1 mL) were added triethylamine (47.9 µL) and isopropyl chloroformate (16.8 mg), and the mixture was stirred at room temperature for 16 hours. To the reaction mixture was added water, and then the mixture was extracted with chloroform. The organic layer was concentrated, and the resulting residue was purified by column chromatography on NH silica-gel (Chromatorex; Fuji Silysia Chemical Ltd., solvent; hexane/ethyl acetate = 75/25 to 20/80) to give the titled compound (40 mg) as a powder (yield 73%).
MS(APCI)m/z; 487[M+H]⁺.

### Example 5

Preparation of 1-ethylpropyl 4-[4-(4-dimethylcarbamoyl-2-fluorophenylamino)-5-fluoropyrrolo[2,3-d]pyrimidin-7-yl]piperidine-1-carboxylate 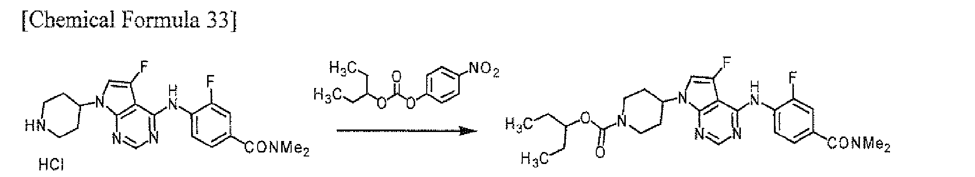 To a solution of 3-fluoro-4-[5-fluoro-7-(piperidin-4-yl)-7H-pyrrolo[2,3-d]pyrimidin-4-ylamino]-N,N-dimethylbenzamide hydrochloride (obtained in Reference Example 4; 50 mg) in dichloromethane (1 mL) were added triethylamine (79.8 µL) and 3-pentyl 4-nitrophenyl carbonate (obtained in Reference Example 5; 34.5 mg), and the mixture was stirred at room temperature for 15 hours. To the reaction mixture were added water and a saturated aqueous sodium hydrogencarbonate solution, and the mixture was extracted with ethyl acetate. The organic layer was concentrated, and the resulting residue was purified by column chromatography on NH silica-gel (Chromatorex; Fuji Silysia Chemical Ltd., solvent; hexane/ethyl acetate = 75/25 to 45/55) to give the titled compound (37.3 mg) as a powder (yield 64%).
MS(APCI)m/z; 515[M+H]⁺.

### Example 6

Preparation of 4-[7-[1-(5-ethylpyrimidin-2-yl)piperidin-4-yl]-5-fluoro-7H-pyrrolo[2,3-d]pyrimidin-4-ylamino]-3-fluoro-N,N-dimethylbenzamide 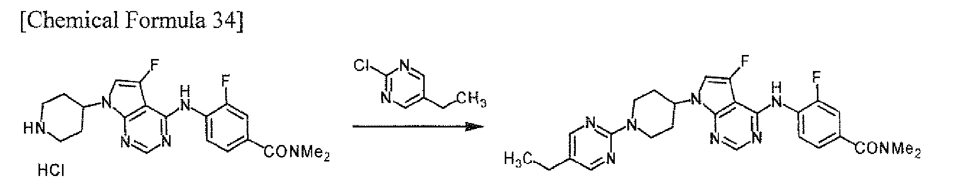 To a solution of 3-fluoro-4-[5-fluoro-7-(piperidin-4-yl)-7H-pyrrolo[2,3-d]pyrimidin-4-ylamino]-N,N-dimethylbenzamide hydrochloride (obtained in Reference Example 4; 50 mg) in dimethylformamide (1 mL) were added diisopropylethylamine (79 µL) and 5-ethyl-2-chloropyrimidine (42 µL), and the mixture was stirred at 80°C for 15 hours. To the reaction mixture was added water, and then the mixture was extracted with ethyl acetate. The organic layer was concentrated, and the resulting residue was purified by column chromatography on NH silica-gel (Chromatorex; Fuji Silysia Chemical Ltd., solvent; hexane/ethyl acetate = 70/30 to 25/75) to give the titled compound (17.9 mg) as a powder (yield 31%).
MS(APCI)m/z; 507[M+H]⁺.

### Example 7

Preparation of 3-fluoro-4-[5-fluoro-7-[1-(pyridin-2-ylmethyl)piperidin-4-yl]-7H-pyrrolo[2,3-d]pyrimidin-4-ylamino]-N,N-dimethylbenzamide 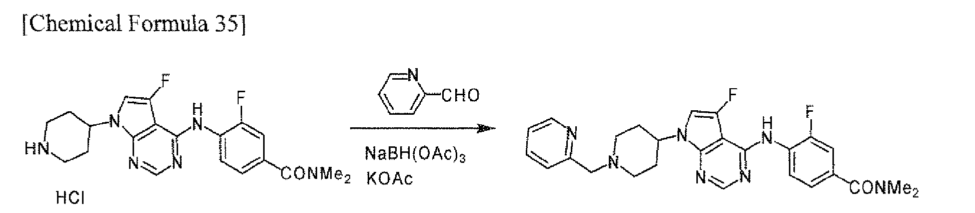 To a solution of 3-fluoro-4-[5-fluoro-7-piperidin-4-yl)-7H-pyrrolo[2,3-d]pyrimidin-4-ylamino]-N,N-dimethylbenzamide (obtained in Reference Example 4; 80 mg) and 2-pyridinecarboxaldehyde (32 mg) in dichloromethane (5 mL) was added potassium acetate (29 mg) at room temperature, and the mixture was stirred for 1 hour. To the reaction mixture was added sodium triacetoxyborohydride (64 mg), and the mixture was stirred at room temperature overnight. The reaction mixture was poured into water and extracted with chloroform three times. The organic layer was dried over magnesium sulfate and then filtered, and the filtrate was concentrated. The resulting residue was purified by column chromatography on silica gel (solvent; chloroform/methanol = 100/0 to 90/10) to give the titled compound (48.8 mg) as a viscous oil (yield 50%).
MS(APCI)m/z; 492[M+H]⁺.

### Example 8

Preparation of 4-[7-[1-(5-ethylpyrimidin-2-yl)piperidin-4-yl]-5-fluoro-7H-pyrrolo[2,3-d]pyrimidin-4-ylamino]-3-fluoro-N-(2-hydroxyethyl)-N-methylbenzamide 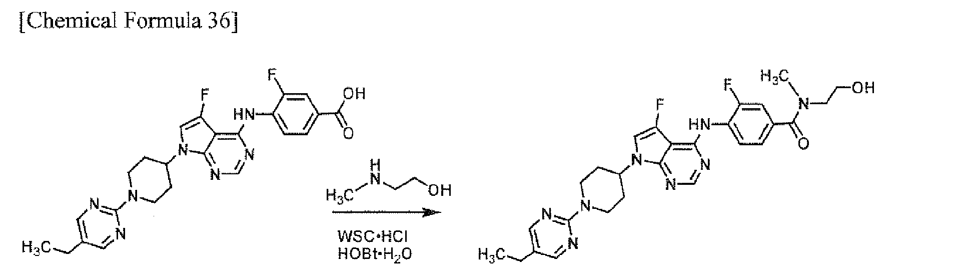 To a solution of 4-[7-[1-(5-ethylpyrimidin-2-yl)piperidin-4-yl]-5-fluoro-7H-pyrrolo[2,3-d]pyrimidin-4-ylamino]-3-fluorobenzoic acid (obtained in Reference Example 24; 50 mg) in dichloromethane (1 mL) were added 2-(methylamino)ethanol (9.4 mg), N-hydroxybenzotriazole monohydrate (HOBt H₂O; 24 mg), triethylamine (43.6 µL) and 1-ethyl-3-(3-dimethylaminopropyl)carbodiimide hydrochloride (WSC HCl; 29.8 mg), and the mixture was stirred at room temperature for 16 hours. To the reaction mixture was added a saturated aqueous sodium hydrogencarbonate solution, and then the mixture was extracted with chloroform. The organic layer was concentrated, and the resulting residue was purified by column chromatography on silica gel (solvent; chloroform/methanol = 100/0→95/5) to give the titled compound (38.6 mg) as a colorless powder (yield 69%).
MS(APCI)m/z; 537[M+H]⁺.

### Example 9

Preparation of tert-butyl 4-[4-[(4-dimethylcarbamoyl-2-fluorophenyl)amino]-5-cyanopyrrolo[2,3-d]pyrimidin-7-yl]piperidine-1-carboxylate 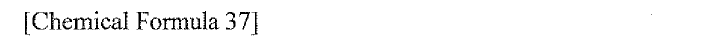 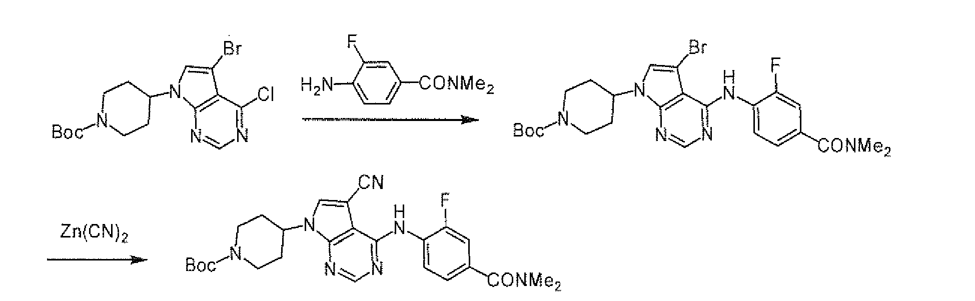
(1) A compound obtained in Reference Example 8(2) (100 mg) was treated in the similar manner to Example 1 to give tert-butyl 4-[4-[(4-dimethylcarbamoyl-2-fluorophenyl)amino]-5-bromopyrrolo[2,3-d]pyrimidin-7-yl]piperidine-1-carboxylate (111 mg) as a colorless powder (yield 82%).
   MS(APCI)m/z; 561/563[M+H]⁺.
(2) To a solution of a compound obtained in the above (1) (98.3 mg) in dimethylformamide (1.8 mL) were added zinc cyanide (12 mg) and tetrakis-triphenylphosphine palladium (20 mg), and the mixture was stirred under nitrogen atmosphere at 110°C for 16 hours. To the reaction mixture was added water, and then the mixture was extracted with ethyl acetate. The organic layer was concentrated, and the resulting residue was purified by column chromatography on NH silica-gel (Chromatorex; Fuji Silysia Chemical Ltd., solvent; hexane/ethyl acetate = 75/25 to 40/60) to give the titled compound (75.8 mg) as a powder (yield 85%).
   MS(APCI)m/z; 508[M+H]⁺.

### Example 10

Preparation of tert-butyl 4-[4-(2-chloro-4-mesylbenzoyl)-5-fluoropyrrolo[2,3-d]pyrimidm-7-yl]piperidine-1-carboxylate 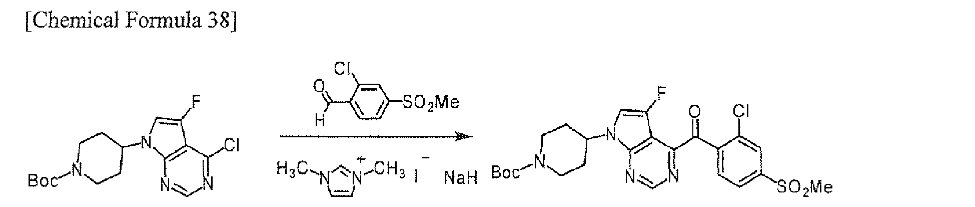 To a solution of tert-butyl 4-(4-chloro-5-fluoropyrrolo[2,3-d]pyrimidin-7-yl)piperidine-1-carboxylate (obtained in Reference Example 1; 710 mg), 2-chloro-4-(methylsulfonyl)benzaldehyde (656 mg) and N,N-dimethylimidazolnium iodide (672 mg) in dioxane (10 mL) was added sodium hydride (60%; 160 mg), and the mixture was stirred at room temperature overnight. The reaction mixture was poured into an aqueous ammonium chloride solution and extracted with ethyl acetate three times. The organic layer was dried over magnesium sulfate, and then filtered. The filtrate was concentrated, and the resulting residue was purified by column chromatography on silica gel (solvent; hexane/ethyl acetate = 40/60 to 70/30) to give the titled compound (74.1 mg) as a solid (yield 7%).
MS(APCI)m/z; 537/539[M+H]⁺.

### Example 11

Preparation of tert-butyl 4-[4-(2-chloro-α,α-difluoro-4-mesylbenzyl)-5-fluoropyrrolo[2,3-d]pyrimidin-7-yl]piperidine-1-carboxylate 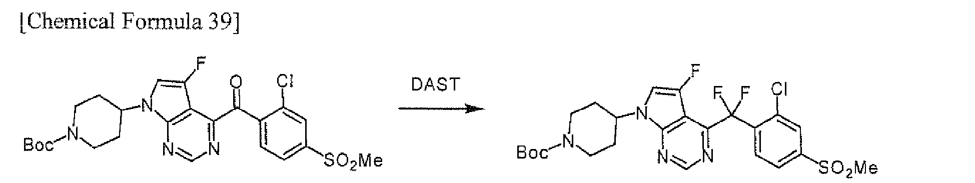 To a solution of tert-butyl 4-[5-fluoro-4-(4-mesylbenzoyl)pyrrolo[2,3-d]pyrimidin-7-yl]piperidine-1-carboxylate (obtained in Example 10; 50 mg) in dichloromethane (10 mL) was added N,N-diethylaminosulfur trifluoride (DAST; 30 mg) at room temperature, and the mixture was stirred at 50°C overnight. The reaction solution was poured into a saturated aqueous sodium hydrogencarbonate solution, and the mixture was extracted with chloroform three times. The organic layer was dried over magnesium sulfate and then filtered, and the filtrate was concentrated. The resulting residue was purified by column chromatography on silica gel (solvent; hexane/ethyl acetate = 35/65 to 55/45) to give the titled compound (1.5 mg) as a solid (yield 3%).
MS(APCI)m/z; 559/561[M+H]⁺.

### Example 12

Preparation of tert-butyl 4-[4-(2-chloro-α-hydroxy-4-mesylbenzyl)-5-fluoropyrrolo[2,3-d]pyrimidin-7-yl]piperidine-1-carboxylate 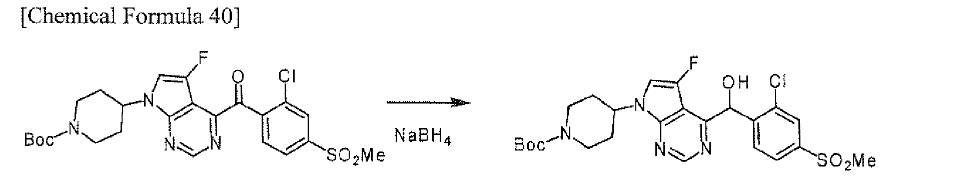 To a solution of tert-butyl 4-[5-fluoro-4-(4-mesylbenzoyl)pyrrolo[2,3-d]pyrimidin-7-yl]piperidine-1-carboxylate (obtained in Example 10; 43 mg) in methanol, (5 mL) was added sodium borohydride (6 mg) at room temperature, and the mixture was stirred at the same temperature overnight. The reaction mixture was poured into water, and the mixture was extracted with ethyl acetate three times. The organic layer was dried over magnesium sulfate and then filtered, and the filtrate was concentrated. The resulting residue was purified by column chromatography on silica gel (solvent; hexane/ethyl acetate = 50/50 to 80/20) to give the titled compound (29.1 mg) as a solid (yield 68%).
MS(APCI)m/z; 539/541 [M+H]⁺.

### Examples 13 to 34

Corresponding starting compounds were treated in the similar manner to Example 1 to give compounds of the following Tables 1 to 3.

Table 1

**[Table 1]**

| Examples | Structural Formula | Physicochemical properties etc. |
|---|---|---|
| 13 | 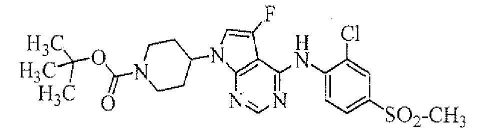 | Powder MS(APCI)m/z: 524/526 [M+H]⁺ |
| 14 | 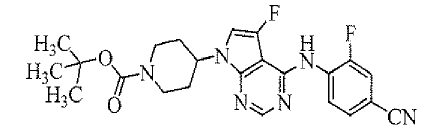 | Powder MS(APCI)m/z: 455 [M+H]⁺ |
| 15 | 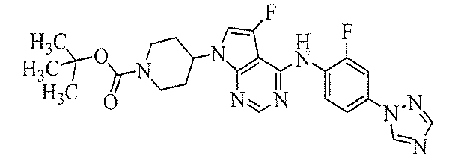 | Powder MS(APCI)m/z: 497 [M+H]⁺ |
| 16 | 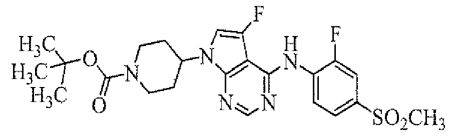 | Powder MS(APCI)m/z: 508 [M+H]⁺ |
| 17 | 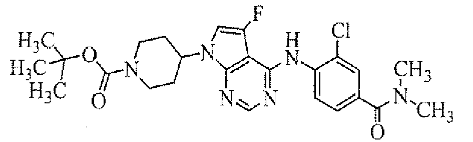 | Powder MS(APCI)m/z: 517/519 [M+H]⁺ |
| 18 | 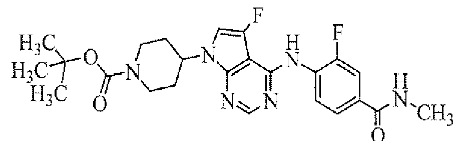 | Powder MS(APCI)m/z: 487 [M+H]⁺ |
| 19 | 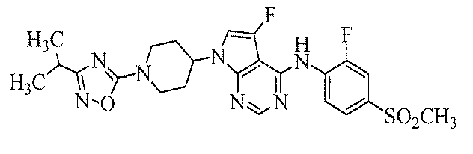 | Powder MS(APCI)m/z: 518 [M+H]⁺ |

Table 2

**[Table 2]**

| Examples | Structural Formula | Physicochemical properties etc. |
|---|---|---|
| 20 | 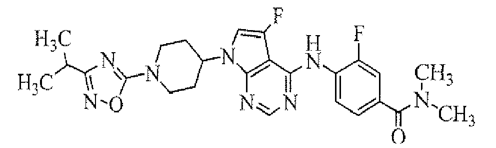 | Powder MS(APCI)m/z: 511 [M+H]⁺ |
| 21 | 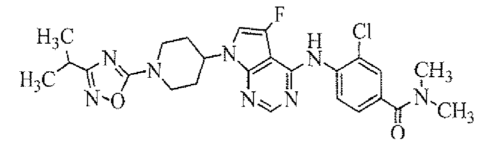 | Powder MS(APCI)m/z: 527/529 [M+H]⁺ |
| 22 | 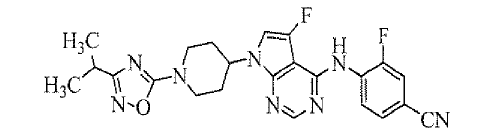 | Powder MS(APCI)m/z: 465 [M+H]⁺ |
| 23 | 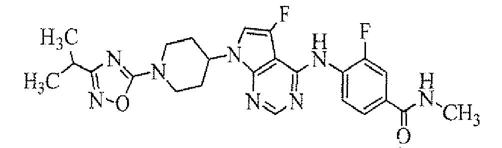 | Powder MS(APCI)m/z: 497 [M+H]⁺ |
| 24 | 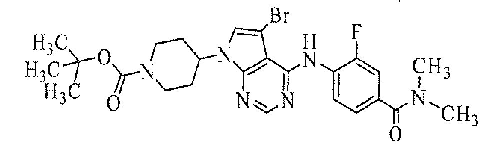 | Powder MS(APCI)m/z: 561/563 [M+H]⁺ |
| 25 | 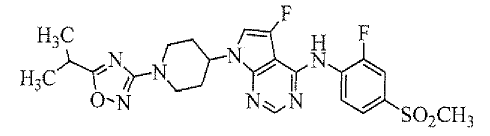 | Powder MS(APCI)m/z: 518 [M+H]⁺ |
| 26 | 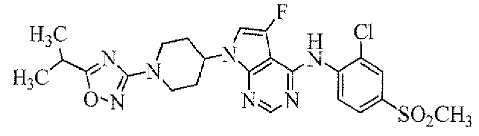 | Powder MS(APCI)m/z: 534/536 [M+H]⁺ |

Table 3

**[Table 3]**

| Examples | Structural Formula | Physicochemical properties etc. |
|---|---|---|
| 27 | 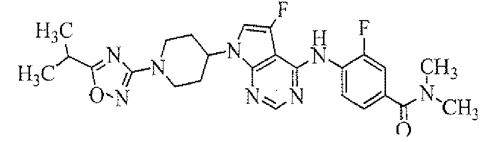 | Powder MS(APCI)m/z: 511 [M+H]⁺ |
| 28 | 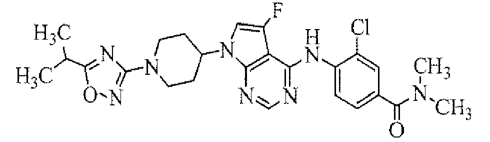 | Powder MS(APCI)m/z; 527/529 [M+H]⁺ |
| 29 | 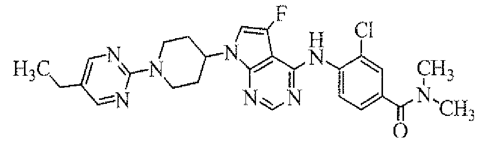 | Powder MS(APCI)m/z: 523/525 [M+H]⁺ |
| 30 | 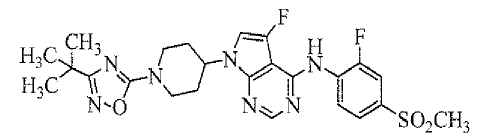 | Powder MS(A-PCI)m/z: 532 [M+H]⁺ |
| 31 | 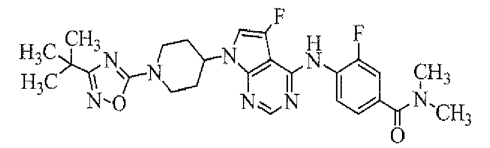 | Powder MS(APCI)m/z: 525 [M+H]⁺ |
| 32 | 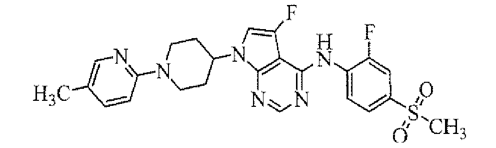 | Powder MS(APCI)m/z: 499 [M+H]⁺ |
| 33 | 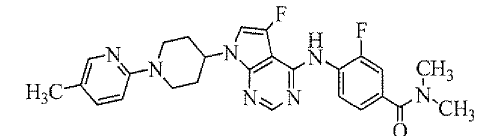 | Powder MS(APCI)m/z: 492 [M+H]⁺ |
| 34 | 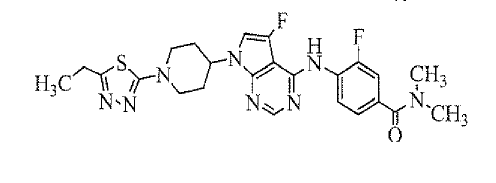 | Powder MS(APCI)m/z: 513 [M+H]⁺ |

### Examples 35 to 51 1

Corresponding starting compounds were treated in the similar manner to Example 3 to give compounds of the following Tables 4 to 6.

Table 4

**[Table 4]**

| Examples | Structural Formula | Physicochemical properties etc. |
|---|---|---|
| 35 | 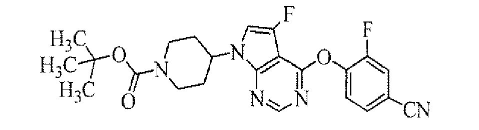 | Powder MS(APCI)m/z: 456 [M+H]⁺ |
| 36 | 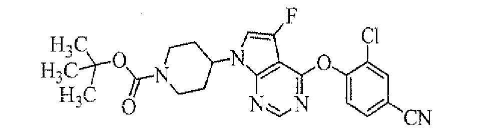 | Powder MS(APCI)m/z: 472/474 [M+H]⁺ |
| 37 | 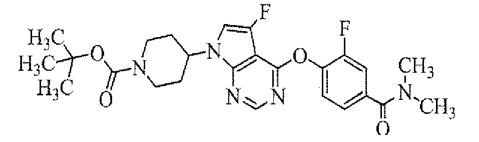 | Powder MS(APCI)m/z: 502 [M+H]⁺ |
| 38 | 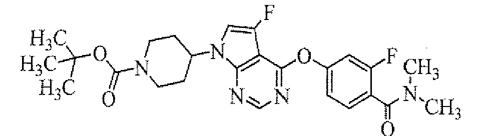 | Powder MS(APCI)m/z: 502 [M+H]⁺ |
| 39 | 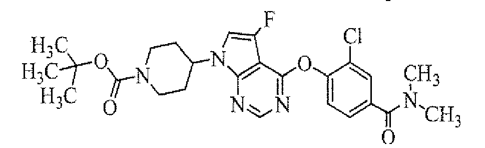 | Powder MS(APCI)m/z: 518/520 [M+H]⁺ |
| 40 | 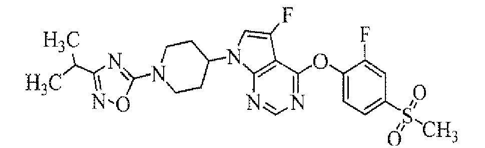 | Powder MS(APCI)m/z: 519 [M+H]⁺ |
| 41 | 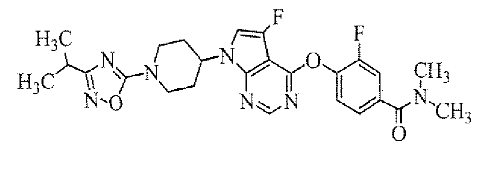 | Powder MS(APCI)m/z: 512 [M+H]⁺ |

Table 5

**[Table 5]**

| Examples | Structural Formula | Physicochemical properties etc. |
|---|---|---|
| 42 | 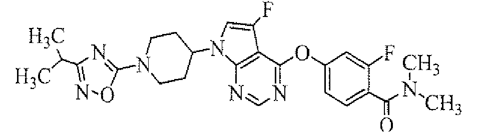 | Powder MS(APCI)m/z: 512 [M+H]⁺ |
| 43 | 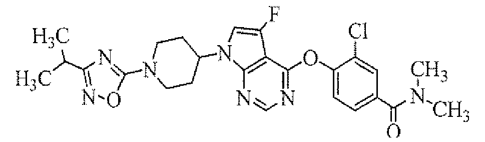 | Powder MS(APCI)m/z: 528/530 [M+H]⁺ |
| 44 | 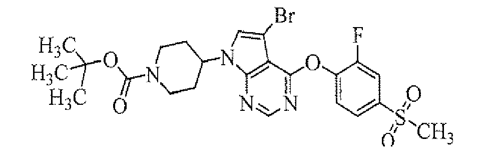 | Powder MS(APCI)m/z: 569/571 [M+H]⁺ |
| 45 | 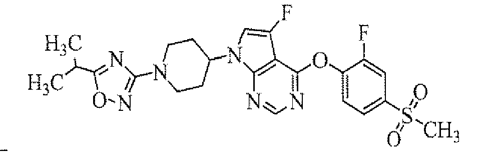 | Powder MS(APCI)m/z: 519 [M+H]⁺ |
| 46 | 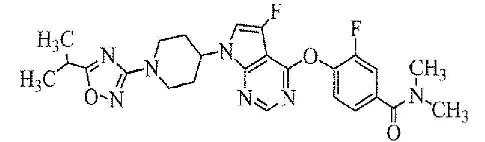 | Powder MS(APCI)m/z: 512 [M+H]⁺ |
| 47 | 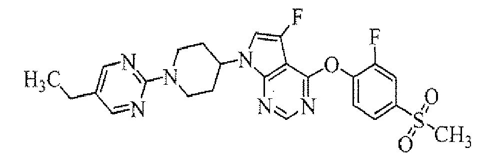 | Powder MS(APCI)m/z: 515 [M+H]⁺ |
| 48 | 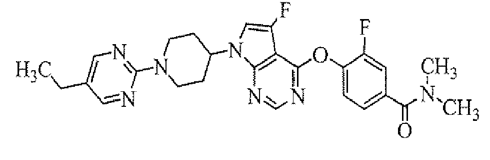 | Powder MS(APCI)m/z: 508 [M+H]⁺ |

Table 6

**[Table 6]**

| Examples | Structural Formula | Physicochemical properties etc. |
|---|---|---|
| 49 | 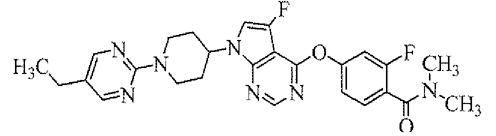 | Powder MS(APCI)m/z: 508 [M+H]⁺ |
| 50 | 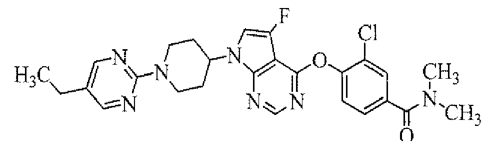 | Powder MS(APCI)m/z: 524/526 [M+H]⁺ |
| 51 | 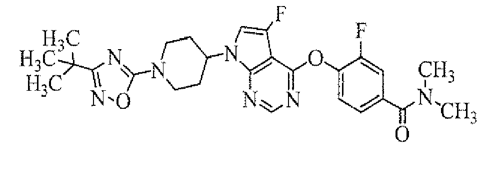 | Powder MS(APCI)m/z: 526 [M+H]⁺ |

### Examples 52 to 53

Corresponding starting compounds were treated in the similar manner to Example 5 to give compounds of the following Table 7.

Table 7

**[Table 7]**

| Examples | Structural Formula | Physicochemical properties etc. |
|---|---|---|
| 52 | 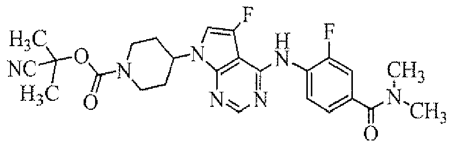 | Powder MS(APCI)m/z: 512 [M+H]⁺ |
| 53 | 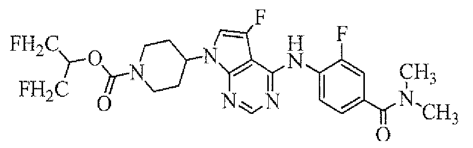 | Powder MS(APCI)m/z: 523 [M+H]⁺ |

### Examples 54 to 56

Corresponding starting compounds were treated in the similar manner to Example 6 to give compounds of the following Table 8.

Table 8

**[Table 8]**

| Examples | Structural Formula | Physicochemical properties etc. |
|---|---|---|
| 54 | 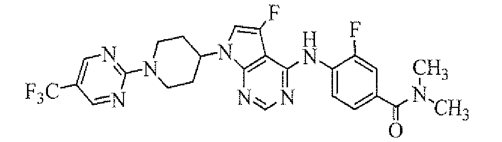 | Powder MS(APCI)m/z: 547[M+H]⁺ |
| 55 | 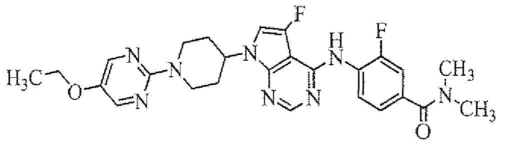 | Powder MS(APCI)m/z: 523 [M+H]⁺ |
| 56 | 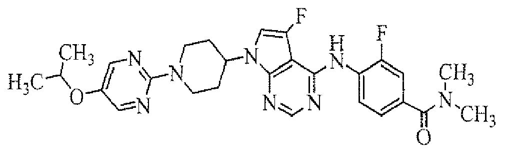 | Powder MS(APCI)m/z: 537 [M+H]⁺ |

### Examples 57 to 58

Corresponding starting compounds were treated in the similar manner to Example 7 to give compounds of the following Table 9.

Table 9

**[Table 9]**

| Examples | Structural Formula | Physicochemical properties etc. |
|---|---|---|
| 57 | 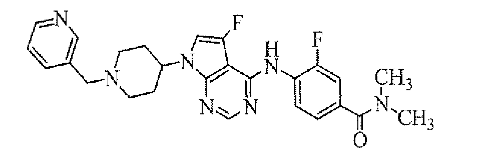 | Viscous oil MS(APCI)m/z: 492 [M+H]⁺ |
| 58 | 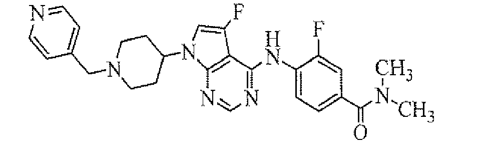 | Powder MS(APCI)m/z: 492 [M+H]⁺ |

### Examples 59 to 63

Corresponding starting compounds were treated in the similar manner to Example 8 to give compounds of the following Table 10.

Table 10

**[Table 10]**

| Examples | Structural Formula | Physicochemical properties etc. |
|---|---|---|
| 59 | 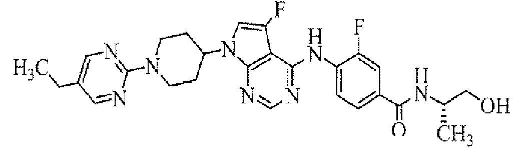 | Powder MS(APCI)m/z: 537 [M+H]⁺ |
| 60 | 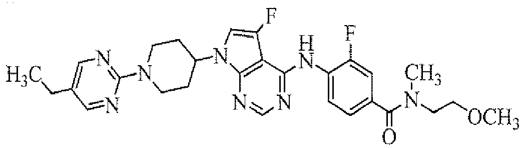 | Powder MS(APCI)m/z: 551 [M+H]⁺ |
| 61 | 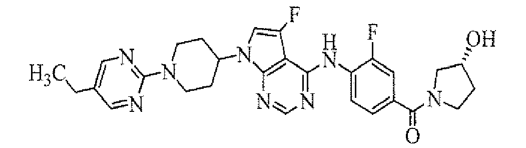 | Powder MS(APCI)m/z: 549 [M+H]⁺ |
| 62 | 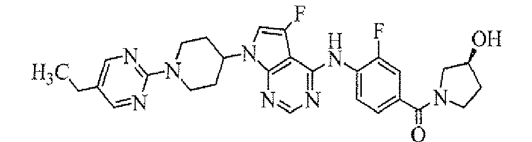 | Powder MS(APCI)m/z: 549 [M+H]⁺ |
| 63 | 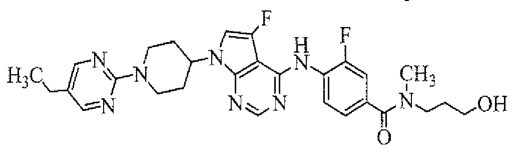 | Powder MS(APCI)m/z: 551 [M+H]⁺ |

### Example 64

Corresponding starting compounds were treated in the similar manner to Example 9 to give compounds of the following Table 11.

Table 11

**[Table 11]**

| Examples | Structural Formula | Physicochemical properties etc. |
|---|---|---|
| 64 | 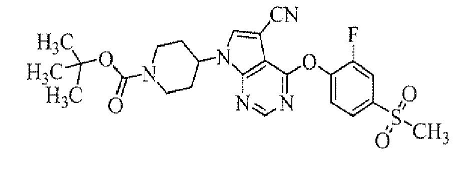 | Powder MS(APCI)m/z: 516 [M+H]⁺ |

### Example 65

Corresponding starting compounds were treated in the similar manner to Example 10 to give compounds of the following Table 12.

Table 12

**[Table 12]**

| Examples | Structural Formula | Physicochemical properties etc. |
|---|---|---|
| 65 | 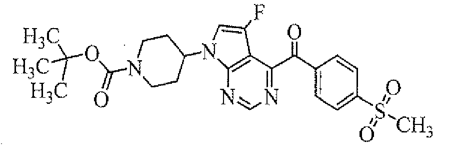 | Viscous oil MS(APCI)m/z: 503 [M+H]⁺ |

### Example 66

Preparation of 3-fluoro-4-[5-fluoro-7-(5'-isopropoxy-3,4,5,6-tetrahydro-2H-[1,2']bipyridi-4-yl)-7H-pynolo[2,3-d]pyrimidin-4-ylamino]-N,N-dimethylbenzamide 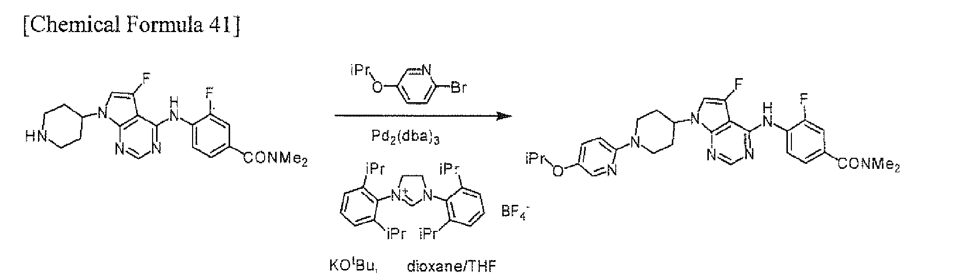 To a solution of 3-fluoro-4-[5-fluoro-7-(piperidin-4-yl)-7H-pyrrolo[2,3-d]pyrimidin-4-ylamino]-N,N-dimethylbenzamide (50 mg), which was obtained by treating a compound obtained in Reference Example 4 with a saturated aqueous sodium hydrogencarbonate solution to extract with chloroform, in 1,4-dioxane (0.5 mL) and tetrahydrofuran (0.5 mL) were added tris(dibenzylideneacetone)dipalladium (3.4 mg), 1,3-bis(2,6-diisopropylphenyl)-4,5-dihydroimidazolium tetrafluoroborate (3 mg) and potassium tert-butoxide (49 mg), and the mixture was stirred in a microwave reactor (Initiator, manufactured by Biotage Inc.) at 130°C for 30 minutes. To the reaction mixture was added water, and the mixture was extracted with ethyl acetate. The organic layer was concentrated, and the resulting residue was purified by column chromatography on NH silica-gel (Chromatorex; Fuji Silysia Chemical Ltd., solvent; hexane/ethyl acetate = 70/30 to 35/65) to give the titled compound (17.4 mg) as a powder (yield 26%).
MS(APCI)m/z; 536[M+H]⁺.

### Example 67

Preparation of 3-fluoro-4-[5-fluoro-7-[1-(5-propyl-[1,2,4]oxadiazol-3-yl)piperidin-4-yl]-7H-pyrrolo[2,3-d]pyrimidin-4-ylamino]-N,N-dimethylbenzamide 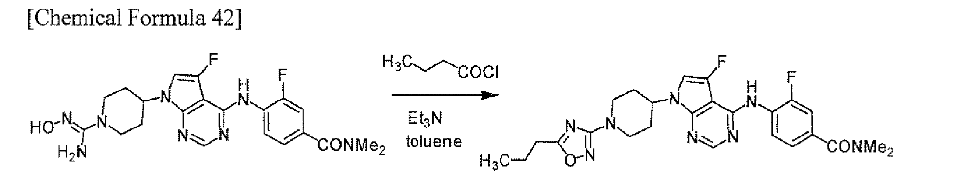 To a solution of 3-fluoro-4-[5-fluoro-7-[1-(N-hydroxycarbamimidoyl)piperidin-4-yl]-7H-pyrrolo[2,3-d]pyrimidin-4-ylamino]-N,N-dimethylbenzamide (obtained in Reference Example 55; 106 mg) in toluene (27 µL) were added under ice-cooling triethylamine (27 µL) and butyryl chloride (24 µL), and the mixture was stirred at 130°C for 2 hours. To the reaction mixture was added water, and the mixture was extracted with ethyl acetate. The organic layer was concentrated, and the resulting residue was purified by column chromatography on NH silica-gel (Chromatorex; Fuji Silysia Chemical Ltd., solvent; hexane/ethyl acetate = 67/33 to 40/60) to give the titled compound (23.5 mg) as a powder (yield 20%).
MS(APCI)m/z; 511 [M+H]⁺.

### Example 68

Preparation of 4-[7-[1-[5-(3,3-difluoroazetidine-1-carbonyl)pyrimidin-2-yl]piperidin-4-yl]-5-fluoro-7H-pyrrolo[2,3-d]pyrimidin-4-ylamino]-3-fluoro-N,N-dimethylbenzamide 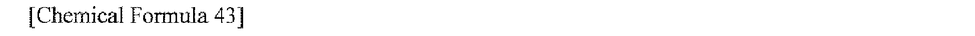 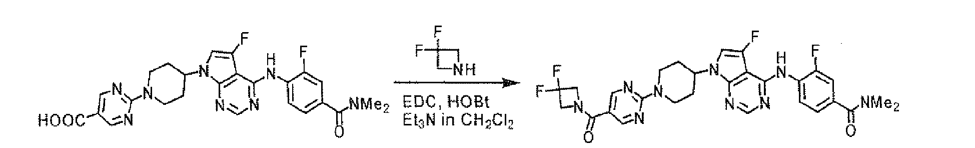 To a solution of 2-[4-[4-(4-dimethylcarbamoyl-2-fluoro-phenylamino)-5-fluoropyrrolo[2,3-d]pyrimidin-7-yl]piperidin-1-yl]pyrimidine-5-carboxylic acid (obtained in Reference Example 56; 52 mg) in methylene chloride (2 mL) were added 3,3-difiuoroazetidine (19 mg), N-hydroxybenzotriazole monohydrate (23 mg), triethylamine (51 mg) and 1-ethyl-3-(3-dimethylaminopropyl)carbodiimide hydrochloride (29 mg), and the mixture was stirred at room temperature overnight. To the reaction mixture was added a saturated aqueous sodium hydrogencarbonate solution, and the mixture was extracted with chloroform. The organic layer was concentrated, and the resulting residue was purified by column chromatography on silica gel (chloroform/methanol = 100/0→90/10) to give the titled compound (88.2 mg) as a white solid (yield 99%).
MS(APCI)m/z; 598[M+H]⁺,

### Examples 69 to 88

Corresponding starting compounds were treated in the similar manner to Example 1 to give compounds of the following Tables 13 to 15.

Table 13

**[Table 13]**

| Examples | Structural Formula | Physicochemical properties etc. |
|---|---|---|
| 69 | 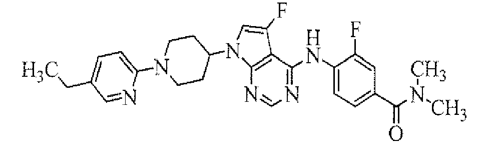 | Powder MS(APCI)m/z: 506 [M+H]⁺ |
| 70 | 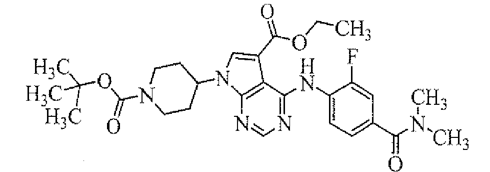 | Powder MS(APCI)m/z: 555 [M+H]⁺ |
| 71 | 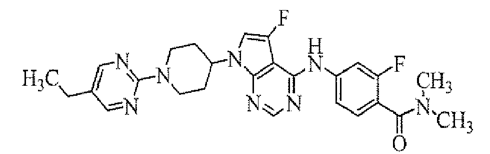 | Powder MS(APCI)m/z: 507 [M+H]⁺ |
| 72 | 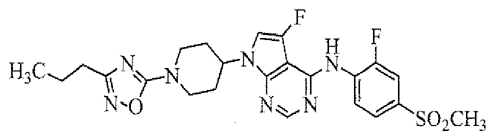 | Powder MS(APCI)m/z: 518 [M+H]⁺ |
| 73 | 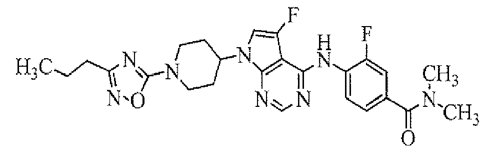 | Powder MS(APCI)m/z: 511 [M+H]⁺ |
| 74 | 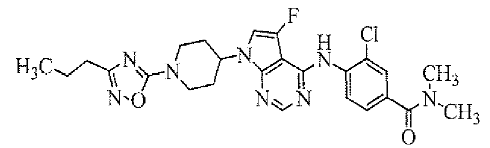 | Powder MS(APCI)m/z: 527/529 [M+H]⁺ |
| 75 | 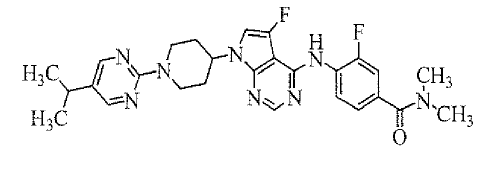 | Powder MS(APCI)m/z: 521 [M+H]⁺ |

Table 14

**[Table 14]**

| Examples | Structural Formula | Physicochemical properties etc. |
|---|---|---|
| 76 | 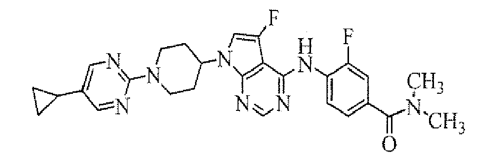 | Powder MS(APCI)m/z: 519 [M+H]⁺ |
| 77 | 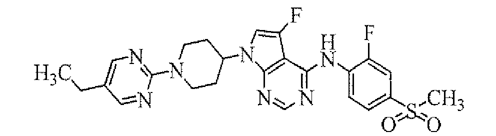 | Powder MS(APCI)m/z: 514 [M+H]⁺ |
| 78 | 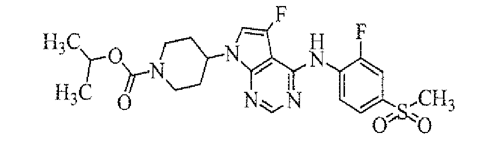 | Powder MS(APCI)m/z: 494 [M+H]⁺ |
| 79 | 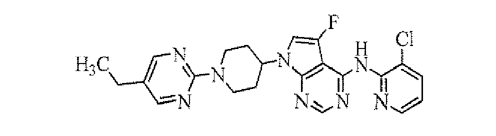 | Powder MS(APCI)m/z: 453/455 [M+H]⁺ |
| 80 | 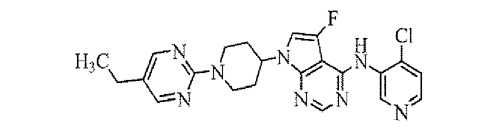 | Powder MS(APCI)m/z: 453/455 [M+H]⁺ |
| 81 | 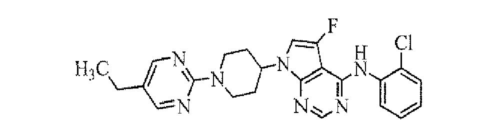 | Powder MS(APCI)m/z: 452/454 [M+H]⁺ |
| 82 | 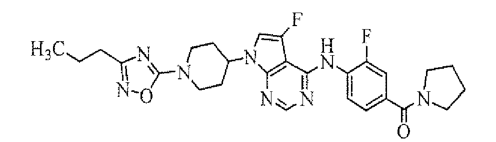 | Powder MS(APCI)m/z: 537 [M+H]⁺ |
| 83 | 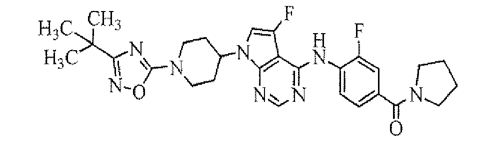 | Powder MS(APCI)m/z: 551 [M+H]⁺ |

Table 15

**[Table 15]**

| Examples | Structural Formula | Physicochemical properties etc. |
|---|---|---|
| 84 | 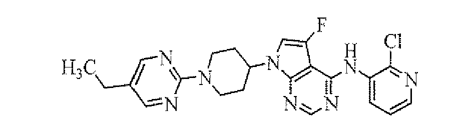 | Powder MS(APCI)m/z: 453/455 [M+H]⁺ |
| 85 | 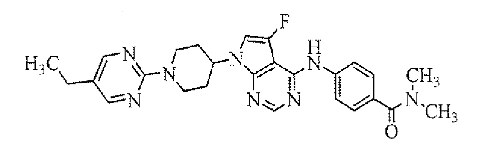 | Powder MS(APCI)m/z: 489 [M+H]⁺ |
| 86 | 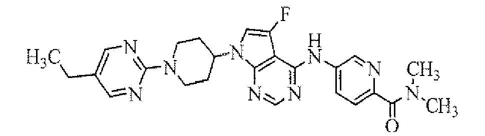 | Powder MS(APCI)m/z: 490 [M+H]⁺ |
| 87 | 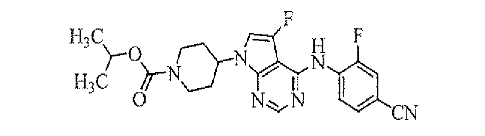 | Powder MS(APCI)m/z: 441 [M+H]⁺ |
| 88 | 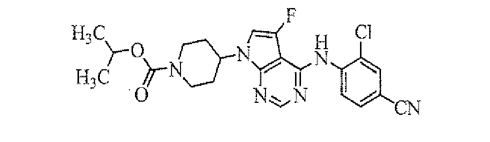 | Solid MS(APCI)m/z: 457/459 [M+H]⁺ |

### Examples 89 to 92

Corresponding starting compounds were treated in the similar manner to Example 3 to give compounds of the following Table 16.

Table 16

**[Table 16]**

| Examples | Structural Formula | Physicochemical properties etc. |
|---|---|---|
| 89 | 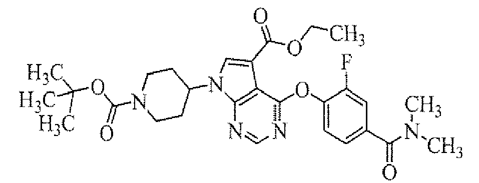 | Powder MS(APCI)m/z: 556 [M+H]⁺ |
| 90 | 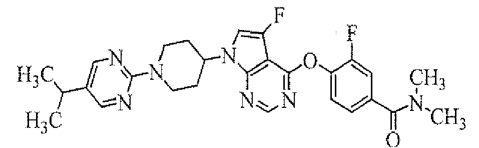 | Powder MS(APCI)m/z: 522 [M+H]⁺ |
| 91 | 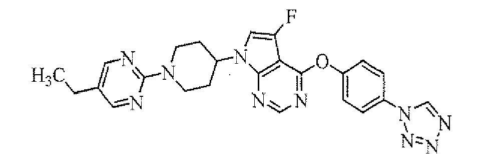 | Powder MS(APCI)m/z: 487 [M+H]⁺ |
| 92 | 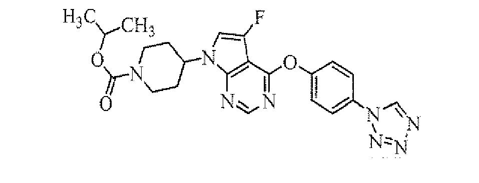 | Powder MS(APCI)m/z: 467 [M+H]⁺ |

### Examples 93 to 96

Corresponding starting compounds were treated in the similar manner to Example 6 to give compounds of the following Table 17.

Table 17

**[Table 17]**

| Examples | Structural Formula | Physicochemical properties etc. |
|---|---|---|
| 93 | 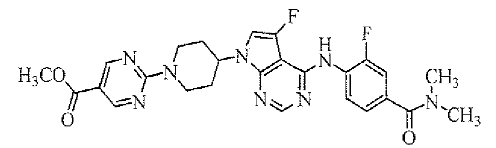 | Powder MS(APCI)m/z: 537 [M+H]⁺ |
| 94 | 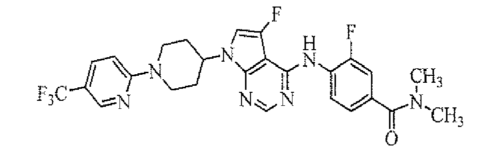 | Powder MS(APCI)m/z: 546 [M+H]⁺ |
| 95 | 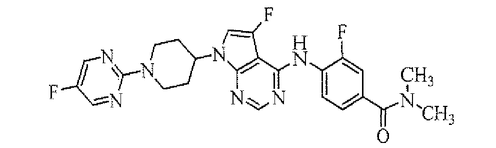 | Powder MS(APCI)m/z: 497 [M+14]⁺ |
| 96 | 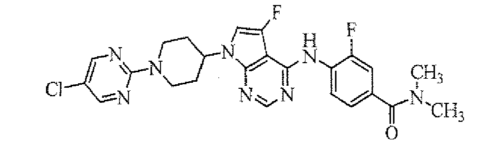 | Powder MS(APCI)m/z: 513/515 [M+H]^{k} |

### Examples 97 to 99

Corresponding starting compounds were treated in the similar manner to Example 66 to give compounds of the following Table 18.

Table 18

**[Table 18]**

| Examples | Structural Formula | Physicochemical properties etc. |
|---|---|---|
| 97 | 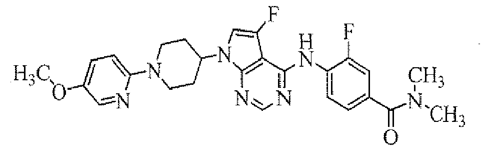 | Powder MS(APCI)m/z: 508 [M+H]⁺ |
| 98 | 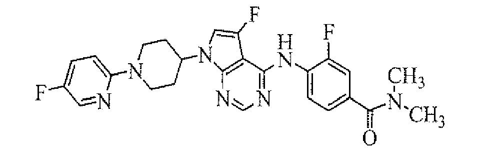 | Powder MS(APCI)m/z: 496 [M+H]⁺ |
| 99 | 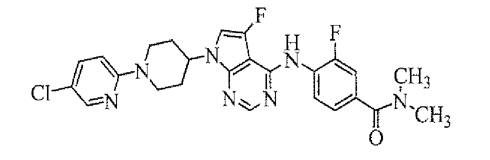 | Powder MS(APCI)m/z: 512/514 [M+H]⁺ |

### Examples 100 to 210

Corresponding starting compounds were treated in the similar manner to Example 8 to give compounds of the following Tables 19 to 32.

Table 19

**[Table 19]**

| Examples | Structural Formula | Physicochemical properties etc. |
|---|---|---|
| 100 | 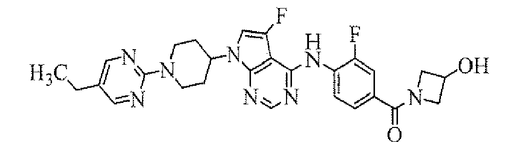 | Powder MS(APCI)m/z: 535 [M+14]⁺ |
| 101 | 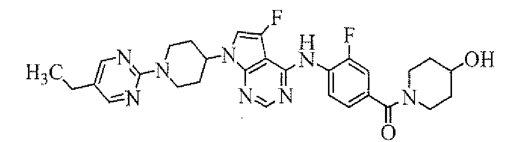 | Powder MS(APCI)m/z: 563 [M+H]⁺ |
| 102 | 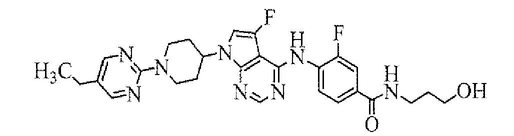 | Powder MS(APCI)m/z: 537 [M+H]⁺ |
| 103 | 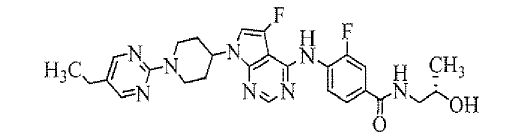 | Powder MS(APCI)m/z: 537 [M+H]⁺ |
| 104 | 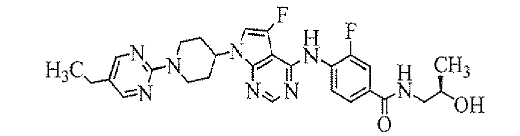 | Powder MS(APCI)m/z: 537 [M+H]⁺ |
| 105 | 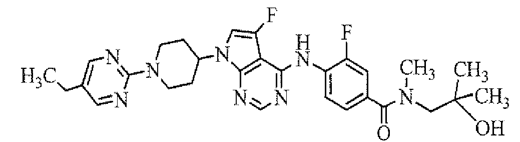 | Powder MS(APCI)m/z: 565 [M+H]⁺ |
| 106 | 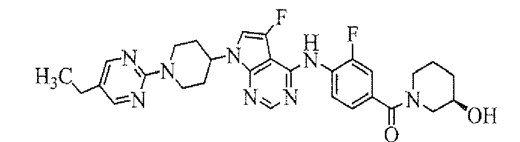 | Powder MS(APCI)m/z: 563 [M+H]⁺ |
| 107 | 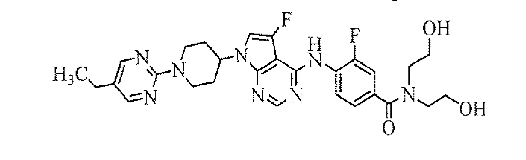 | Powder MS(APCI)m/z: 567 [M+H]⁺ |

Table 20

**[Table 20]**

| Examples | Structural Formula | Physicochemical properties etc. |
|---|---|---|
| 108 | 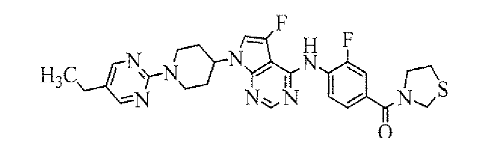 | Powder MS(APCI)m/z: 551 [M+H]⁺ |
| 109 | 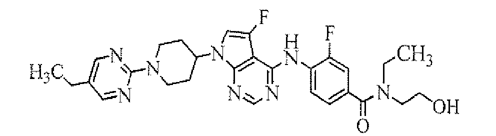 | Powder MS(APCI)m/z: 551 [M+H]⁺ |
| 110 | 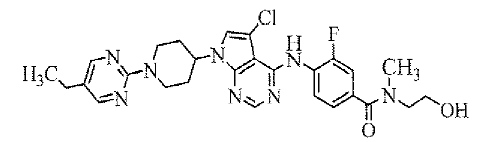 | Powder MS(APCI)m/z: 553/555 [M+H]⁺ |
| 111 | 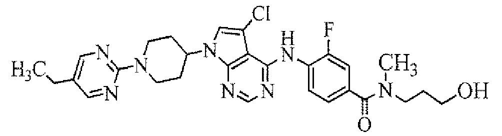 | Powder MS(APCI)m/z: 567/569 [M+H]⁺ |
| 112 | 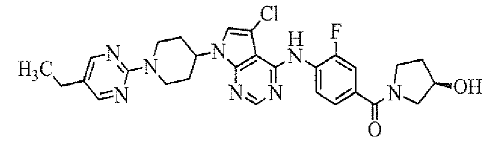 | Powder MS(APCI)m/z: 565/567 [M+14]⁺ |
| 113 | 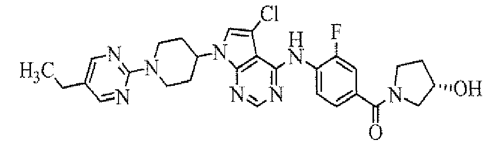 | Powder MS(APCI)m/z: 565/567 [M+H]⁺ |
| 114 | 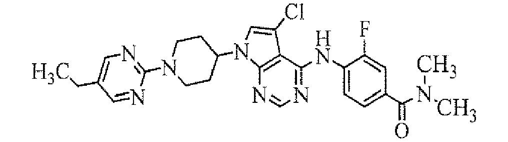 | Powder MS(APCI)m/z: 523/525 [M+H]⁺ |
| 115 | 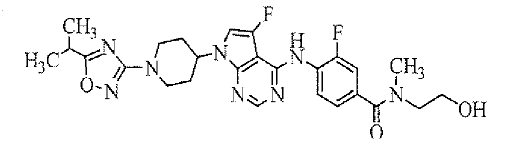 | Powder MS(APCI)m/z: 541 [M+H]⁺ |

Table 21

**[Table 21]**

| Examples | Structural Formula | Physicochemical properties etc. |
|---|---|---|
| 116 | 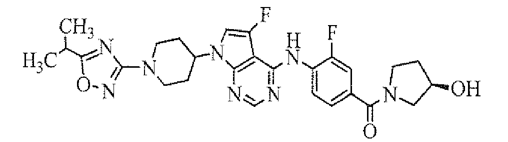 | Powder MS(APCI)m/z: 553 [M+H]⁺ |
| 117 | 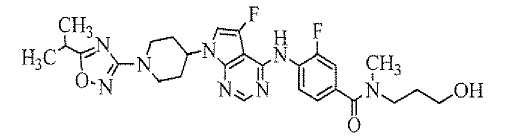 | Powder MS(APCI)m/z: 555 [M+H]⁺ |
| 118 | 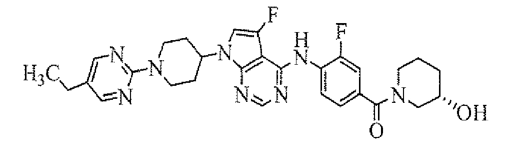 | Powder MS(APCI)m/z: 563 [M+H]⁺ |
| 119 | 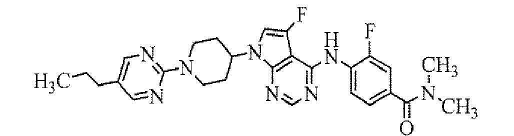 | Powder MS(APCI)m/z: 521 [M+H]⁺ |
| 120 | 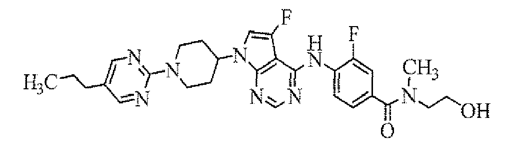 | Powder MS(APCI)m/z: 551 [M+H]⁺ |
| 121 | 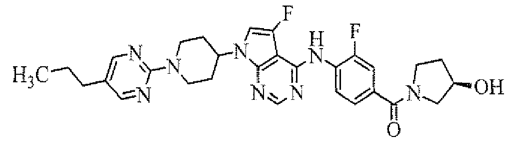 | Powder MS(APCI)m/z: 563 [M+H]⁺ |
| 122 | 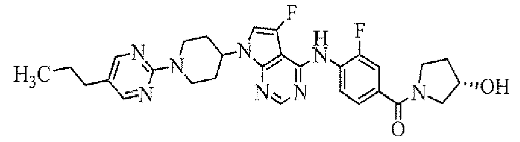 | Powder MS(APCI)m/z: 563 [M+H]⁺ |
| 123 | 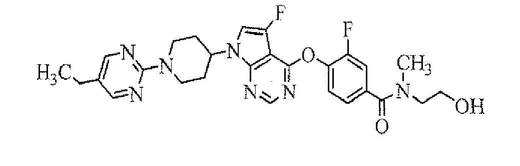 | Powder MS(APCI)m/z: 538 [M+H]⁺ |

Table 22

**[Table 22]**

| Examples | Structural Formula | Physicochemical properties etc. |
|---|---|---|
| 124 | 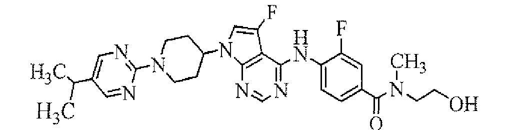 | Powder MS(APCI)m/z: 551 [M+H]⁺ |
| 125 | 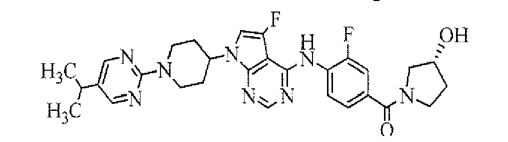 | Powder MS(APCI)m/z: 563 [M+H]⁺ |
| 126 | 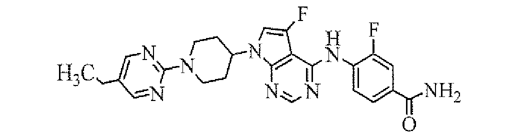 | Powder MS(APCI)m/z: 479 [M+H]⁺ |
| 127 | 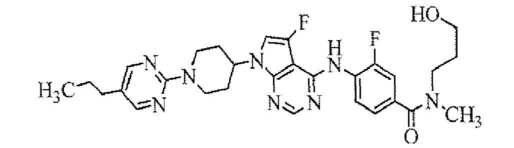 | Powder MS(APCI)m/z: 565 [M+H]⁺ |
| 128 | 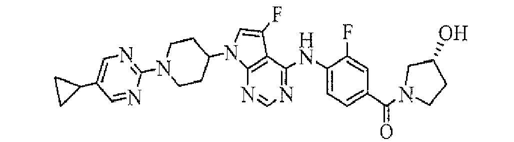 | Powder MS(APCI)m/z: 561 [M+H]⁺ |
| 129 | 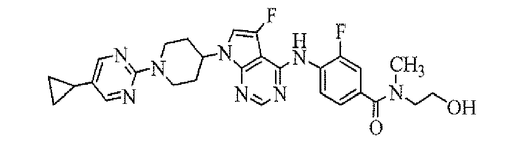 | Powder MS(APCI)m/z: 549 [M+H]⁺ |
| 130 | 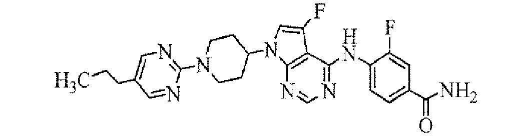 | Powder MS(APCI)m/z: 493 [M+H]⁺ |
| 131 | 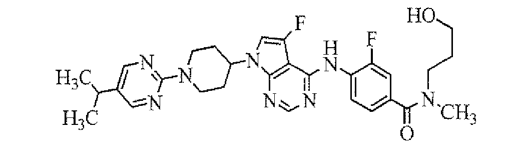 | Powder MS(APCI)m/z: 565 [M+H]⁺ |

Table 23

**[Table 23]**

| Examples | Structural Formula | Physicochemical properties etc. |
|---|---|---|
| 132 | 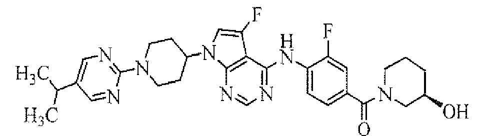 | Powder MS(APCI)m/z: 577 [M+H]⁺ |
| 133 | 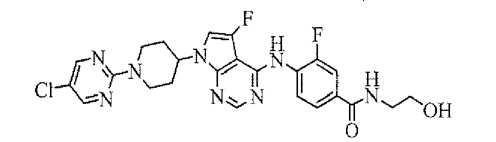 | Powder MS(APCI)m/z: 529/531 [M+H]⁺ |
| 134 | 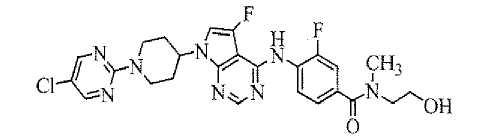 | Powder MS(APCI)m/z: 543/545 [M+H]⁺ |
| 135 | 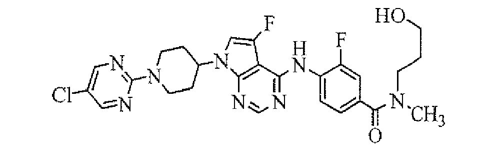 | Powder MS(APCI)m/z: 557/559 [M+H]⁺ |
| 136 | 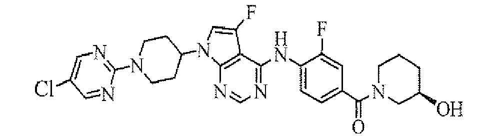 | Powder MS(APCI)m/z: 569/571 [M+H]⁺ |
| 137 | 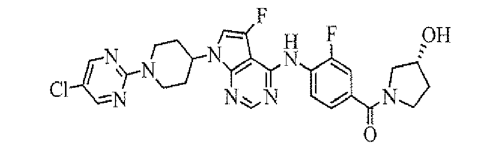 | Powder MS(APCI)m/z: 555/557 [M+H]⁺ |
| 138 | 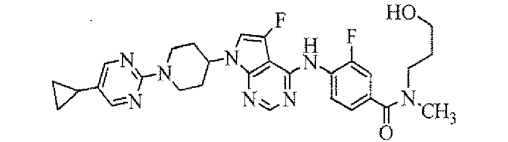 | Powder MS(APCI)m/z: 563 [M+H]⁺ |
| 139 | 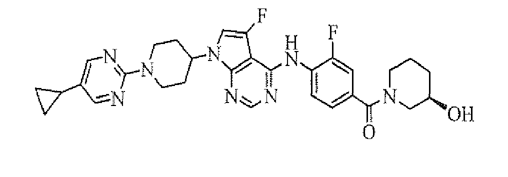 | Powder MS(APCI)m/z: 575 [M+H]+ |

Table 24

**[Table 24]**

| Examples | Structural Formula | Physicochemical properties etc. |
|---|---|---|
| 140 | 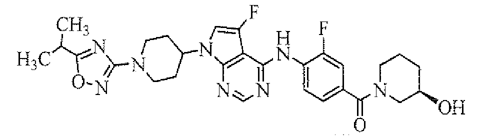 | Powder MS(APCI)m/z: 567 [M+H]+ |
| 141 | 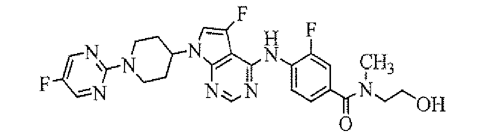 | Powder MS(APCI)m/z: 527 [M+H]+ |
| 142 | 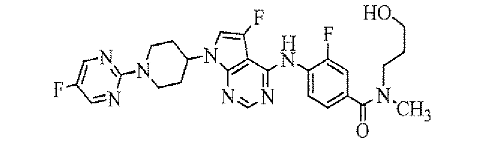 | Powder MS(APCI)m/z: 541 [M+H]⁺ |
| 143 | 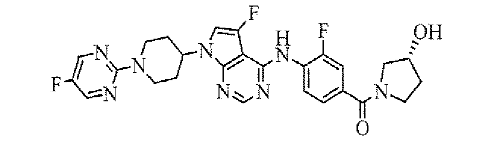 | Powder MS(APCI)m/z: 539 [M+H]⁺ |
| 144 | 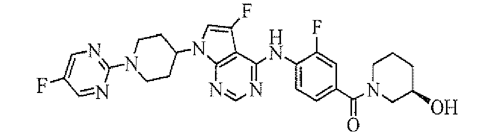 | Powder MS(APCI)m/z: 553 [M+H]⁺ |
| 145 | 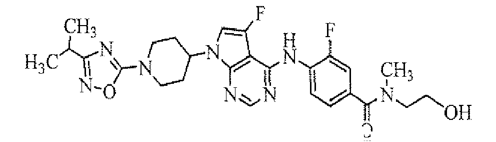 | Powder MS(APCI)m/z: 541 [M+H]⁺ |
| 146 | 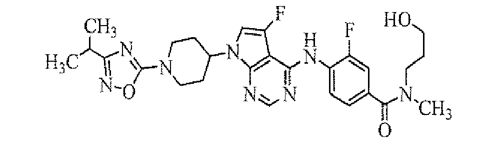 | Powder MS(APCI)m/z: 555 [M+H]⁺ |
| 147 | 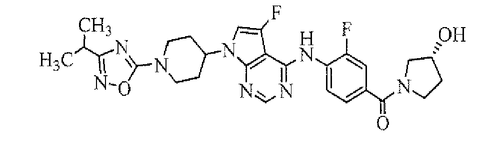 | Powder MS(APCI)m/z: 553 [M+H]⁺ |

Table 25

**[Table 25]**

| Examples | Structural Formula | Physicochemical properties etc. |
|---|---|---|
| 148 | 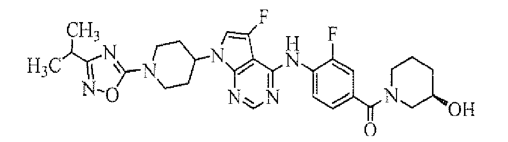 | Powder MS(APCI)m/z: 567 [M+H]⁺ |
| 149 | 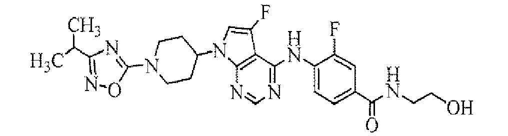 | Powder MS(APCI)m/z: 527 (M+14]⁺ |
| 150 | 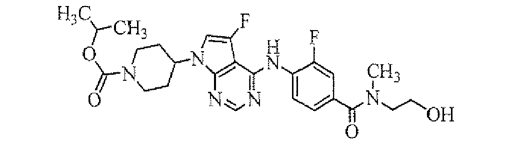 | Powder MS(APCI)m/z: 517 [M+H]⁺ |
| 151 | 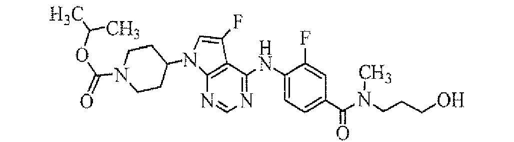 | Powder MS(APCI)m/z: 531 [M+H]⁺ |
| 152 | 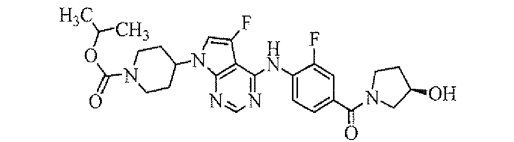 | Powder MS(APCI)m/z: 529 [M+H]⁺ |
| 153 | 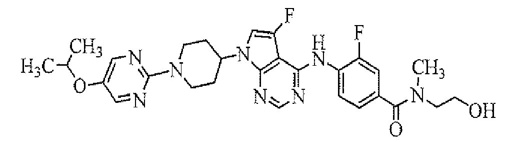 | Powder MS(APCI)m/z: 567 [M+H]⁺ |
| 154 | 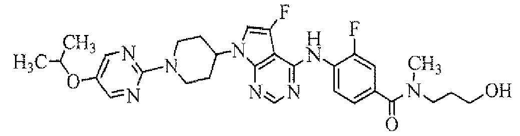 | Powder MS(APCI)m/z: 581 [M+H]⁺ |
| 155 | 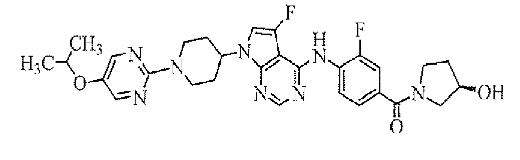 | Powder MS(APCI)m/z: 579 [M+H]⁺ |

Table 26

**[Table 26]**

| Examples | Structural Formula | Physicochemical properties etc. |
|---|---|---|
| 156 | 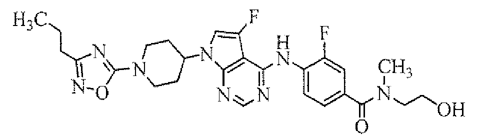 | Powder MS(APCI)m/z: 541 [M+H]⁺ |
| 157 | 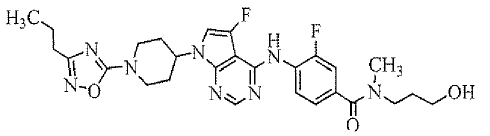 | Powder MS(APCI)m/z: 555 [M+H]⁺ |
| 158 | 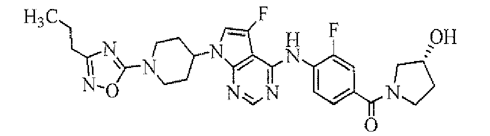 | Powder MS(APCI)m/z: 553 [M+H]⁺ |
| 159 | 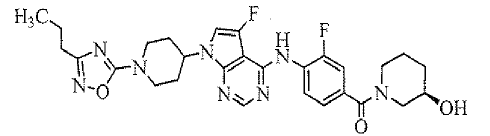 | Powder MS(APCI)m/z: 567 [M+H]⁺ |
| 160 | 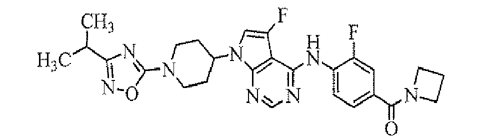 | Powder MS(APCI)m/z: 523 [M+H]+ |
| 161 | 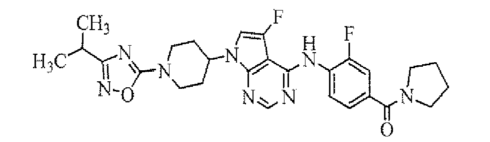 | Powder MS(APCI)m/z: 537 [M+H]+ |
| 162 | 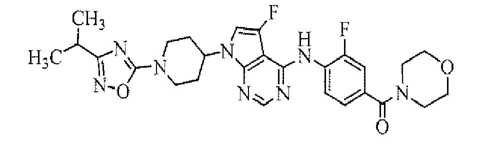 | Powder MS(APCI)m/z: 553 [M+H]+ |

Table 27

**[Table 27]**

| Examples | Structural Formula | Physicochemical properties etc. |
|---|---|---|
| 163 | 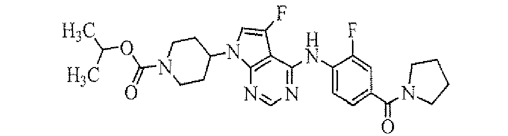 | Powder MS(APCI)m/z: 513 [M+H]⁺ |
| 164 | 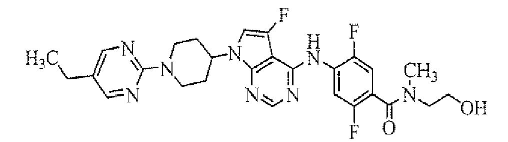 | Powder MS(APCI)m/z: 555 [M+H]⁺ |
| 165 | 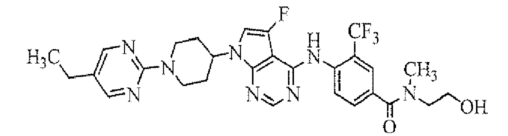 | Powder MS(APCI)m/z: 587 [M+H]⁺ |
| 166 | 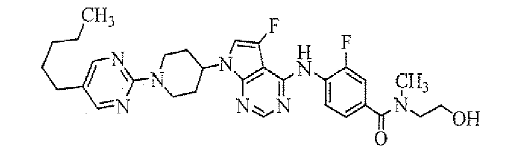 | Powder MS(APCI)m/z: 579 [M+H]⁺ |
| 167 | 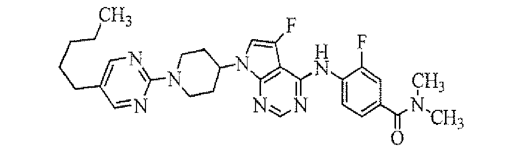 | Powder MS(APCI)m/z: 549 [M+H]⁺ |
| 168 | 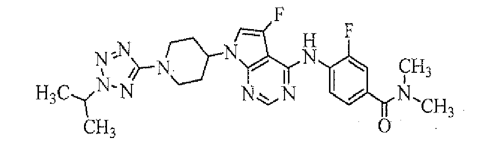 | Powder MS(APCI)m/z: 511 [M+H]+ |
| 169 | 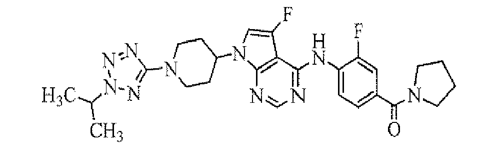 | Powder MS(APCI)m/z: 537 [M+H]+ |
| 170 | 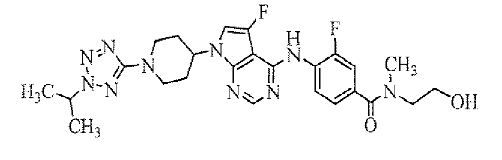 | Powder MS(APCI)m/z: 541 [M+H]+ |

Table 28

**[Table 28]**

| Examples | Structural Formula | Physicochemical properties etc. |
|---|---|---|
| 171 | 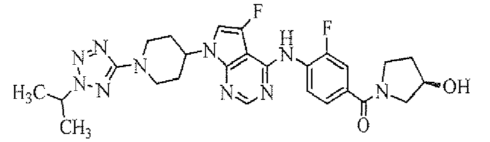 | Powder MS(APCI)m/z: 553 [M+H]⁺ |
| 172 | 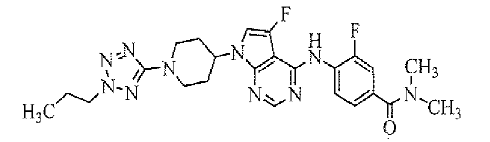 | Powder MS(APCI)m/z: 511 [M+H]⁺ |
| 173 | 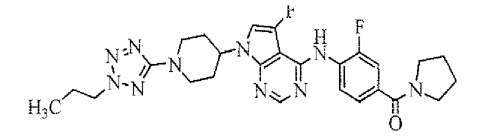 | Powder MS(APCI)m/z: 537 [M+H]⁺ |
| 174 | 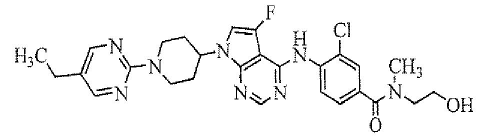 | Powder MS(APCI)m/z: 553/555 [M+H]⁺ |
| 175 | 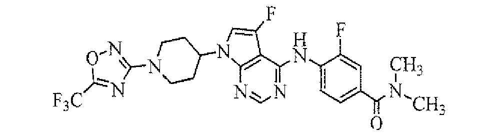 | Powder MS(APCI)m/z: 537 [M+H]⁺ |
| 176 | 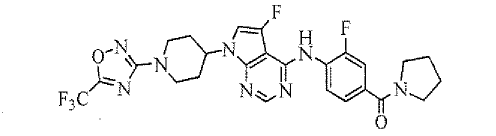 | Powder MS(APCl)m/z: 563 [M+H]+ |
| 177 | 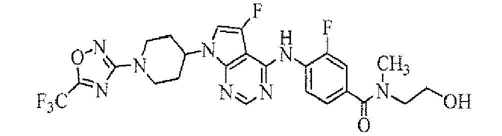 | Powder MS(APCI)m/z: 567 [M+H]+ |
| 178 | 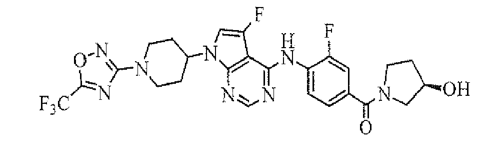 | Powder MS(APCI)m/z: 579 [M+H]+ |

Table 29

**[Table 29]**

| Examples | Structural Formula | Physicochemical properties etc. |
|---|---|---|
| 179 | 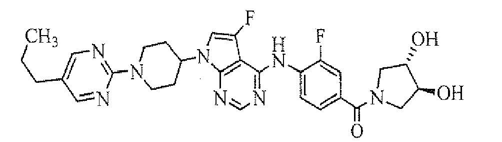 | Powder MS(APCI)m/z: 579 [M+H]⁺ |
| 180 | 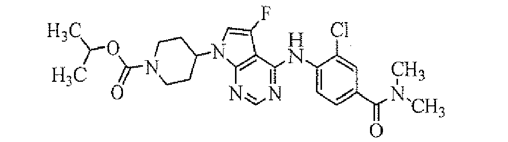 | Powder MS(APCI)m/z: 503/505 [M+H]⁺ |
| 181 | 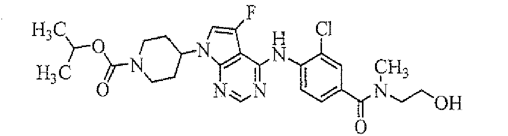 | Powder MS(APCI)m/z: 533/535 [M+H]⁺ |
| 182 | 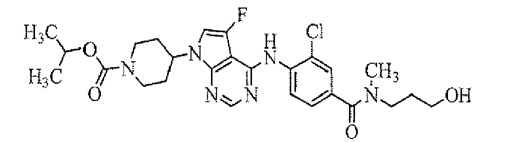 | Powder MS(APCI)m/z: 547/549 [M+H]⁺ |
| 183 | 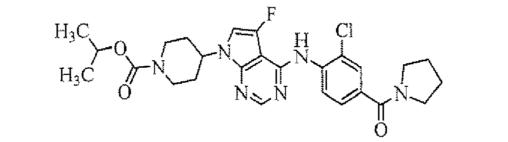 | Powder MS(APCI)m/z: 529/531 [M+H]⁺ |
| 184 | 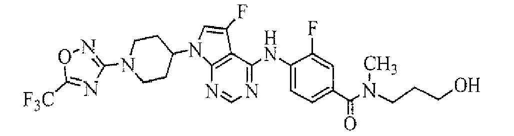 | Viscous oil MS(APCI)m/z: 581 [M+H]+ |
| 185 | 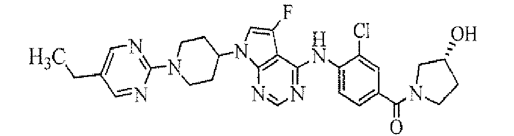 | Powder MS(APCI)m/z: 565/567 [M+H]+ |
| 186 | 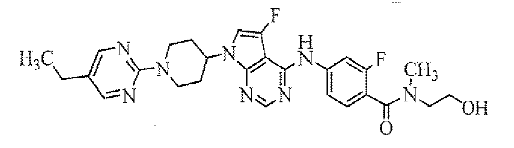 | Powder MS(APCI)m/z: 537 [M+H]+ |

Table 30

**[Table 30]**

| Examples | Structural Formula | Physicochemical properties etc. |
|---|---|---|
| 187 | 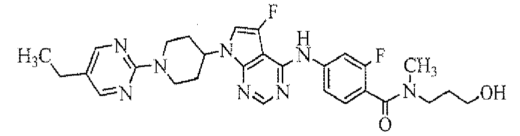 | Powder MS(APCI)m/z: 551 [M+H]⁺ |
| 188 | 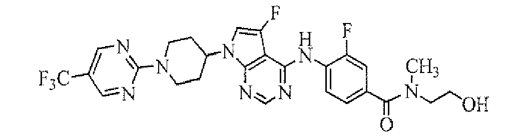 | Powder MS(APCI)m/z: 577 [M+H]⁺ |
| 189 | 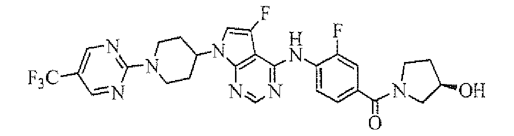 | Powder MS(APCI)m/z: 589 [M+H]⁺ |
| 190 | 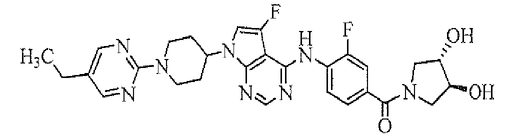 | Powder MS(APCI)m/z: 565 [M+H]⁺ |
| 191 | 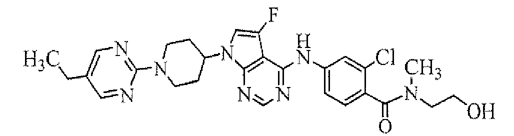 | Powder MS(APCI)m/z: 553/555 [M+H]+ |
| 192 | 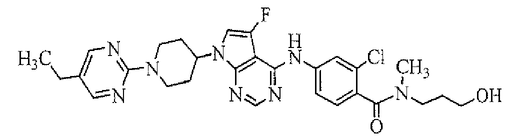 | Powder MS(APCI)m/z: 567/569 [M+H]+ |
| 193 | 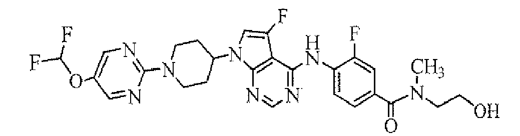 | Powder MS(APCI)m/z: 575[M+H]+ |
| 194 | 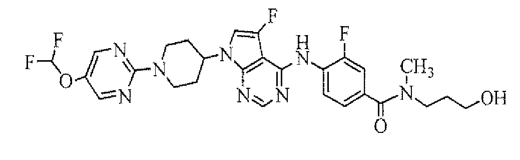 | Powder MS(APCI)m/z: 589 [M+H]+ |

Table 31

**[Table 31]**

| Examples | Structural Formula | Physicochemical properties etc. |
|---|---|---|
| 195 | 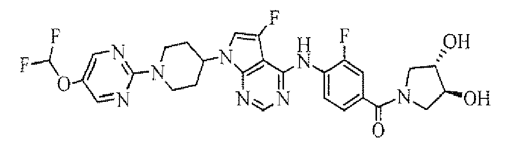 | Powder MS(APCI)m/z: 603 [M+H]⁺ |
| 196 | 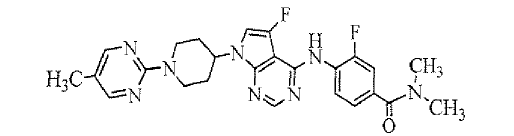 | Powder MS(APCI)m/z: 493 [M+H]⁺ |
| 197 | 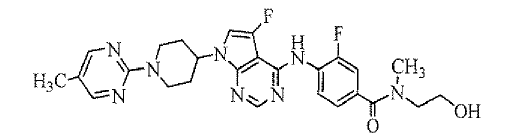 | Powder MS(APCI)m/z: 523 [M+H]⁺ |
| 198 | 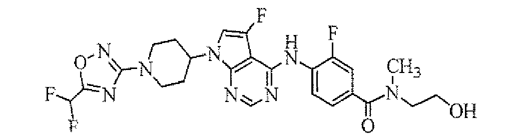 | Powder MS(APCI)m/z: 549 [M+H]⁺ |
| 199 | 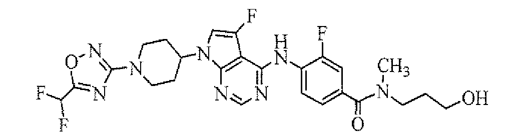 | Powder MS(APCI)m/z: 563 [M+H]+ |
| 200 | 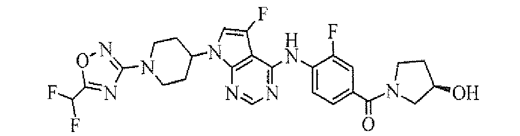 | Powder MS(APCI)m/z: 561 [M+H]+ |
| 201 | 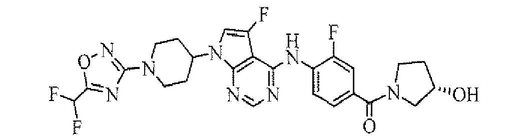 | Powder MS(APCI)m/z: 561 [M+H]+ |
| 202 | 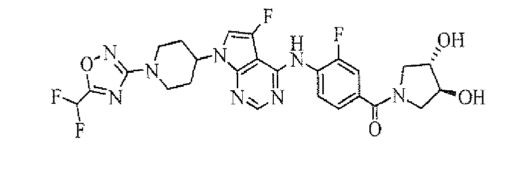 | Powder MS(APCI)m/z: 577 [M+H]+ |

Table 32

**[Table 32]**

| Examples | Structural Formula | Physicochemical properties etc. |
|---|---|---|
| 203 | 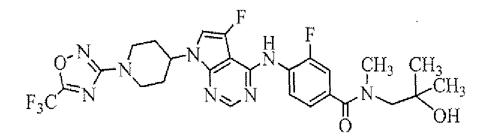 | Powder MS(APCI)m/z: 595 [M+H]⁺ |
| 204 | 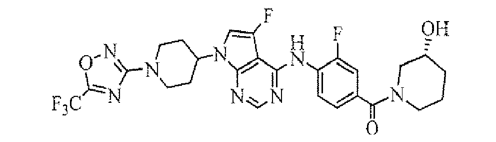 | Powder MS(APCI)m/z: 593 [M+H]⁺ |
| 205 | 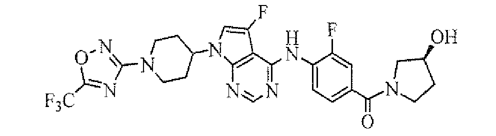 | Powder MS(APCI)m/z: 579 [M+H]⁺ |
| 206 | 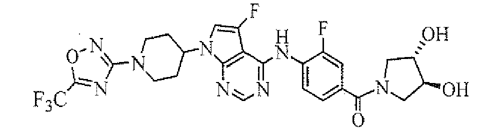 | Powder MS(APCI)m/z: 595 [M+H]⁺ |
| 207 | 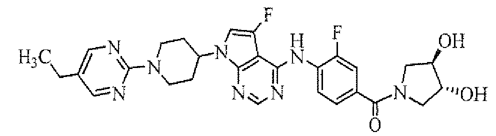 | Powder MS(APCI)m/z: 565 [M+H]+ |
| 208 | 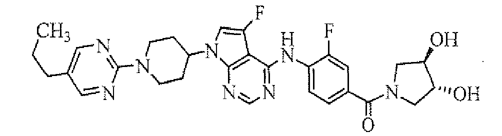 | Powder MS(APCI)m/z: 579 [M+H]+ |
| 209 | 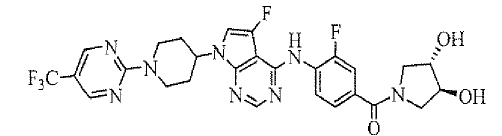 | Powder MS(APCI)m/z: 605 [M+H]+ |
| 210 | 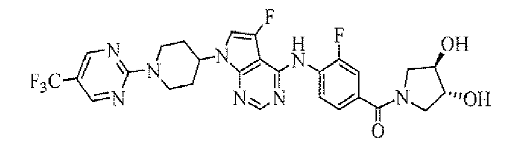 | Powder MS(APCI)m/z: 605 [M+H]+ |

### Examples 211 to 214

Corresponding starting compounds were treated in the similar manner to Example 67 to give compounds of the following Table 33.

Table 33

**[Table 33]**

| Examples | Structural Formula | Physicochemical properties etc. |
|---|---|---|
| 211 | 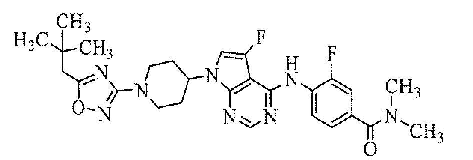 | Powder MS(APCI)m/z: 539 [M+H]⁺ |
| 212 | 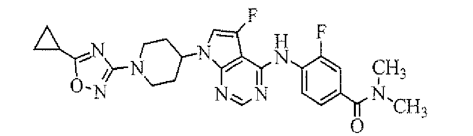 | Powder MS(APCI)m/z: 509 [M+H]⁺ |
| 213 | 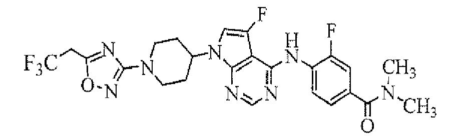 | Powder MS(APCI)m/z: 551 [M+H]⁺ |
| 214 | 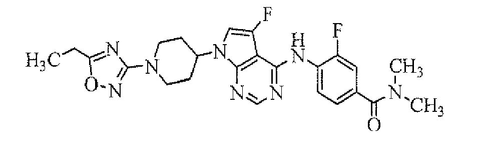 | Powder MS(APCI)m/z: 497 [M+H]⁺ |

### Example 215

Preparation of 4-[7-[1-(3-cyclopropyl-[1,2,4]oxadiazol-5-yl)-piperidin-4-yl]-5-fluoro-7H-pyrrolo[2,3-d]pyrimidin-4-ylamino]-3-fluoro-N,N-dimethylbenzamide 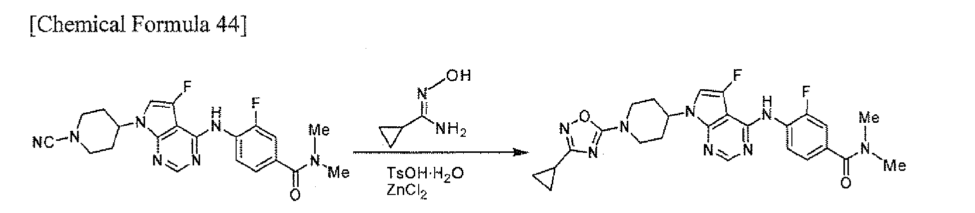 To a solution of cyclopropane carbonitrile (27 mg) in ethanol (4 mL) was added 50% aqueous hydroxylamine solution (53 mg), and the mixture was heated to reflux for 2.5 hours. The solvent was concentrated, and thereto were added DMF (2 mL), 4-[7-(1-cyano-piperidin-4-yl)-5-fluoro-7H-pyrrolo[2,3-d]pyrimidin-4-ylamino]-3-fluoro-N,N-dimethylbenzamide (obtained in Reference Example 55(1); 85 mg), p-toluenesulfonic acid hydrate (15 mg), zinc chloride (11 mg). The mixture was stirred at 90°C for 18 hours. To the reaction mixture was added a saturated aqueous sodium hydrogencarbonate solution, and the mixture was extracted with ethyl acetate. The organic layer was concentrated, and the resulting residue was purified by column chromatography on NH silica-gel (Chromatorex; Fuji Silysia Chemical Ltd., solvent; hexane/ethyl acetate = 80/20 to 30/70) to give the titled compound (47 mg) as a powder (yield 46%).
MS(APCI)m/z; 509[M+H]⁺.

### Examples 216 to 221

Corresponding starting compounds were treated in the similar manner to Example 215 to give compounds of the following Table 34.

Table 34

**[Table 34]**

| Examples | Structural Formula | Physicochemical properties etc. |
|---|---|---|
| 216 | 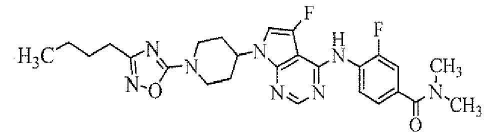 | Powder MS(APCI)m/z: 525 [M+H]⁺ |
| 217 | 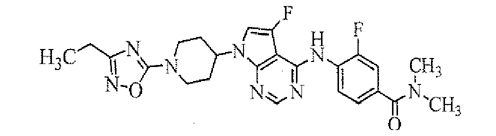 | Powder MS(APCI)m/z: 497 [M+H]⁺ |
| 218 | 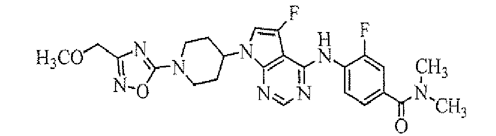 | Powder MS(APCI)m/z: 513 [M+H]⁺ |
| 219 | 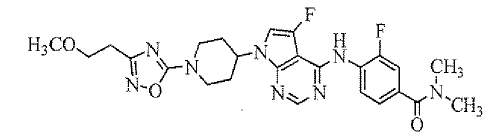 | Powder MS(APCI)m/z: 527 [M+H]⁺ |
| 220 | 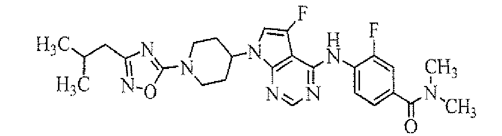 | Powder MS(APCI)m/z: 525 [M+H]⁺ |
| 221 | 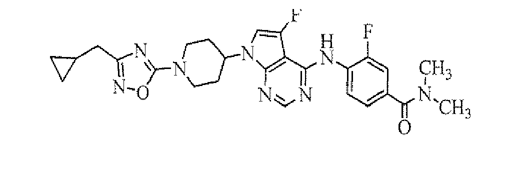 | Powder MS(APCI)m/z: 523 [M+H]⁺ |

### Reference Example 1

### Preparation of tert-butyl 4-(4-chloro-5-fluoropyrrolo[2,3-d]pyrimidin-7-yl)piperidine-1 carboxylate

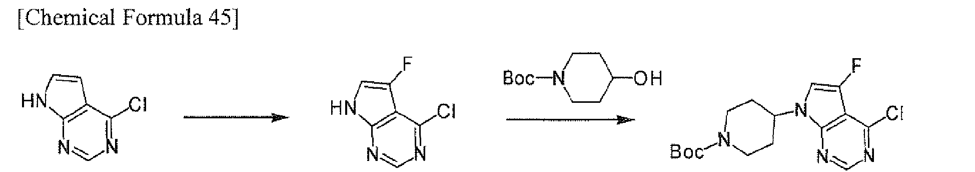

(1) To a solution of 4-chloro-7H-pyrrolo[2,3-d]pyrimidine (2.00 g) in acetonitrile (100 mL) were added acetic acid (20 mL) and N-fluoro-N'-(chloromethyl)triethylenediamine bis(tetrafluoroborate) (6.92 g), and the mixture was stirred under nitrogen atmosphere at 70°C for 18 hours. The reaction mixture was cooled to room temperature, and then concentrated under reduced pressure. To the residue was added methylene chloride/ethyl acetate (1/1), and the solution was passed through a column packed with silica gel (100 mL) and then extracted with methylene chloride/ethyl acetate = 1/1 (2 L). The extract was concentrated, and the resulting residue was purified by column chromatography on silica gel (hexane/ethyl acetate = 70/30 to 35/65) to give 4-chloro-5-fluoro-7H-pyrrolo[2,3-d]pyrimidine (1.30 g) as a powder (yield: 58%).
   MS(APCI)m/z; 172/174[M+H]⁺.
(2) To a solution of the compound (1.20 g) obtained in the above (1) in tetrahydrofuran (215 mL) were added 1-tert-butoxycarbonyl-4-hydroxypiperidine (3.52 g), triphenylphosphine (7.33 g) and a solution of diethyl azodicarboxylate in toluene (12.7 mL), and the mixture was stirred under nitrogen atmosphere at room temperature for 1 hour. The reaction mixture was concentrated under reduced pressure, and the resulting residue was purified by column chromatography on silica gel (solvent; hexane/ethyl acetate = 80/20 to 60/40) to give the titled compound (1.47 g) as a powder (yield: 59%).
   MS(APCI)m/z; 355/357[M+H]⁺.

### Reference Example 2

Preparation of 4-amino-3 -fluoro-N,N-dimethylbenzamide 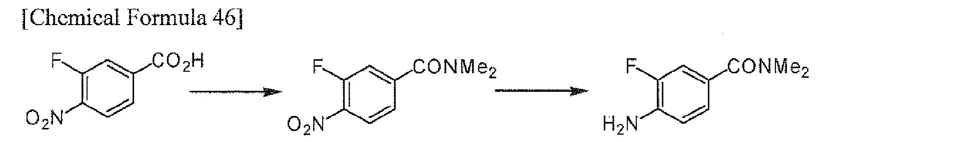
(1) To a solution of 3-fluoro-4-nitrobenzoic acid (4.99 g) in methylene chloride (50 mL) were added under ice-cooling oxalyl chloride (2.5 mL) and one drop of dimethylformamide, and the mixture was stirred at room temperature for 4 hours. The reaction mixture was concentrated under reduced pressure, and to the resulting residue was added methylene chloride (100 mL). Thereto were added dropwise under ice-cooling a solution of dimethylamine hydrochloride (1.98 g) and triethylamine (11.27 mL) in methylene chloride (40 mL), and the mixture was stirred for 1 hour. To the reaction mixture was added water, and the mixture was extracted with chloroform and the organic layer was washed with brine and then dried over magnesium sulfate and filtered. The filtrate was concentrated under reduced pressure, and the resulting residue was purified by column chromatography on silica gel (solvent; hexane:ethyl acetate = 67/33 to 0/100) to give 3-fluoro-4-nitro-N,N-dimethylbenzamide (4.45 g) as a powder (yield: 78%).
   MS(APCI)m/z; 213[M+H]⁺.
(2) To a mixture of the compound (4.45 g) obtained in the above (1), ethanol (80 mL), tetrahydrofuran (80 mL) and water (16 mL) were added ammonium chloride (4.49 g) and iron (4.69 g), and the mixture was stirred at 90°C for 1 hour. The reaction mixture was cooled to room temperature, and then filtered through Celite®. The filtrate was concentrated under reduced pressure, and the resulting residue was purified by column chromatography on silica gel (solvent; hexane/ethyl acetate = 50/50 to 0/100) to give the titled compound (3.71 g) as a powder (yield: 97%).
   MS(APCI)m/z; 183[M+H]⁺.

### Reference Example 3

Preparation of 4-amino-3-fluoro-N-methylbenzamide 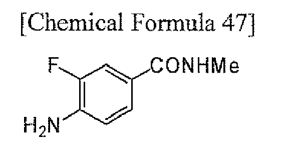 A corresponding starting compound was treated in the similar manner to Reference Example 2 to give the titled compound (yield: 26%).
MS(APCI)m/z; 169[M+H]⁺.

### Reference Example 4

Preparation of 3-fluoro-4-[5-fluoro-7-(piperidin-4-yl)-7H-pyrrolo[2,3-d]pyrimidin-4-ylamino]-N,N-dimethylbenzamide hydrochloride 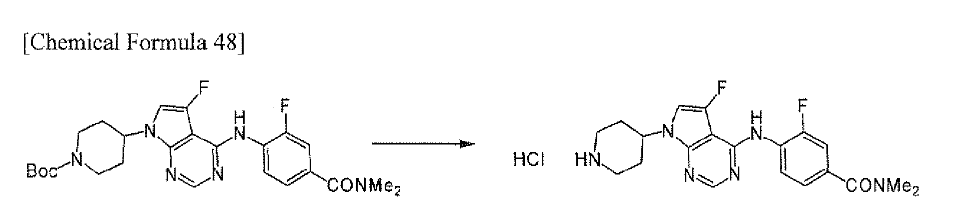 To a solution of the compound (482 mg) obtained in Example 1 in 1,4-dioxane (4 mL) was added 4N hydrochloric acid-dioxane solution (4 mL), and the mixture was stirred at room temperature for 1 hour. To the reaction mixture was added methanol (2 mL), and then the mixture was stirred for another 30 minutes. The reaction mixture was concentrated under reduced pressure, and to the resulting residue was added diethylether. The precipitates were collected by filtration to give the titled compound (540 mg) as a powder (yield: 100%).
MS(APCI)m/z; 401 [M+H]⁺.

### Reference Example 5

Preparation of 3-pentyl 4-nitrophenylcarbonate 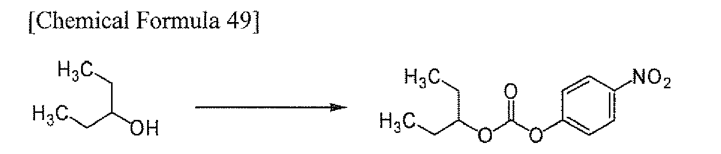 To a solution of 3-pentanol (210 mg) in methylene chloride (5 mL) were added triethylamine (490 µL) and 4-nitrophenyl chloroformate (472 mg), and the mixture was stirred at room temperature for 14 hours. To the reaction mixture was added water, and then the mixture was extracted with chloroform. The organic layer was concentrated, and the resulting residue was purified by column chromatography on silica gel (solvent; hexane/ethyl acetate = 95/5 to 70/30) to give the titled compound (251 mg) as a colorless liquid (yield: 42%).
MS(APCI)m/z; 254[M+H]⁺.

### Reference Example 6

Preparation of (2-cyanoprop-2-yl)-4-nitrophenylcarbonate 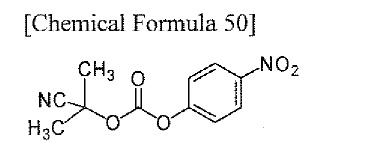 A corresponding starting compound was treated in the similar manner to Reference Example 5 to give the titled compound (yield: 42%).

### Reference Example 7

Preparation of (1,3-difluoroprop-2-ylt4-nitrophenylcarbonate 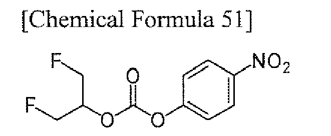 A corresponding starting compound was treated in the similar manner to Reference Example 5 to give the titled compound (yield: 58%).
MS(APCI)m/z; 262[M+H]⁺.

### Reference Example 8

Preparation of tert-butyl 4-(5-bromo-4-chloropyrrolo[2,3-d]pyrimidin-7-yl)piperidine-1-carboxylate 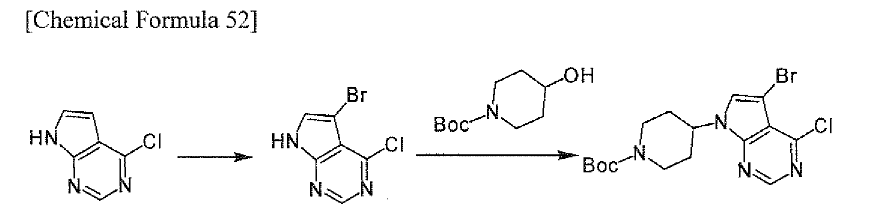
(1) To a solution of 4-chloro-7H-pyrrolo[2,3-d]pyrimidine (3.00 g) in chloroform (85 mL)) was added N-bromosuccinimide (3.55 g), and the mixture was heated to reflux for 1 hour. The reaction mixture was cooled to room temperature, and the precipitates were collected by filtration and purified by column chromatography on silica gel (solvent; hexane/ethyl acetate = 70/30 to 20/80) to give 5-bromo-4-chloro-7H-pyrrolo[2,3-d]pyrimidine (3.83 g) as a colorless powder (yield: 84%).
   MS(APCI)m/z; 232/234[M+H]⁺.
(2) The compound (450 mg) obtained in the above (1) was treated in the similar manner to Reference Example 1(2) to give the titled compound (684 mg) as a colorless powder (yield: 85%).
   MS(APCI)m/z; 415/417[M+H)⁺.

### Reference Example 9

Preparation of 3-fluoro-4-hydroxy-N,N-dimethylbenzamide 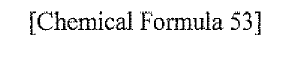 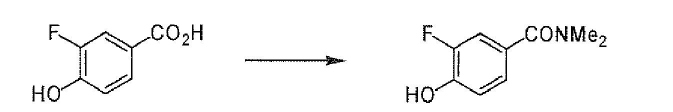 To a solution of 3-fluoro-4-hydroxybenzole acid (1.00 g), dimethylamine hydrochloride (1.57 g), triethylamine (2.68 mL) and N-hydroxybenzotriazole monohydrate (1.47 g) in methylene chloride (20 mL) was added 1-ethyl-3-(3-dimethylaminopropyl)carbodiimide hydrochloride (1.83 g), and the mixture was stirred at room temperature overnight. To the reaction mixture was added diluted hydrochloric acid water, and then the mixture was extracted with chloroform. The organic layer was washed with a saturated aqueous sodium hydrogencarbonate solution and then concentrated under reduced pressure, and the resulting residue was purified by column chromatography on silica gel (solvent; chloroform/methanol = 100/0 to 89/11) to give the titled compound (515 mg) as a colorless solid (yield: 44%).
MS(APCI)m/z; 184[M+H]⁺.

### Reference Example 10

Preparation of 2-fluoro-4-hydroxy-N,N-dimethylbenzamide 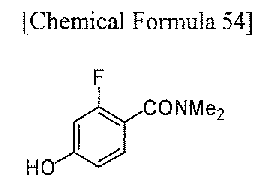 A corresponding starting compound was treated in the similar manner to Reference Example 9 to give the titled compound (yield: 47%).
MS(APCI)m/z; 184[M+H]⁺.

### Reference Example 11

Preparation of 3-chloro-4-hydroxy-N,N-dimethylbenzamide 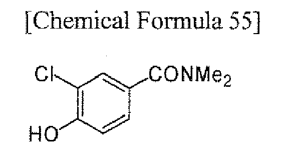 A corresponding starting compound was treated in the similar manner to Reference Example 9 to give the titled compound (yield: 47%).
MS(APCI)m/z; 200/202[M+H]⁺,

### Reference Example 12

Preparation of 4-amino-3-chloro-N,N-dimethylbenzamide 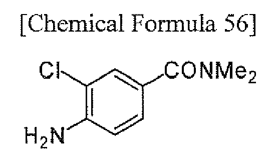 A corresponding starting compound was treated in the similar manner to Reference Example 9 to give the titled compound (yield: 53%).
MS(APCI)m/z; 199/201[M+H]⁺.

### Reference Example 13

Preparation of 1-(3-isopropyl-1,2,4-oxadiazol-5-yl)piperidin-4-ol 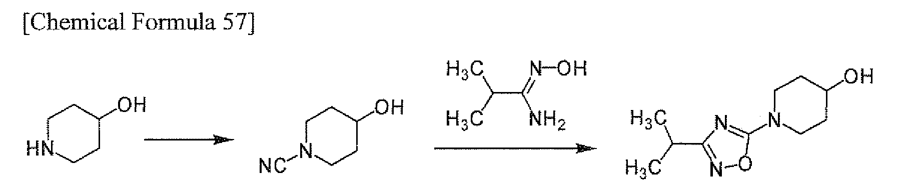
(1) To a solution of 4-hydroxypiperidine (8.00 g) in ethanol (160 mL) were added udder ice-cooling cyanogen bromide (8.38 g) and sodium hydrogencarbonate (20.2 g), and the mixture was stirred at room temperature overnight. The reaction mixture was filtered, and the filtrate was concentrated under reduced pressure. The resulting residue was purified by column chromatography on silica gel (solvent; ethyl acetate) to give 4-hydroxypiperidine-1-carbonitrile (9.59 g) as a pale yellow liquid (yield: 96%).
   MS(APCI)m/z; 127[M+H]⁺.
(2) To a solution of the compound (9.59 g) obtained in the above (1) in ethyl acetate (350 mL) was added N-hydroxy.isobutyramidine (9.79 g), and then thereto was added dropwise 1.0M zinc chloride-diethylether solution (92 mL). The mixture was stirred at room temperature for 1 hour. To the reaction mixture was added diethylether, and the precipitated solid was collected by filtration. To the resulting solid were added ethanol (80 mL) and concentrated hydrochloric acid (40 mL), and the mixture was stirred at 95°C for 1 hour. The reaction mixture was left to be cooled to room temperature, and then the reaction solution was neutralized with an aqueous sodium hydrogencarbonate solution and then extracted with methylene chloride. The organic layer was washed successively with water and brine, and then dried over magnesium sulfate and filtered. The filtrate was concentrated under reduced pressure to give the titled compound (8.76g) as a pale yellow liquid (yield: 54%).
   MS(APCI)m/z; 212[M+H]⁺.

### Reference Example 14

Preparation of 1-(3-tert-butyl-1,2,4-oxadiazol-5-yl)piperidin-4-ol 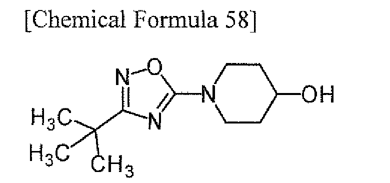 A corresponding starting compound was treated in the similar manner to Reference Example 13 to give the titled compound (yield: 26%).
MS(APCI)m/z; 226[M+H]⁺.

### Reference Example 15

Preparation of 4-chloro-5-fluoro-7-[1-(3-isopropyl-1,2,4-oxadiazol-5-yl)piperidin-4-yl]-7H-pyrrolo[2,3-d]pyrimidine 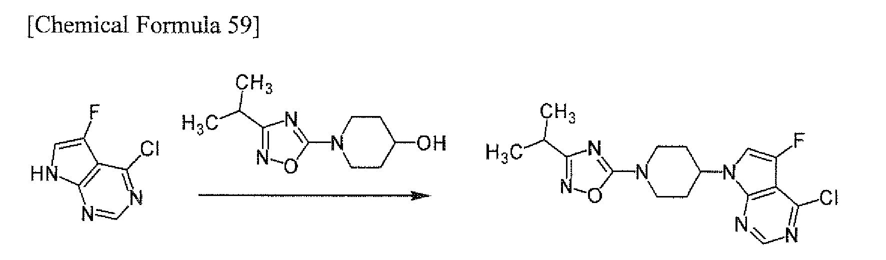 4-Chloro-5-fluoro-7H-pyrrolo[2,3-d]pyrimidine (obtained in Reference Examples 1(1); 300 mg) was treated with the compound (0.92 g) obtained in Reference Example 13 in the similar manner to Reference Example 1(2) to give the titled compound (220 mg) as a colorless powder (yield: 34%).
MS(APCI)m/z; 365/367[M+H]⁺

### Reference Example 16

Preparation of 4-chloro-5-fluoro-7-[1-(3-tert-butyl-1,2,4-oxadiazol-5-yl)piperidin-4-yl]-7H-pyrrolo[2,3-d]pyrimidine 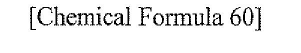 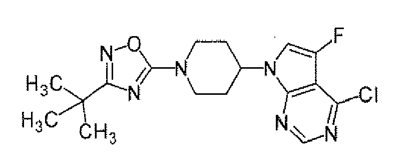 The compound (500 mg) obtained in Reference Example 1(1) was treated with the compound (1.05 g) obtained in Reference Example 14 in the similar manner to Reference Example 1(2) to give the titled compound (yield: 55%).
MS(APCI)m/z; 379/381[M+H]⁺,

### Reference Example 17

Preparation of 4-chloro-5-fluoro-7-[1-(5-cthylpyrimidin-2-yl)piperidin-4-yl]-7H-pyrrolo[2,3-d]pyrimidine 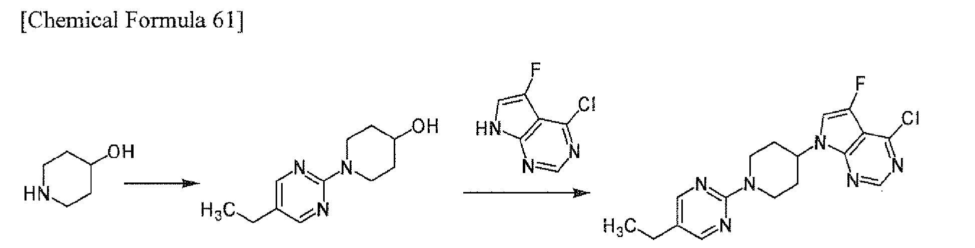
(1) To a solution of 4-hydroxypiperidine (710 mg) in ethanol (5 mL) was added 5-ethyl-2-chloropyrimidine (425 µL), and the mixture was stirred at 80°C overnight. To the reaction mixture was added water, and then the mixture was extracted with ethyl acetate, and the organic layer was dried over magnesium sulfate and then filtered. The filtrate was concentrated under reduced pressure, and the resulting residue was purified by column chromatography on silica gel (solvent; chloroform/methanol = 100/0 to 90/10) to give 1-(5-ethylpyrimidin-2-yl)piperidin-4-ol (699 mg) as a colorless solid (yield: 96%).
   MS(APCI)m/z; 208[M+H]⁺.
(2) The compound (903 mg) obtained in Reference Example 1(1) was treated with the compound (2.18 g) obtained in the above (1) in the similar manner to Reference Example 1(2) to give the titled compound (1.17 g) (yield: 62%).
   MS(APCI)m/z; 361/363[M+H]⁺.

### Reference Example 18

Preparation of 4-chloro-5-fluoro-7-[5-isopropyl-(1,2,4-oxadiazol-3-yl)piperidn-4-yl]-7H-pyrrolo[2,3-d]pyrimidine 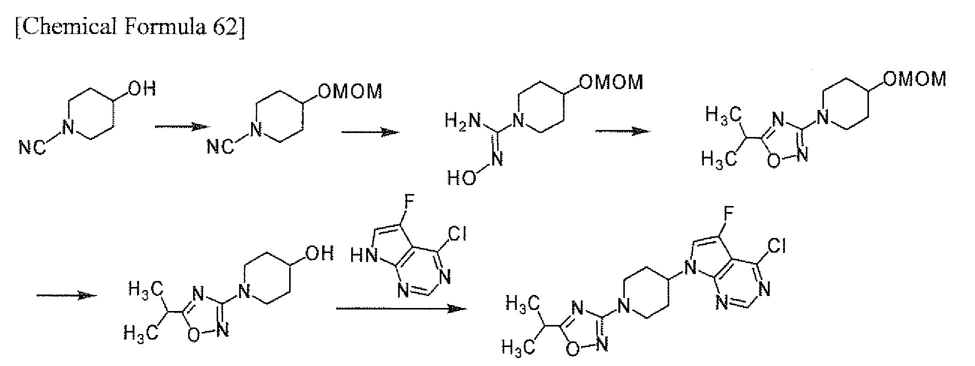
(1) To a solution of 4-hydroxypiperidine-1-carbonitrile (obtained in Reference Example 13(1); 2.00 g) in methylene chloride (40 mL) were added under ice-cooling diisopropylethylamine (5.5 mL) and methoxymethyl chloride (1.80 mL), and the mixture was stirred at room temperature for 19 hours. To the reaction mixture were further added diisopropylethylamine (2.75 mL) and methoxymethyl chloride (0.60 mL), and then the mixture was stirred for 4.5 hours. To the reaction mixture was added water, and then the mixture was extracted with chloroform. The organic layer was washed with brine, dried over magnesium sulfate and then filtered, and the filtrate was concentrated under reduced pressure. The resulting residue was purified by column chromatography on silica gel (solvent; hexane/ethyl acetate = 70/30 to 40/60) to give 4-methoxymethoxypiperidine-e-carbonitrile (2.31 g) as a colorless liquid (yield: 86%).
   MS(APCI)m/z; 171 [M+H]⁺.
(2) To a solution of the compound (2.31 g) obtained in the above (1) in 2-propanol (2 mL) was added a solution of 50% aqueous hydroxylamine solution (1.79 g) in 2-propanol (3 mL), and the mixture was stirred at 90°C for 5 hours. The reaction mixture was left to be cooled to room temperature, and then diluted with ethyl acetate, dried over magnesium sulfate and then filtered. The filtrate was concentrated under reduced pressure to give N-hydroxy-4-methoxymethoxypipcridine-1-carboxamidine (2.93 g) as a colorless liquid (yield: 100%).
   MS(APCI)m/z; 204[M+H]⁺.
(3) To a solution of the compound (2.93 g) obtained in the above (2) and triethylamine (1.89 mL) in toluene (30 mL) was added dropwise under ice-cooling a solution of isobutyryl chloride (1.42 mL) in toluene (10 mL), and then the mixture was stirred at 130°C for 3 hours. To the reaction mixture was added water, and then the mixture was extracted with ethyl acetate. The organic layer was washed with brine, dried over magnesium sulfate, and then filtered. The filtrate was concentrated under reduced pressure, and the resulting residue was purified by column chromatography on silica gel (solvent; hexane/ethyl acetate = 95/5 to 80/20) to give 1-(5-isopropyl-1,2,4-oxadiazol-3-yl)-4-methoxymethoxypiperidine (1.86 g) as a colorless liquid (yield: 54%).
   MS(APCI)m/z; 256[M+H]⁺.
(4) To a solution of the compound (1.86 g) obtained in the above (3) in 1,4-dioxane (10 mL) was added 4N hydrochloric acid-dioxane (5 mL), and the mixture was stirred at room temperature for 3 hours. To the reaction mixture was added additional 4N hydrochloric acid-dioxane (1 mL), and then the mixture was stirred for 1 hour. The reaction mixture was concentrated under reduced pressure, and to the resulting residue was added a saturated aqueous sodium hydrogencarbonate solution, and the mixture was extracted with chloroform. The organic layer was dried over magnesium sulfate and then filtered, and the filtrate was concentrated under reduced pressure to give 1-(5-isopropyl-1,2,4-oxadiazol-3-yl)piperidin-4-ol (1.60 g) as a colorless liquid (yield: 100%).
   MS(APCI)m/z; 212[M+H]⁺.
(5) The compound (500 mg) obtained in Reference Example 1(1) was treated with the compound (1.60 g) obtained in the above (4) in the similar manner to Reference Example 1(2) to give the titled compound (505 mg) (yield: 48%).
   MS(APCI)m/z; 365/367[M+H]⁺

### Reference Example 19

Preparation of 4-chloro-5-fluoro-7-[1-(5-methylpyridin-2-yl)piperidin-4-yl]-7H-pyrrolo[2,3-d]pyrimidine 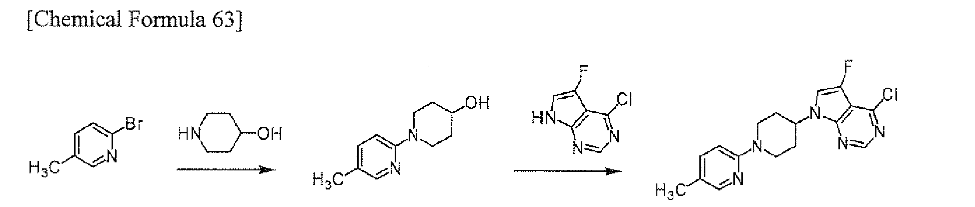
(1) To a solution of 4-hydroxypiperidine (1.42 g) in N-methylpyrrolidone (12 mL) were added 2-bromo-5-methylpyridine (1.20 g) and diisopropylethylamine (3.67 mL), and the mixture was stirred in a microwave reactor (Initiator, manufactured by Biotage Inc.) at 200°C for 1 hour. To the reaction mixture was added water, and then the mixture was extracted with ethyl acetate. The organic layer was washed with water and brine, and then dried over magnesium sulfate and filtered. The filtrate was concentrated under reduced pressure, and the resulting residue was purified by column chromatography on silica gel (solvent; chloroform/methanol = 100/0 to 93/7) to give 1-(5-methylpyridin-2-yl)piperidin-4-ol (0.81 g) as a pale brown solid (yield: 60%).
   MS(APCI)m/z; 193[M+H]⁺,
(2) The compound (250 mg) obtained in Reference Example 1(1) was treated with the compound (476 mg) obtained in the above (2) in the similar manner to Reference Example 1(2) to give the titled compound (363 mg) (yield: 72%).
   MS(APCI)m/z; 346/348[M+H]⁺.

### Reference Example 20

Preparation of 4-chloro-5-fluoro-7-[1-(5-ethyl-1 ,3,4-thiadiazol-2-yl)piperidin-4-yl]-7H-pyrrolo[2,3-d]pyrimidine 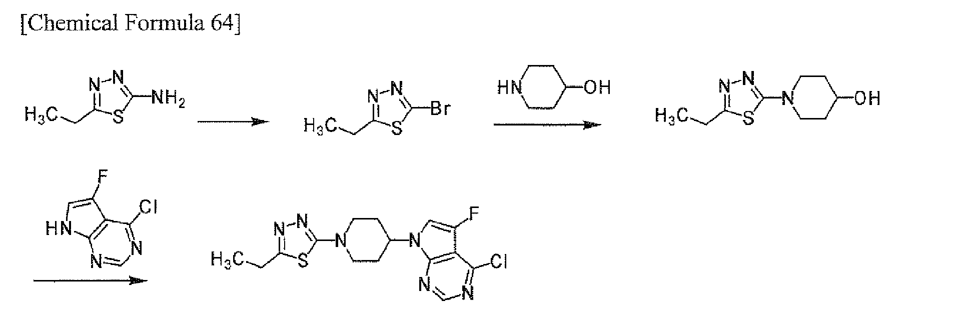
(1) To a solution of 2-amino-5-ethyl-1,3,4-thiadiazole (1.00 g) in acetonitrile (20 mL)/dimethylacetamide (20 mL) were added copper (II) bromide (2.07 g) and n-amyl nitrite (1.40 mL), and the mixture was stirred at room temperature for 2 hours. The reaction mixture was concentrated under reduced pressure, and to the residue was added a saturated aqueous ammonium chloride solution, and then the mixture was extracted with ethyl acetate. The organic layer was washed successively with water and brine, dried over magnesium sulfate and then filtered. The filtrate was concentrated under reduced pressure, and the resulting residue was purified by column chromatography on silica gel (solvent; hexane/ethyl acetate = 95/5 to 80/20) to give 2-bromo-5-ethyl-1,3,4-thiadiazole (0.75 g) as a colorless liquid (yield: 50%).
   MS(APCI)m/z; 193/195[M+H]⁺.
(2) To a solution of the compound (640 mg) obtained in the above (1) in ethanol (5 mL) was added 4-hydroxypiperidine (671 mg), and the mixture was stirred in a microwave reactor (Initiator, manufactured by Biotage Inc.) at 140°C for 1 hour. The reaction mixture was concentrated under reduced pressure, and the resulting residue was purified by column chromatography on silica gel (solvent; chloroform/methanol = 100/0 to 93/7) to give 1-(5-ethyl-1,3,4-thiadiazol-2-yl)piperidin-4-ol (707 mg) as a colorless liquid (yield 100%). MS(APCI)m/z; 214[M+H]⁺.
(3) The compound (200 mg) obtained in Reference Example 1(1) was treated with the compound (373 mg) obtained in the above (2) in the similar manner to Reference Example 1(2) to give the titled compound (86 mg) (yield: 20%).
   MS(APCI)m/z; 367/369[M+H]⁺.

### Reference Example 21

Preparation of 2-chloro-5-ethoxypyrimidine 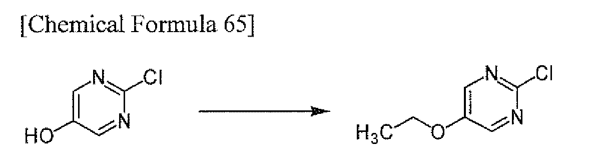 To a solution of 2-chloro-5-hydroxypyrimidine (1.00 g) in dimethylformamide (15 mL) were added potassium carbonate (1.59 g) and ethyl iodide (1.84 mL), and the mixture was stirred at 50°C for 1 hour. To the reaction mixture was added water, and then the mixture was extracted with ethyl acetate. The organic layer was washed with water and brine, and then dried over magnesium sulfate and filtered. The filtrate was concentrated under reduced pressure, and the resulting residue was purified by column chromatography on silica gel (solvent; hexane/ethyl acetate = 99/1 to 78/22) to give the titled compound (1.07 g) as a colorless solid (yield: 88%).
MS(APCI)m/z; 159/161[M+H]⁺.

### Reference Example 22

Preparation of 2-chloro-5-isopropyloxypyrimidine 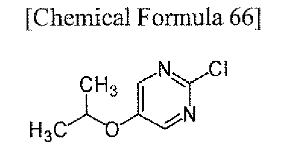 A corresponding starting compound was treated in the similar manner to Reference Example 21 to give the titled compound (yield: 89%).
MS(APCI)m/z; 173/175[M+H]⁺.

### Reference Example 23

Preparation of tert-butyl 4-amino-3-fluorobenzoate 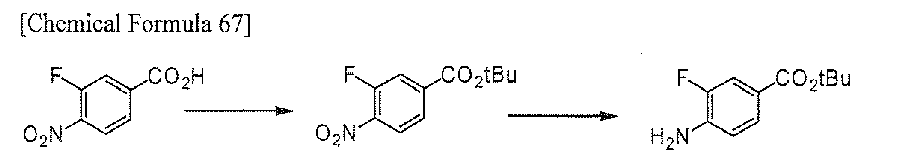
(1) To a solution of 3-fluoro-4-nitrobenzoic acid (2.00 g) in methylene chloride (32 mL) were added under ice-cooling tert-butanol (4.2 mL), 4-dimethylaminopyridine (198 mg) and 1-ethyl-3-(3-dimethylaminopropyl)carbodiimide hydrochloride (2.47 g), and the mixture was stirred at room temperature for 16 hours. To the reaction mixture was added a saturated aqueous sodium hydrogencarbonate solution, and the mixture was extracted with chloroform. The organic layer was distilled under reduced pressure, and the resulting residue was purified by column chromatography on silica gel (solvent; hexane/ethyl acetate = 99/1 to 92/8) to give tert-butyl 3-fluoro-4-nitrobenzoate (1.94 g) as a pale yellow powder (yield 75%).
(2) The compound (1.74 g) obtained in the above (1) was treated in the similar manner to Reference Example 2(2) to give the titled compound (1.42 g) as a colorless powder (yield: 88%).
   MS(APCI)m/z; 212[M+H]⁺.

### Reference Example 24

Preparation of 4-[7-[1-(5-ethylpyrimidin-2-yl)piperidin-4-yl]-5-fluoro-7H-pyrrolo[2,3-d]pyrimidin-4-ylamino]3-fluorobezoic acid dihydrochloride 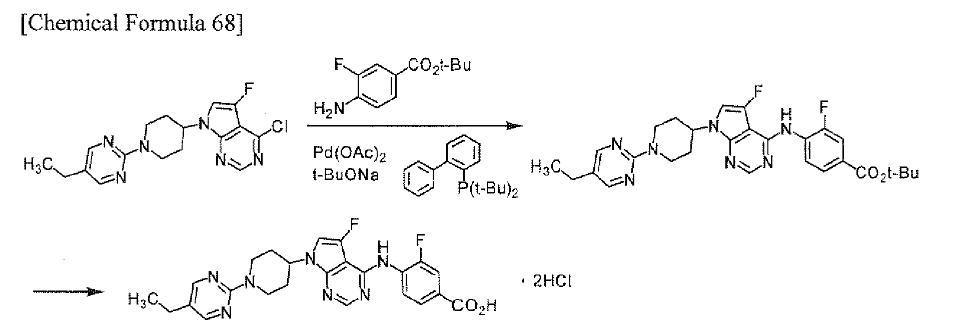
(1) The compound (400 mg) obtained in Reference Example 17 was treated with tert-butyl 4-amino-3-fluorobenzoate (258 mg) in the similar manner to Example 1 to give tert-butyl 4-[7-[1-(5-ethylpyrimidin-2-yl)piperidin-4-yl]-5-fluoro-7H-pyrrolo[2,3-d]pyrimidin-4-ylamino]-3-fluorobenzoate (286 mg) as a colorless powder (yield: 48%).
   MS(APCI)m/z; 536[M+H]⁺.
(2) To a solution of the compound (300 mg) obtained in the above (1) in methylene chloride (4 mL) was added 4N hydrochloric acid-dioxane (3 mL), and the mixture was stirred at room temperature for 18 hours. To the reaction mixture was added additional 4N hydrochloric acid-dioxane (2 mL), and then the mixture was stirred for 7 hours. The reaction mixture was concentrated under reduced pressure to give the titled compound (340 mg) as a crude produet.
   MS(APCI)m/z; 480[M+H]⁺.

### Reference Example 25

Preparation of isopropyl 4-(4-chloro-3-cyano-2-formimidoylaminopyrrol-1-yl)piperidine-l-carboxylate 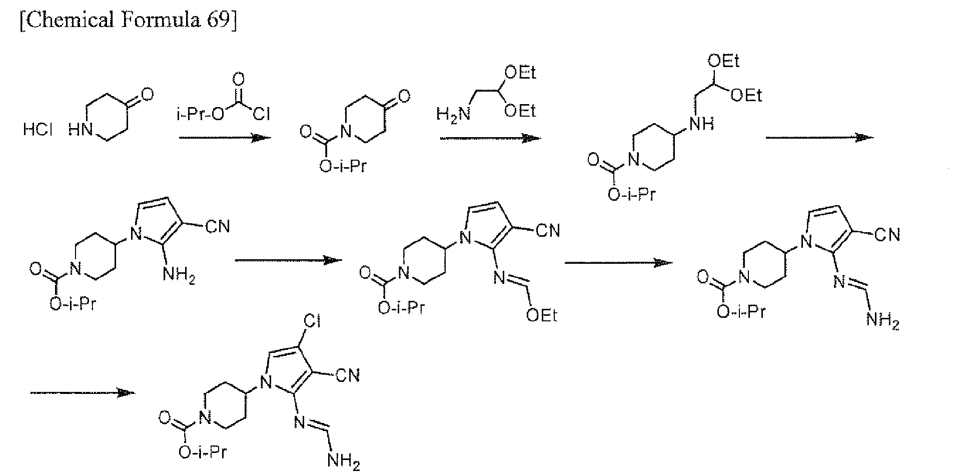
(1) To a solution of 4-piperidone hydrochloride monohydrate (5.00 g) in methylene chloride (100 mL) were added dropwise under ice-cooling triethylamine (11.3 mL) and isopropyl chlorocarbonate (6.1 mL), and the mixture was stirred at room temperature for 1 hour. The reaction mixture was poured into water, and the mixture was extracted with chloroform. The extract was dried over magnesium sulfate and then filtered, and the filtrate was concentrated under reduced pressure. The resulting residue was purified by column chromatography on silica gel (solvent; ethyl acetate/hexane = 50/50→33/67) to give 4-oxopiperidine-1-carboxylic acid isopropyl ester (3.05 g) as a liquid (yield: 50%).
   MS(APCI)m/z; 186[M+H]⁺.
(2) To a solution of the compound (3.05 g) obtained in the above (1) in methylene chloride (200 mL) were added at room temperature aminoacetaldehyde diethyl acetal (2.74 g) and acetic acid (1.2 mL), and the mixture was stirred at the same temperature for 1 hour. To the reaction mixture was added sodium triacetoxyborohydride (4.36 g), and the mixture was stirred at room temperature overnight. The reaction mixture was poured into a saturated aqueous sodium hydrogencarbonate solution, and the mixture was extracted with chloroform. The extract was dried over magnesium sulfate and then filtered, and the filtrate was concentrated under reduced pressure to give isopropyl 4-(2,2-diethoxyethylaminopiperidine-1-carboxylate (6.35 g) as a liquid.
   MS(APCI)m/z; 303[M+H]⁺.
(3) To a solution of the compound (6.35 g) obtained in the above (2) in methylene chloride (150 mL) were added at room temperature malononitrile (2.17 g) and p-toluenesulfonic acid monohydrate (6.26 g), and the mixture was stirred at room temperature overnight. The reaction solution was poured into a saturated aqueous sodium hydrogencarbonate solution, and the mixture was extracted with chloroform. The extract was dried over magnesium sulfate and then filtered, and the filtrate was concentrated under reduced pressure. The resulting residue was purified by column chromatography on silica gel (solvent; ethyl acetate/hexane = 50/50→67/33) to give isopropyl 4-(2-amino-3-cyanopyrrol-1-yl)piperidine-1-carboxylate (2.30 g) as a solid (overall yields of steps (2) through (3): 50%).
   MS(APCI)m_{/}z; 277[M+H]⁺.
(4) To a solution of the compound (0.50 g) obtained in the above (3) in acetonitrile (3 mL) were added at room temperature triethyl orthoformate (0.80 g) and acetic acid (0.11 1 g), and the mixture was stirred at 80°C for 2 hours. The reaction mixture was cooled to room temperature and then concentrated under reduced pressure, and the resulting residue was purified by column chromatography on silica gel (solvent; ethyl acetate/hexane = 50/50→67/33) to give isopropyl 4-(3-cyano-2-ethoxymethyleneaminopyrrol-1-yl)piperidine-1-carboxylate (641 mg) as an oil.
   MS(APCI)m/z; 333 [M+H]⁺.
(5) To a solution of the compound (641 mg) obtained in the above (4) in methanol (2.5 mL) was added at room temperature 7N ammonia-methanol solution, and the mixture was stirred at the same temperature overnight. The precipitates were collected by filtration and dried to give isopropyl 4-(3-cyano-2-fomnimidoylaminopyrrol-1-yl)piperidine-1-carboxylate (220 mg) as a solid (overall yields of steps (4) through (5): 38%).
   MS(APCI)m/z; 304[M+H]⁺.
(6) To a solution of the compound (303 mg) obtained in the above (5) in acetonitrile (10 mL) was added at room temperature N-chlorosuccinimide (161 mg), and the mixture was stirred overnight. The reaction mixture was poured into a saturated aqueous sodium hydro gen carboante solution, and the mixture was extracted with ethyl acetate three times. The organic layer was dried over magnesium sulfate and then filtered, and the filtrate was concentrated under reduced pressure. The resulting residue was purified by column chromatography on silica gel (solvent; ethyl acetate/hexane = 35/65→55/45) to give the titled compound (197 mg) as a powder (yield: 58%).
   MS(APCI) 308[M+H]⁺.

### Reference Example 26

Preparation of 2-fluoro-4-(1,2,4-triazol-1-yl)phenylamine 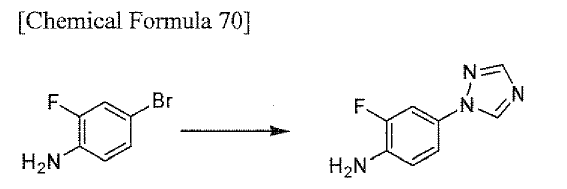 To a solution of 4-bromo-2-fluoroaniline (570 mg) in N-methylpiperidone (4 mL) were added at room temperature 1,2,4-triazole (414 mg), copper (I) iodide (37 mg) and potassium carbonate (829 mg), and the mixture was stirred in a microwave reactor (Initiator, manufactured by Biotage Inc.) at 195°C for 3 hours. The reaction mixture was cooled to room temperature and then poured into water, and the mixture was extracted with ethyl acetate. The extract was dried over magnesium sulfate and then filtered, and the filtrate was concentrated under reduced pressure. The resulting residue was purified by column chromatography on silica gel (solvent; ethyl acetate/hexane = 70/30→100/0) to give the titled compound (323.4 mg) as a solid (yield: 61%).
MS(APCI)m/z; 179[M+H]⁺.

### Reference Example 27

Preparation of 2-fluoro-4-methanesulfonylphenol 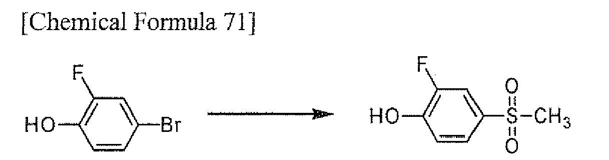 To a solution of 4-bromo-2-fluorophenol (5.0 g) in dimethylsulfoxide (25 mL) were added sodium methanesulfinate (10.69 g), copper (I) trifluoromethanesulfoniate benzene complex (1.19 g) and N,N'-dimethylethylenediamine (560 µL), and the mixture was stirred under nitrogen atmosphere at 140°C for 21 hours. The reaction mixture was cooled to room temperature, and thereto was added water. Then, the mixture was extracted with ethyl acetate. The organic layer was washed successively with water and brine, dried over magnesium sulfate, and then filtered. The filtrate was concentrated, and the resulting residue was purified by column chromatography on silica gel (solvent; hexane/ethyl acetate = 75/25 to 20/80), and then triturated with hexane to give the titled compound (2.32 g) as a colorless solid (yield: 47%). MS(APCI)m/z; 191 [M+H]⁺.

### Reference Example 28

Preparation of 5,6-dichloro-N,N-dimethylnicotinamide 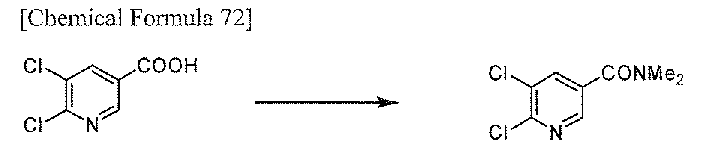 To a suspension of 5,6-dichloronicotinic acid (0.96 g) in methylene chloride (10 mL) was added at room temperature carbonyldiimidazole (0.97 g), and the mixture was stirred for 1 hour. To the reaction mixture was added 2.0N dimethylamine-tetrahydrofuran solution (5.0 mL) at room temperature, and the mixture was stirred overnight. The reaction mixture was poured into a saturated aqueous sodium hydrogencarbonate solution, and extracted with ethyl acetate. The organic layer was washed successively with water and brine, and then dried over magnesium sulfate and filtered. The filtrate was concentrated under reduced pressure, and the resulting residue was purified by column chromatography on silica gel (solvent; hexane/ethyl acetate = 30/70 to 90/10) to give the titled compound (198 mg) as a colorless solid (yield: 18%). MS(APCI)m/z; 219/221[M+H]⁺.

### Reference Example 29

Preparation of 5-ethyl-2-fluoropyridine 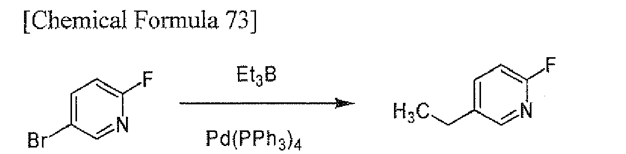 To a solution of 5-bromo-2-fluoropyridine (5.00 g) in dimethylformamide (75 mL) were added 1.0M triethylborane-tetrahydrofuran solution (43 mL), potassium carbonate (15.70 g) and tetrakistriphenylphosphine palladium (1.64 g), and the mixture was stirred under nitrogen atmosphere at 85°C for 4 hours. To the reaction mixture was added water, and then the mixture was extracted with hexane. The organic layer was washed successively with water and brine, and then magnesium sulfate and filtered. The filtrate was concentrated under reduced pressure, and the resulting residue was purified by column chromatography on silica gel (solvent; hexane/methylene chloride = 1/1) to give the titled compound (1.89 g) as a pale yellow liquid (yield: 53%).
MS(APCI)m/z; 126[M+H]⁺.

### Reference Example 30

Preparation of 4-chloro-5-fluoro-7-[1-(5-ethylpyridin-2-yl)piperidin-4-yl]-7H-pyrrolo[2,3-d]pyrimidine 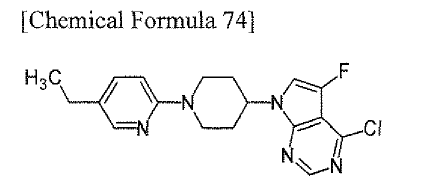 The compound obtained in Reference Example 29 was treated in the similar manner to Reference Example 19 to give the titled compound.
MS(APCI)m/z; 360/362[M+H]⁺.

### Reference Example 31

Preparation of 7-(1-tert-butoxycarbonylpiperidin-4-yl)-4-chloro-7H-pyrrolo[2,3-d]pyrimidine-5-carboxylic acid ethyl ester 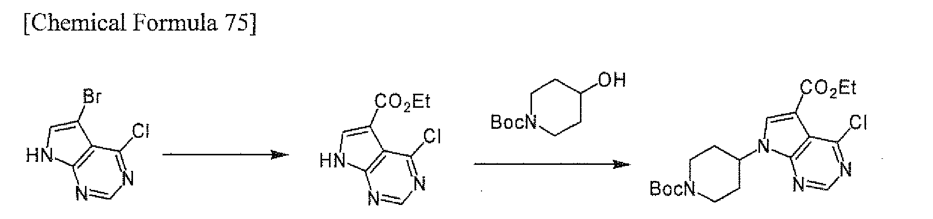
(1) To a solution of 5-bromo-4-chloro-7H-pyrrolo[2,3-d]pyrimidine (obtained in Reference Example 8(1); 2.00 g) in tetrahydrofuran (50 mL) was added dropwise under nitrogen atmosphere at -65°C 2.64M butyllithium-hexane solution (7.2 mL), and then the mixture was stirred for 30 minutes. To the reaction mixture was added a solution of ethyl chloroformate (905 µL) in tetrahydrofuran (5 mL), and the mixture was stirred at room temperature overnight. To the reaction mixture was added a saturated aqueous ammonium chloride solution, and then the mixture was extracted with ethyl acetate. The organic layer was dried over magnesium sulfate and then filtered, and the filtrate was concentrated under reduced pressure. The resulting residue was purified by column chromatography on silica gel (solvent; hexane/ethyl acetate = 65/35 to 20/80) to give 4-chloro-7H-pyrrolo[2,3-d]pyrimidine-5-carboxylic acid ethyl ester (1.43 g) as a colorless solid (yield: 74%).
   MS(APCI)m/z; 226/228[M+H]⁺.
(2) The compound (424 mg) obtained in the above (1) was treated in the similar manner to Reference Example 1(2) to give the titled compound (655 mg) as a colorless powder (yield 85%).
   MS(APCI)m/z; 409/411 [M+H]⁺.

### Reference Example 32

Preparation of 4-amino-2-fluoro-N,N-dimethylbenzamide 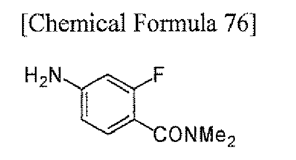 A corresponding starting compound was treated in the similar manner to Reference Example 9 to give the titled compound (yield: 31%).
MS(APCI)m/z; 183[M+H]⁺.

### Reference Example 33

Preparation of 1-(3-n-propyl-1,2,4-oxadiazol-5-yl)piperidin-4-ol 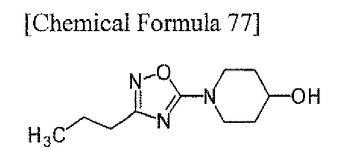 A corresponding starting compound was treated in the similar manner to Reference Example 13 to give the titled compound (yield: 47%).
MS(APCI)m/z; 212[M+H]⁺.

### Reference Example 34

Preparation of 4-chloro-5-fluoro-7-[1-(3-n-propyl-1,2,4-oxadiazol-5-yl)piperidin-4-yl]-7H-pyrrolo[2,3-d]pyrimidine 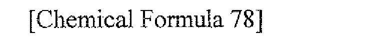 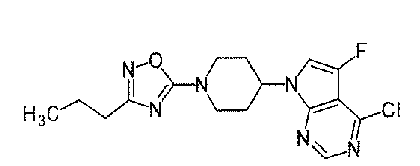 The compound (640 mg) obtained in Reference Example 1(1) was treated with the compound (1.57 g) obtained in Reference Example 33 in the similar manner to Reference Example 1(2) to give the titled compound (yield: 44%).
MS(APCI)m/z; 365/367[M+H]⁺.

### Reference Example 35 5

Preparation of 1-(5-isopropenylpyrimidin-2-yl)piperdin-4-ol 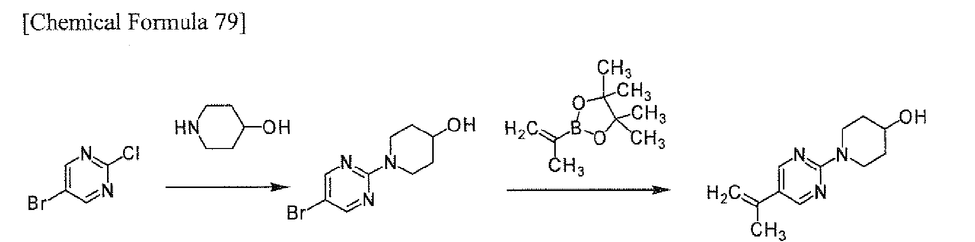
(1) 5-Bromo-2-chloropyrimidine (5.8 g) was treated in the similar manner to Reference Example 17(1) to give 1-(5-bromopyrimidin-2-yl)piperidin-4-ol (7.8 g) as a powder (yield: 100%).
   MS(APCI)m/z; 258/260[M+H]⁺.
(2) To a mixed solution of the compound (4 g) obtained in the above (1) in 1,4-dioxane (160 mL) and water (40 mL) were added cesium carbonate (10.1 g), isopropenylboronic acid pinacol ester (3.5 mL) and tetrakis-triphenylphosphine palladium (895 mg), and the mixture was stirred at 80°C for 5 hours. The reaction mixture was poured into water, and the mixture was extracted with ethyl acetate three times. The organic layer was dried over magnesium sulfate and then concentrated, and the resulting residue was purified by column chromatography on silica gel (solvent; hexane/ethyl acetate = 70/30 to 10/90) to give the titled compound (3.4 g) as a powder (yield: 100%).
   MS(APCI)m/z; 220[M+H]⁺.

### Reference Example 36

Preparation of 4-chloro-5-fluoro-7-[1-(5-isopropylpyrimidin-2-yl)piperidin-4-yl]-7H-pyrrolo[2,3-d]pyrimidine 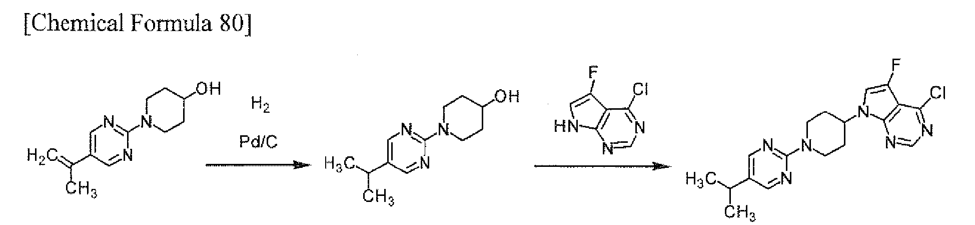
(1) A mixture of the compound (1.5 g) obtained in Reference Example 35, 10% palladium carbon (700 mg) and methanol (70 mL) was stirred under nitrogen atmosphere at room temperature for 20 hours. The reaction mixture was filtered, and the filtrate was concentrated under reduced pressure to give 1-(5-isopropylpyrimidin-2-yl)piperidin-4-ol (1.6 g) as a crude product.
(2) The compound (1.6 g) obtained in the above (1) was treated in the similar manner to Reference Example 1(2) to give the titled compound (1.24 g) as a powder (yield: 48%). MS(APCI)m/z; 375/377[M+H]⁺.

### Reference Example 37

Preparation of 1-(5-cyclopropylpyrimidin-2-yl)piperidin-4-ol 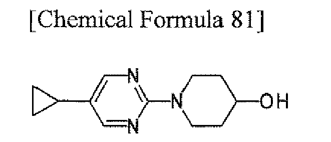 The compound (2 g) obtained in Reference Example 35(1) was treated with cyclopropylboronic acid pinacol ester (1.56 g) in the similar manner to Reference Example 35(2) to give the titled compound (377 mg; yield: 22%).
MS(APCI)m/z; 220[M+H]⁺.

### Reference Example 38

Preparation of 4-chloro-7-[1-(5-cyclopropylpyrimidin-2-yl)piperidin-4-yl]-5-fluoro-7H-pyrrolo[2,3-d]pyrimidine 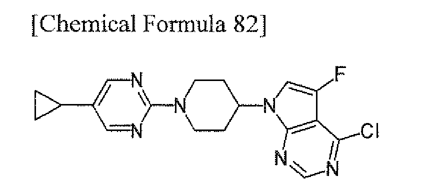 The compound (570 mg) obtained in Reference Example 1(1) was treated with the compound (800 mg) obtained in Reference Example 37 in the similar manner to Reference Example 1(2) to give the titled compound (770 mg; yield: 57%).
MS(APCI)m/z; 375/377[M+H]⁺.

### Reference Example 39

Preparation of 4-chloro-7-[1-(5-chloropyrimidin-2-yl)piperidin-4-yl]-5-fluoro-7H-pyrrolo[2,3-d]pyrimidine 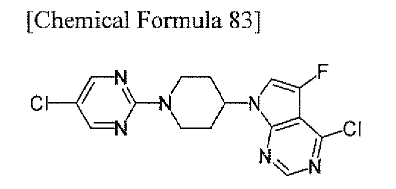 A corresponding starting compound was treated in the similar manner to Reference Example 17 to give the titled compound (yield: 68%).
MS(APCI)m/z; 367/369[M+H]⁺.

### Reference Example 40

Preparation of isopropyl 4-(4-chloro-5-fluoropyrrolo[2,3-d]pyrimidin-7-yl)piperidine-1-carboxylate 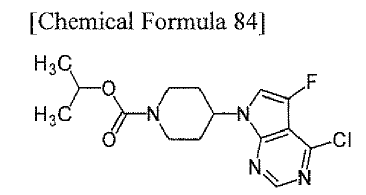 A corresponding starting compound was treated in the similar manner to Reference Example 1(2) to give the titled compound (yield: 45%).
MS(APCI)m/z; 341/343[M+H]⁺.

### Reference Example 41

Preparation of 4,5-dichloro-7-[1-(5-ethylpyrimidin-2-yl)prperidin-4-yl]-7H-pyrrolo[2,3-d]pyrimidine 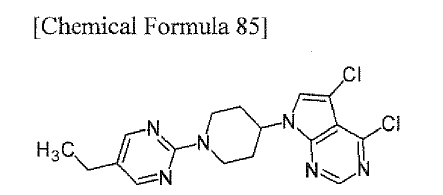 A corresponding starting compound was treated in the similar manner to Reference Example 1(2) to give the titled compound (yield: 62%).
MS(APCI)m/z; 377/379[M+H]⁺.

### Reference Example 42

Preparation of 4-[5-chloro-7-[1-(5-ethylpyrimidin-2-yl)piperidin-4-yl]-7H-pyrrolo[2,3-d]pyrimidin-4-ylamino]-3-fluorobenzoic acid hydrochloride 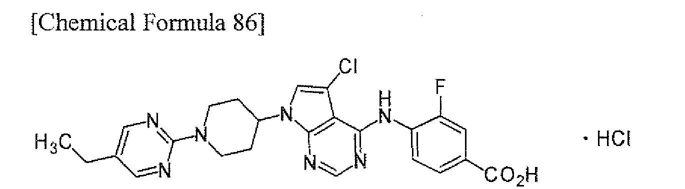 A corresponding starting compound was treated in the similar manner to Reference Example 24 to give the titled compound (yield: 35%).
MS(APCI)m/z; 552/554[M+H]⁺.

### Reference Example 43

Preparation of 3-fluoro-4-[5-fluoro-7-[1-(5-isopropyl-[1,2,4]oxadiazol-3-yl)piperidin-4-yl]-7H-pyrrolo[2,3-d]pyrimidin-4-ylamino]benzoic acid hydrochloride 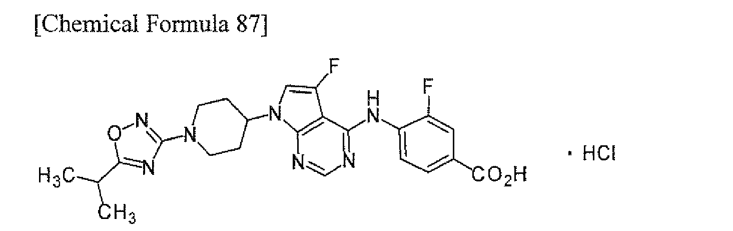 A corresponding starting compound was treated in the similar manner to Reference Example 24 to give the titled compound (yield: 35%).
MS(APCI)m/z; 484[M+H]⁺.

### Reference Example 44

Preparation of 3-fluoro-4-[5-fluoro-7-[1-(5-propylpyrimidin-2-yl)piperidin-4-yl]-7H-pyrrolo[2,3-d]pyrimidin-4-ylaminoo]benzoic acid dihydrochloride 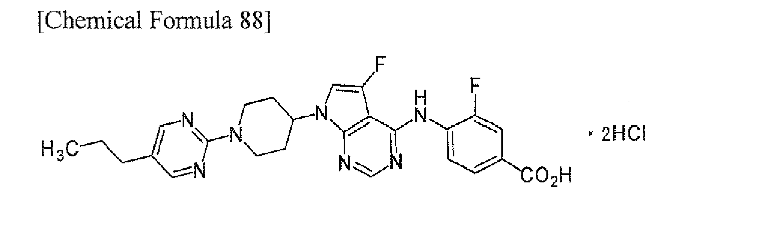 A corresponding starting compound was treated in the similar manner to Reference Example 24 to give the titled compound (yield: 60%).
MS(APCI)m/z; 494[M+H]⁺.

### Reference Example 45

Preparation of 3-fluoro-4-[5-fluoro-7-[1-(5-isopropylpyrirmidin-2-yl)piperidin-4-yl]-7H-pyrrolo[2,3-d]pyrimidin-4-ylamino]benzoic acid dihydrochloride 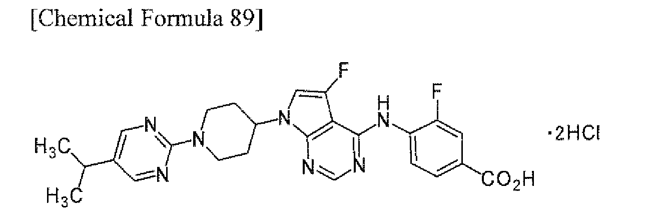 A corresponding starting compound was treated in the similar manner to Reference Example 24 to give the titled compound (yield: 64%).
MS(APCI)m/z; 494[M+H]⁺.

### Reference Example 46

Preparation of 4-[7-[1-(5-cyclopropylpyrimidin-2-yl)piperidin-4-yl]-5-fluoro-7H-pyrrolo[2,3-d]pyrimidin-4-ylamino]-3-fluorobenzoic acid dihydrochloride 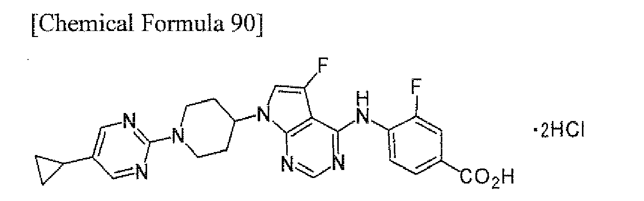 A corresponding starting compound was treated in the similar manner to Reference Example 24 to give the titled compound (yield: 54%).
MS(APCI)m/z; 492[M+H]⁺.

### Reference Example 47

Preparation of 4-[7-[1-(5-chloropyrimidin-2-yl)piperidin-4-yl]-5-fluoro-7H-pyrrolo[2,3-d]pyrimidin-4-ylamino]-3-fluorobenzoic acid dihydrochloride 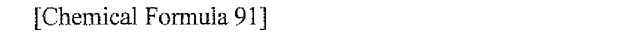 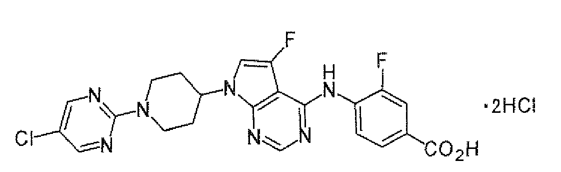 A corresponding starting compound was treated in the similar manner to Reference Example 24 to give the titled compound (yield: 32%).
MS(APCI)m/z; 486/488 [M+H]⁺.

### Reference Example 48

Preparation of 3-fluoro-4-[5-ftuoro-7-[1-(5-fluoropyrimidin-2-yl)piperidin-4-yl]-7H-pyrrolo[2,3-d]pyrimidin-4-ylamino]benzoic acid dihydrochloride 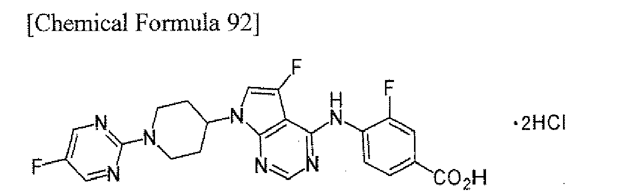 A corresponding starting compound was treated in the similar manner to Reference Example 24 to give the titled compound (yield: 49%).
MS(APCI)m/z; 470[M+H]⁺.

### Reference Example 49

Preparation of 3-fluoro-4-[5-fluoro-7-[1-(3-isopropyl-[1,2,4]oxadiazol-5-yl)piperidin-4-yl]-7H-pyrrolo[2,3-d]pyrimidin-4-ylamino]benzoic acid hydrochloride 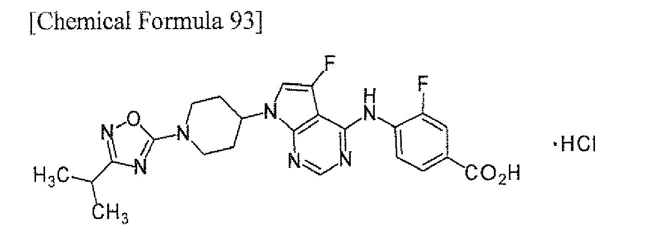 A corresponding starting compound was treated in the similar manner to Reference Example 24 to give the titled compound (yield: 91%).
MS(APCI)m/z; 484[M+H]⁺.

### Reference Example 50

Preparation of isopropyl 4-[4-(4-carboxy-2-fluorophenylamino)-5-fluoropyrrolo[2,3-d]pyrimidin-7-yl]piperidine-1-carboxylate hydrochloride 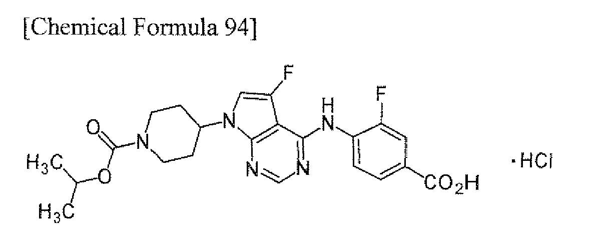 A corresponding starting compound was treated in the similar manner to Reference Example 24 to give the titled compound (yield: 80%).
MS(APCI)mlz; 460[M+H]⁺.

### Reference Example 51 1

Preparation of 3-fluoro-4-[5-fluoro-7-[1-(5-isopropoxypyrimidin-2-yl)piperidin-4-yl]-7H-pyrrolo[2,3-d]pyrimidin-4-ylamino]benzoic acid dihydrochloride 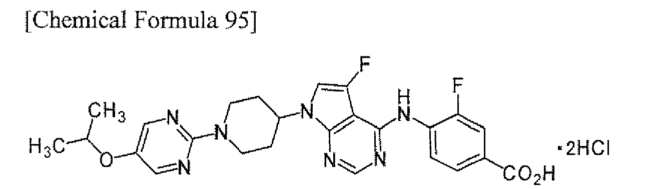 A corresponding starting compound was treated in the similar manner to Reference Example 24 to give the titled compound (yield: 53%).
MS(APCI)m/z; 510[M+H]⁺.

### Reference Example 52

Preparation of 3-fluoro-4-[5-fluoro-7-[1-(3-propyl-[1,2,4]oxadiazol-5-yl)piperidin-4-yl]-7H-pyrrolo[2,3-d]pyrimidin-4-ylamino]benzoic acid hydrochloride 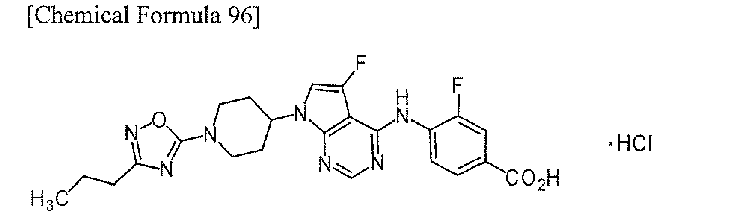 A corresponding starting compound was treated in the similar manner to Reference Example 24 to give the titled compound (yield: 34%).
MS(APCI)m/z; 484[M+H]⁺.

### Reference Example 53

Preparation of 4-chloro-5-fluoro-7-[1-(5-propylpyrimidin-2-yl)piperidin-4-yl]-7H-pyrrolo[2,3-d]pyrimidine 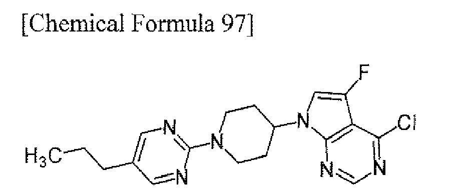 A corresponding starting compound was treated in the similar manner to Reference Example 1(2) to give the titled compound (yield: 60%).
MS(APCI)m/z; 375/377[M+H]⁺.

### Reference Example 54

Preparation of 4-chloro-5-fluoro-7-[1-(5-fluoropyrimidin-2-yl)piperidn-4-yl]-7H-pyrrolo[2,3-d]pyrimidine 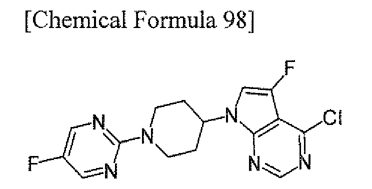 A corresponding starting compound was treated in the similar manner to Reference Example 17 to give the titled compound (yield: 41%).
MS(APCI)m/z; 351/353[M+H]⁺.

### Reference Example 55 5

Preparation of 3-fluoro-4-[5-fluoro-7-[1-(N-hydroxycarbamimidoyl)piperidin-4-yl]-7H-pyrrolo[2,3-d]pyrimidin-4-ylarmino]-N,N-dimethylbenzamide 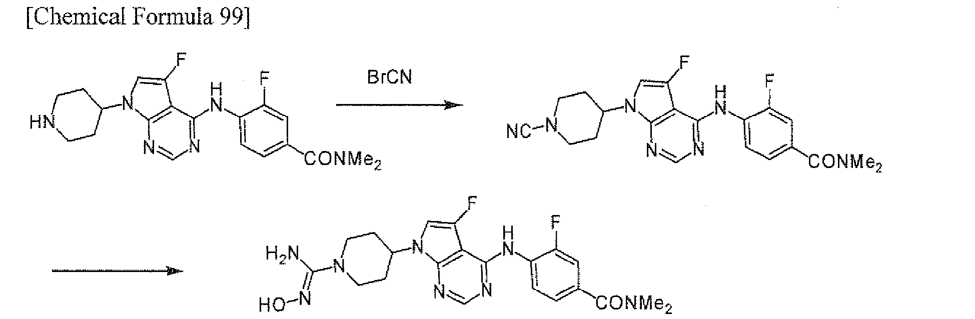
(1) To a solution of 3-fluoro-4-[5-fluoro-7-(piperidin-4-yl)-7H-pyrrolo[2,3-d]pyrimidin-4-ylamino]-N,N-dimethylbenzamide (100 mg), which was obtained by treating the compound obtained in Reference Example 4 with a saturated aqueous sodium hydrogencarbonate solution and then extracting with chloroform, in ethanol (2 mL)/tetrahydrofuran (1 mL) were added cyanogen bromide (29 mg) and sodium hydrogencarbonate (64 mg), and the mixture was stirred at room temperature for 16 hours. To the reaction mixture was added methylene chloride, and then the mixture was filtered. The filtrate was concentrated, and the resulting residue was purified by column chromatography on silica gel (solvent; chloroform/methanol = 99/1 to 96/4) to give 4-[7-(1-cyanopiperidin-4-yl)-5-fluoro-7H-pyrrolo[2,3-d]pyrimidin-4-ylamino]-3-fLuoro-N,N-dimethylbenzamide (96.7 mg) as a powder (yield 91%).
   MS(APCI)m/z; 426[M+H]⁺.
(2) To a solution of the compound (96 mg) obtained in the above (1) in isopropanol (1 mL) was added 50% aqueous hydroxylamine solution (30 mg), and the mixture was stirred at 90°C for 4 hours. The reaction mixture was concentrated to give the titled compound (106 mg) as a crude product.
   MS(APCI)m/z; 459[M+H]⁺.

### Reference Example 56

Preparation of 2-[4-[4-(4-dimethylcarbamoyl-2-fluorophenylamino)-5-fluoropyrrolo[2,3-d]pyrimidin-7-yl]piperidin-1-yl]pyrimidine-5-carboxylic acid 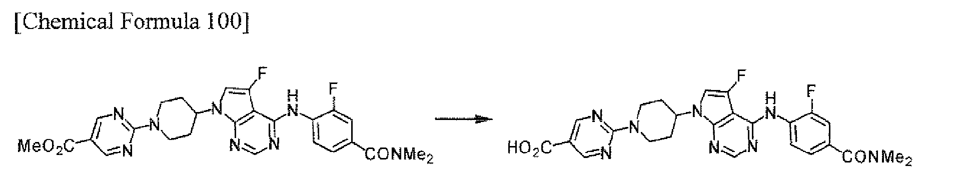 To a solution of methyl 2-[4-[4-(4-dimethylcarbamoyl-2-fluorophenylamino)-5-fluoropyrrolo[2,3-d]pyrimidin-7-yl]piperidin-1-yl]pyrimidine-5-carboxylate (obtained in Example 93; 300 mg) in methanol (3 mL)/tetrahydrofuran (3 mL) was added 2N aqueous sodium hydroxide solution (0.56 mL), and the mixture was stirred at 60°C for 1 hour. To the reaction mixture was added under ice-cooling 2N hydrochloric acid water (0.56 mL), and then the mixture was extracted with chloroform. The organic layer was concentrated, and the resulting residue was triturated with chloroform to give the titled compound (323 mg) as a crude product.
MS(APCI)m/z; 523[M-H]⁺.

### Reference Example 57

Preparation of (4-amino-3-fluoro-phenyl)(pyrrolidin-1-yl)methanone 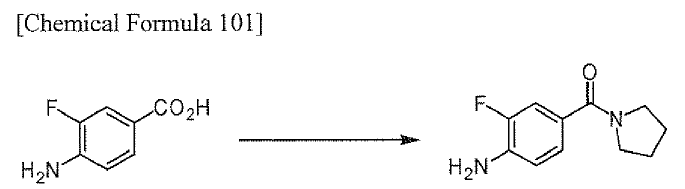 To a solution of 4-amino-3-fluorobenzoic acid (1.00 g) in methylene chloride (20 mL) were added pyrrolidine (700 µL), N-hydroxybenzotriazole monohydrate (1.28 g), 1-ethyl-3-(3-dimethylaminopropyl)carbodiimide hydrochloride (1.6 g), and the mixture was stirred at room temperature overnight. To the reaction mixture was added a saturated sodium hydrogencarbonate solution, and then the mixture was extracted with chloroform. The organic layer was concentrated under reduced pressure, and the resulting residue was purified by column chromatography on NH silica-gel (Chromatorex; Fuji Silysia Chemical Ltd., solvent; hexane/ethyl acetate = 80/20 to 25/75) to give the titled compound (1.04 g) as a powder (yield: 78%).
MS(APCI)m/z; 209[M+H]⁺.

### Reference Example 58 8

Preparation of 4-ammo-N,N-dimethylbenzamide 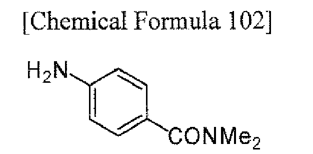 A corresponding starting compound was treated in the similar manner to Reference Example 9 to give the titled compound (yield: 61%).
MS(APCI)m/z; 165[M+H]⁺.

### Reference Example 59

Preparation of N,N-dimethyl-5-aminopyridine-2-carboxarmide 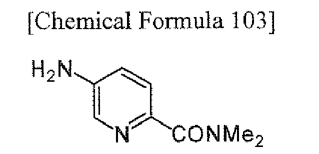 A corresponding starting compound was treated in the similar manner to Reference Example 9 to give the titled compound (yield: 12%).
MS(APCI)m/z; 166[M+H]⁺.

### Reference Example 60

Preparation of tert-butyl 4-amino-2,5-difluorobenzoate 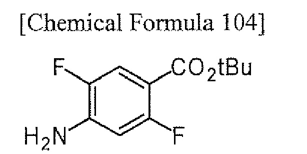 A corresponding starting compound was treated in the similar manner to Reference Example 23 to give the titled compound (yield: 48%).
MS(APCI)m/z; 230[M+H]⁺.

### Reference Example 61 1

Preparation of tert-butyl 4-amino-3-trifluoromethylbenzoate 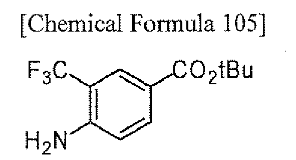 A corresponding starting compound was treated in the similar manner to Reference Example 23 to give the titled compound (yield; 54%).

### Reference Example 62

Preparation of 4-[7-[1-(5-ethyl-pyrimidin-2-yl)-piperidin-4-yl]-5-fluoro-7H-pyrrolo[2,3-d]pyrimidin-4-ylamino]-2,5-difluorobenzoic acid dihydrochloride 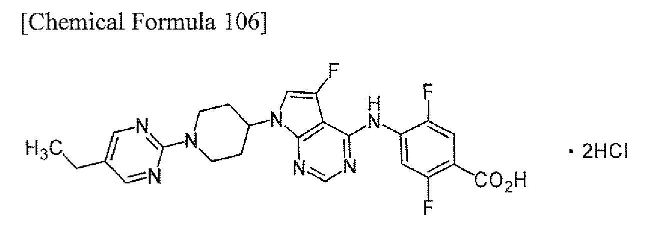 A corresponding starting compound was treated in the similar manner to Reference Example 24 to give the titled compound (yield: 30%).
MS(APCI)m/z; 498[M+H]⁺.

### Reference Example 63

Preparation of 4-[7-[1-(5-ethyl-pyrimidin-2-yl)-piperidin-4-yl]-5-fluoro-7H-pyrrolo[2,3-d]pyrimidin-4-ylamino]-3-trifluoromethylbenzoic acid dihydrochloride 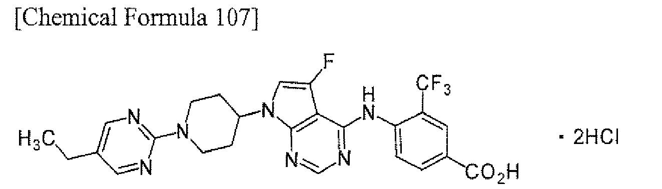 A corresponding starting compound was treated in the similar manner to Reference Example 24 to give the titled compound (yield: 18%).
MS(APCI)m/z; 530[M+H]⁺.

### Reference Example 64

Preparation of 4-chloro-5-fluoro-7-[1-(5-pentyl-pyrimidin-2-yl)-piperidin-4-yl]-7H-pyrrolo[2,3-d]pyrimidine 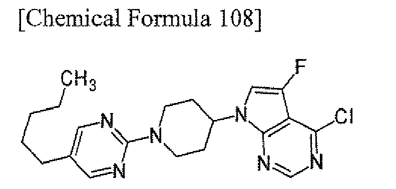 A corresponding starting compound was treated in the similar manner to Reference Example 1(2) to give the titled compound (yield: 55%).
MS(APCI)m/z; 403/405[M+H]⁺.

### Reference Example 65

Preparation of 3-fluoro-4-[5-fluoro-7-[1-(5-pentylpyrimidin-2-yl)piperidin-4-yl]-7H-pyrrolo[2,3-d]pyrimidin-4-ylamino]benzoic acid dihydrochloride 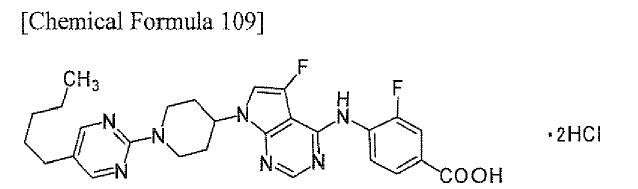 A corresponding starting compound was treated in the similar manner to Reference Example 24 to give the titled compound (yield: 33%).
MS(APCI)m/z; 522[M+H]⁺.

### Reference Example 66

Preparation of 1-(2-isopropyl-2H-tetrazol-5-yl)-piperidm-4-ol 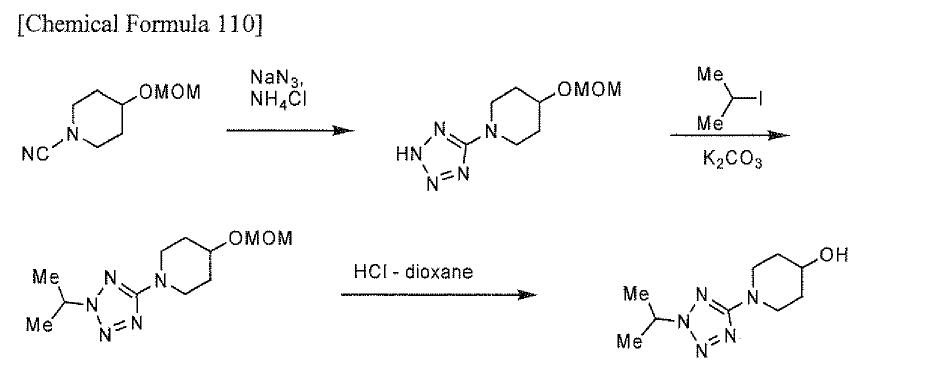
(1) To a solution of 4-methoxymethoxypiperidine-1-carbonitrile (obtained in Reference Exaznple 18(1); 8.14 g) in dimethylformamide (50 mL) were added sodium azide (7.77 g) and ammonium chloride (6.91 g), and the mixture was stirred at 100°C for 18 hours. The reaction mixture was cooled to room temperature, and thereto was added 10% aqueous citric acid solution, and the mixture was extracted with ethyl acetate. The organic layer was washed with brine, dried over magnesium sulfate, and then filtered, and the filtrate was concentrated under reduced pressure. The resulting residue was purified by column chromatography on silica gel (solvent; chloroform/methanol = 100/0 to 92/8) to give 4-methoxymethoxy-1-(2H-tetrazol-5-yl)piperidine (6.79 g; yield: 67%).
   MS(APCI)m/z; 214[M+H]⁺.
(2) To a solution of the compound (3.39 g) obtained in the above (1) in dimethylformamide (50 mL) were added potassium carbonate (4.39 g) and isopropyl iodide (2.38 mL), and the mixture was stirred at room temperature for 2 hours. To the reaction mixture was added water, and then the mixture was extracted with ethyl acetate. The organic layer was washed successively with water and brine, dried over magnesium sulfate, and then filtered. The filtrate was concentrated under reduced pressure, and the resulting residue was purified by silica-gel column chromatography (solvent; hexane/ethyl acetate = 90/10 to 65/35) to give 1-(2-isopropyl-2H-tetrazol-5-yl)-4-methoxymethoxypiperidine (2.96 g; yield: 73%).
   MS(APCI)m/z; 256[M+H]⁺.
(3) The compound (2.96 g) obtained in the above (2) was treated in the similar manner to Reference Example 18(4) to give the titled compound (yield: 93%).
   MS(APCI)m/z; 212[M+H]⁺.

### Reference Example 67

Preparation of 1-(2-n-propyl-2H-tetrazol-5-yl)-piperidin-4-ol 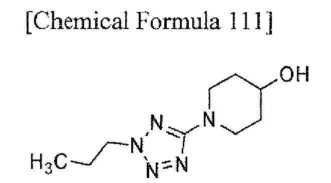 A corresponding starting compound was treated in the similar manner to Reference Example 66 to give the titled compound.
MS(APCI)m/z; 212[M+H]⁺.

### Reference Example 68

Preparation of 4-chloro-5-fluoro-7-[1-(2-isopropyl-2H-tetrazol-5-yl)piperidn-4-yl]-7H-pyrrolo[2,3-d]pyrimidine 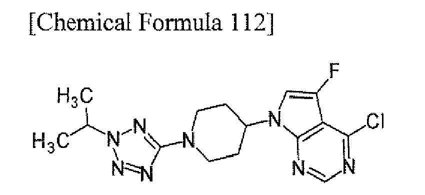 A corresponding starting compound was treated in the similar manner to Reference Example 1(2) to give the titled compound (yield: 73%).
MS(APCI)m/z; 365/367[M+H.]⁺.

### Reference Example 69

Preparation of 4-chloro-5-fluoro-7-[1-(2-n-propyl-2H-tetrazol-5-yl)piperidin-4-yl]-7H-pyrrolo[2,3-d]pyrimidine 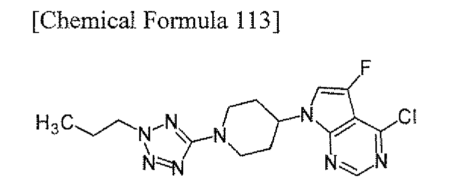 A corresponding starting compound was treated in the similar manner to Reference Example 1(2) to give the titled compound (yield: 74%).
MS(APCI)m/z; 365/367[M+H]⁺.

### Reference Example 70

Preparation of 4-chloro-5-fluoro-7-[1-(5-trifluoromethyl-[1,2,4]oxadiazol-3-yl)piperidin-4-yl]-7H-pyrrolo[2,3-d]pyrimidine 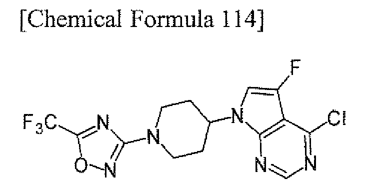 A corresponding starting compound was treated in the similar manner to Reference Example 18 to give the titled compound.
MS(APCI)m/z; 391/393[M+H]⁺.

### Reference Example 71 1

Preparation of 2-methylsulfanyl-5-trifluoromethyl pyrimidine 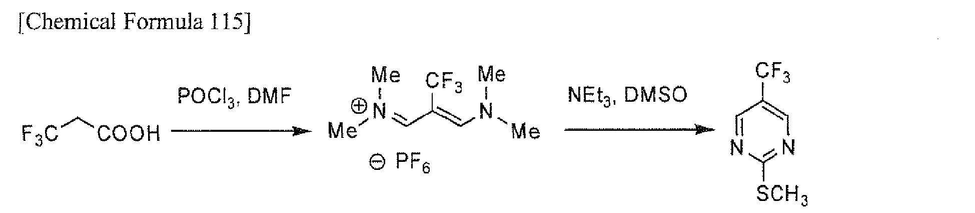
(1) A solution of 3,3,3-trifluoropropionic acid (6.4 g) in N,N-dimethylformamide (50 mL) was heated to 60°C, and thereto was added dropwise phosphorus oxychloride (14 mL) over 2 hours so that the internal temperature was 70°C or below. Then, the mixture was stirred at 70°C for 1 hour. The reaction solution was cooled to room temperature, and then added dropwise together with 5N aqueous sodium hydroxide solution (28 mL) to a mixed solution of ice-cooled water (60 mL), 5N aqueous sodium hydroxide solution (15 mL) and 60% hexafluorophosphoric acid (13 g) over 30 minutes. The mixture was stirred at the same temperature for 1.5 hours. The precipitate was filtered, washed with water, and then dried at 40°C with blowing to give 3-dimethylamino-2-trifluoromethylallylidyne)dimethyl ammonium hexafluorophosphate (7.04 g) as a powder (yield 41 %).
   MS(APCI)m/z: 195 [M-F₆P]⁺.
(2) To a solution of the compound (2.35 g) obtained in (1) in dimethylsulfoxide (20 mL) were added 2-methyl-isothiourea 1/2 sulfate (1.14 g) and triethylamine (2.8 mL), and the mixture was stirred at room temperature for 3 hours. To the reaction mixture was added water, and then the mixture was stirred under ice-cooling for 15 minutes. The precipitates were collected by filtration and washed with water, and then dried under reduced pressure to give the titled compound (1.04 g, yield 78%).
   MS(APCI)m/z: 195[M+H]⁺.

### Reference Example 72

Preparation of 1-(5-trifluoromethylpyrimidin-2-yl)piperidin-4-ol 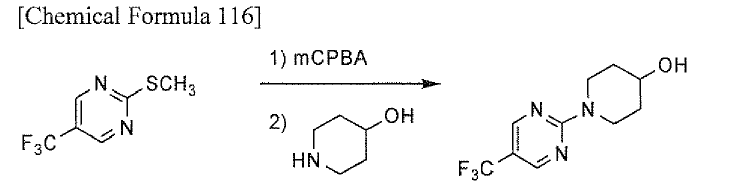 To a solution of 2-methylsulfanyl-5-trifluoromethylpyrimidine (i.e., the compound obtained in Reference Example 71) (0.97 g) in methylene chloride (25 mL) was added meta-chloroperoxybenzoic acid (25% aqueous) (2.30 g) under ice-cooling. The mixture was stirred at room temperature for 1 hour, and then thereto were added 4-hydroxypiperidine (1.01 g) and triethylamine (2.02). The mixture was stirred at room temperature overnight. The reaction solution was poured into a saturated aqueous sodium hydrogencarbonate solution, and the organic layer was separated. The aqueous layer was extracted with chloroform twice, and the organic layer was dried over magnesium sulfate, and then filtered. The filtrate was concentrated under reduced pressure. The resulting mixture was purified by column chromatography on silica gel to give the titled compound (0.93 g; yield 75%).
MS(APCI)m/z:248[M+H]⁺.

### Reference Example 73

Preparation of 4-chloro-5-fluoro-7-[1-(5-trifluoromethyl-pyrimidin-2-yl)piperidn-4-yl]-7H-pyrrolo[2,3-d]pyrimidine 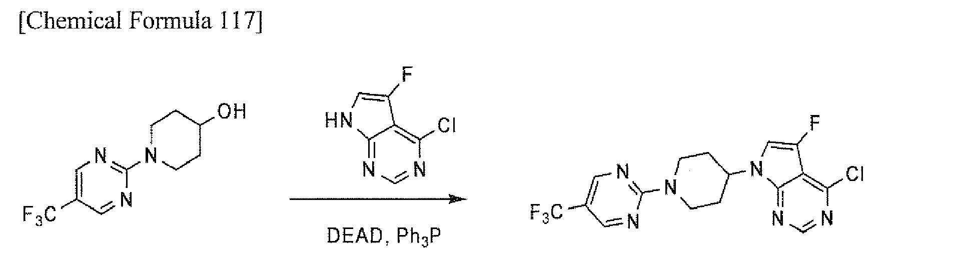 A corresponding starting compound was treated in the similar manner to Reference Example 1(2) to give the titled compound (yield: 23%).
MS(APCI)m/z:401/403[M+H]⁺.

### Reference Example 74

Preparation of 3-chloro-4-[7-[1-(5-ethylpyrimidin-2-yl)piperidin-4-yl]-5-fluoro-7H-pyrrolo[2,3-d]pyrimidin-4-ylamino]benzoic acid 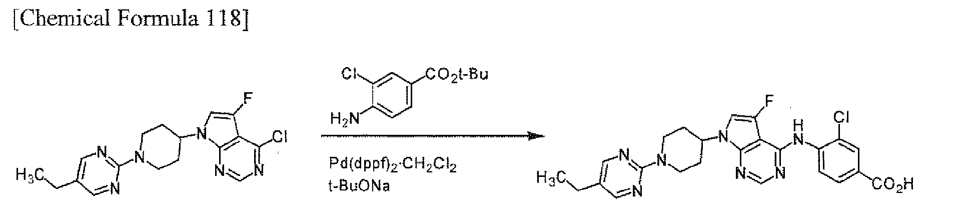 To a solution of 4-chloro-5-fluoro-7-[1-(5-ethylpyrimidin-2-yl)piperidin-4-yl]-7H-pyrroio[2,3-d]pyrimidine (i.e., the compound obtained in Reference Example 17) (890 mg), tert-butyl 4-amino-3-chlorobenzoate (i.e., the compound obtained in Reference Example 76) (563 mg) and [1,1'-bis(diphenylphosphino)ferrocene]palladium (II) dichloride dichloromethane complex (1:1) (101 mg) in 1,4-dioxane (25 mL) was added sodium tert-butoxide (594 mg), and the mixture was stirred at 100°C for 1 hour. To the reaction mixture was added water, and the mixture was washed with ethyl acetate. The aqueous layer was adjusted to pH6 to 7 by the addition of 1N HCl, and extracted with ethyl acetate. The organic layer was dried over magnesium sulfate, and then filtered. The filtrate was concentrated under reduced pressure. The resulting residue was triturated by dichloromethane-hexane to give the titled compound (473 mg) as a powder (yield 39%).
MS(APCI)m/z; 496/498[M+H]⁺.

### Reference Example 75

Preparation of 3-fluoro-4-[5-fluoro-7-[1-(5-trifluoromethyl-[1,2,4]oxadiazol-3-yl)piperidin-4-yl]-7H-pyrrolo[2,3-d]pyrimidin-4-ylamino]benzoic acid hydrochloride 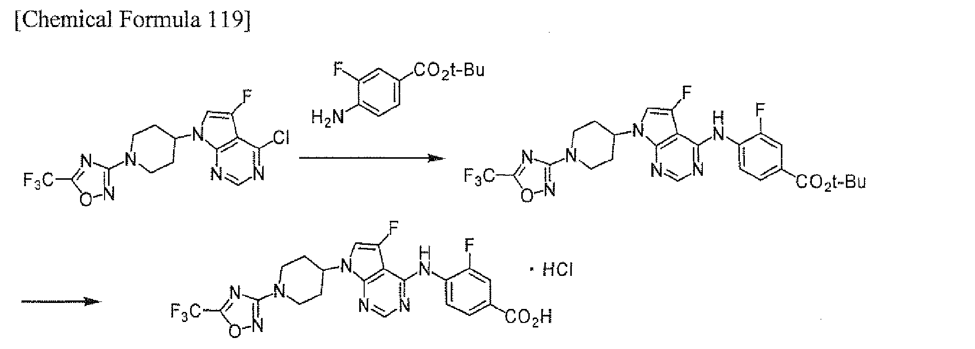
(1) To a solution of the compound obtained in Reference Example 70 (952 mg) and tert-butyl 4-amino-3-fluorobenzoate (i.e., the compound obtained in Reference Example 23) (1.03 g) in 2-propanol (19 mL) was added 4N hydrochloric acid-dioxane solution (61 µL), and the mixture was stirred at 80°C for 17 hours. The reaction mixture was cooled to room temperature, and then thereto was added a saturated aqueous sodium hydrogencarbonate solution. The mixture was extracted with ethyl acetate. The organic layer was concentrated under reduced pressure. The resulting residue was purified by column chromatography on NH-silica gel (Chromatorex; Fuji Silysia Chemical Ltd., solvent; chloroform/methanol = 100/0 to 92/8), and then purified by gel permeation chromatography (JAIGEL-1H,2H; Japan Analytical Industry, Co., Ltd., mobile phase; chloroform) to give tert-butyl 3-fluoro-4-[5-fluoro-7-[1-(5-trifluoromethyl-[1,2,4]oxadiazol-3-yl)piperidin-4-yl]-7H-pyrrolo[2,3-d]pyrimidin-4-ylamino]benzoate (594 mg; yield 43%).
   MS(APCI)m/z; 566[M+H]⁺.
(2) The compound obtained in the above (1) (653 mg) was treated in the similar manner to Reference Example 24 (2) to give the titled compound (595 mg; yield 95%).
   MS(APCI)m/z; 510[M+H]⁺.

### Reference Example 76

Preparation of tert-butyl 4-amino-3 -chlorobenzoate 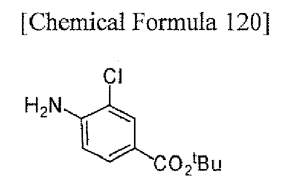 A corresponding starting compound was treated in the similar manner to Reference Example 23 to give the titled compound.
MS(APCI)m/z; 228/230[M+H]⁺.

### Reference Example 77

Preparation of tert-butyl 4-amino-2-fluorobenzoate 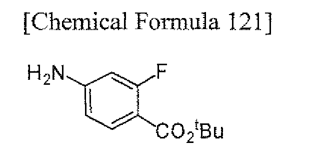 A corresponding starting compound was treated in the similar manner to Reference Example 23 to give the titled compound.
MS(APCI)m/z; 212[M+H]⁺.

### Reference Example 78

Preparation of tert-butyl 4-amino-2-chlorobenzoate 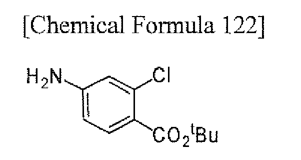 A corresponding starting compound was treated in the similar manner to Reference Example 23 to give the titled compound.
MS(APCI)m/z; 228/230[M+H]⁺.

### Reference Example 79

Preparation of 3-fluoro-4-[5-fluoro-7-[1-(2-isopropyl-2H-tetrazol-5-yl)piperidin-4-yl]-7H-pyrrolo[2,3-d]pyrimidin-4-ylamino]benzoic acid dihydrochloride 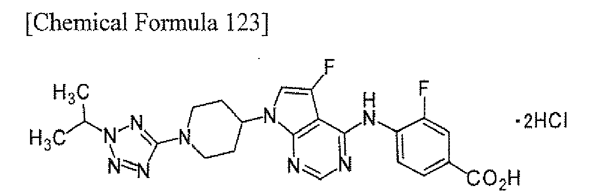 A corresponding starting compound was treated in the similar manner to Reference Example 24 to give the titled compound (yield: 54%).
MS(APCI)m/z; 484[M+H]⁺.

### Reference Example 80

Preparation of 3-fluoto-4-[5-fluoro-7-[1-(2-propyl-2H-tetrazol-5-yl)piperidin-4-yl]-7H-pyrrolo[2,3-d]pyrimidin-4-ylamino]benzoic acid dihydrochloride 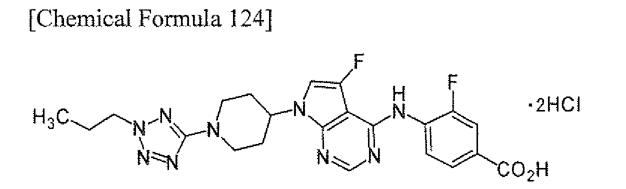 A corresponding starting compound was treated in the similar manner to Reference Example 24 to give the titled compound (yield: 52%).
MS(APCI)m/z; 484[M+H]⁺.

### Reference Example 81

Preparation of 2-chloro-4-[7-[1-(5-ethylpyrimidin-2-yl)piperidin-4-yl]-5-fluoro-7H-pyrrolo[2,3-d]pyrimidin-4-ylamino]benzoic acid dihydrochloride 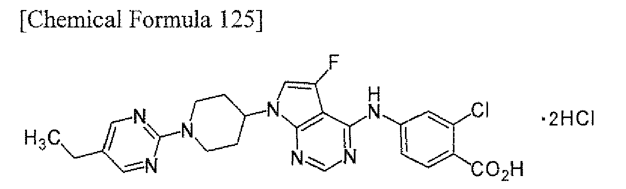 A corresponding starting compound was treated in the similar manner to Reference Example 24 to give the titled compound (yield: 63%).
MS(APCI)m/z; 496/498[M+H]⁺.

### Reference Example 82

Preparation of 2-fluoro-4-[7-[1-(5-ethylpyrimidin-2-yl)piperidin-4-yl]-5-fluoro-7H-pyrrolo[2,3-d]pyrimidin-4-ylamino]benzoic acid dihydrochloride 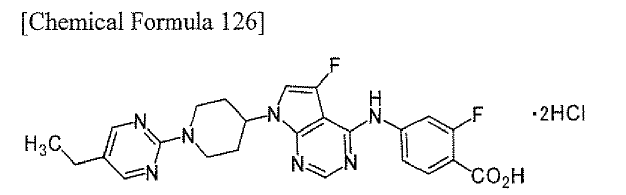 A corresponding starting compound was treated in the similar manner to Reference Example 24 to give the titled compound (yield: 40%).
MS(APCI)m/z; 480[M+H]⁺.

### Reference Example 83

Preparation of 3-fluoro-4-[5-fluoro-7-[1-(5-trifluoromethylpyrimidin-2-yl)piperidin-4-yl]-7H-pyrrolo[2,3-d]pyrimidn-4-ylamino]benzoic acid dihydrochloride 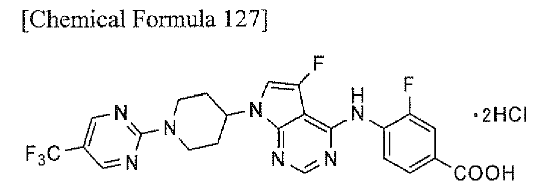 A corresponding starting compound was treated in the similar manner to Reference Example 24 to give the titled compound (yield: 20%).
MS(APCI)m/z; 520[M+H]⁺.

### Reference Example 84

Preparation of isopropyl 4-[4-(4-carboxy-2-chlorophenylamino)-5-fluoro-pyrrolo[2,3-d]pyrimidin-7-yl]piperidine-1-carboxylate hydrochloride 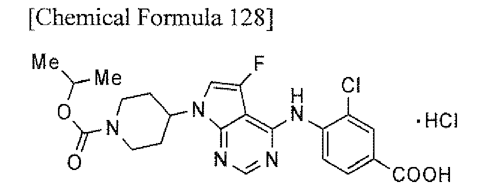 A corresponding starting compound was treated in the similar manner to Reference Example 24 to give the titled compound (yield: 26%).
MS(APCI)m/z; 476/478[M+H]⁺.

### Reference Example 85

Preparation of (3S,4S)-pyrrolidine-3,4-diol 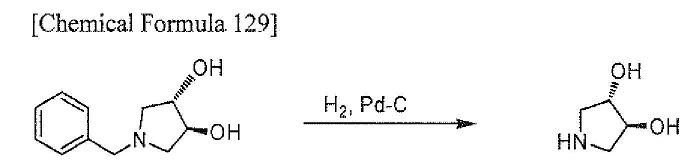 To a solution of (3S,4S)-1-benzylpynolidne-3,4-diol (522 mg) in ethanol (15 mL) were added 10% palladium-carbon (100 mg) and acetic acid (10 mL), and the mixture was reacted under pressurized hydrogen (40psi) at room temperature for 7 hours in Parr hydrogenation apparatus. The reaction solution was filtered through Celite, and the filtrate was concentrated under reduced pressure. To the resulting residue was added 4N hydrochloric acid-dioxane solution, and then the mixture was concentrated under reduced pressure to give the titled compound (373 mg) as a yellow solid (yield 99%).
MS(APCI)m/z; 104[M+H]⁺.

### Reference Example 86

Preparation of 3-fluoro-4-[5-fluoro-7-[1-(5-difluoromethyl-[1,2,4]oxadiazol-3-yl)piperidin-4-yl]-7H-pyrrolo[2,3-d]pyrimidin-4-ylamino]benzoic acid hydrochloride 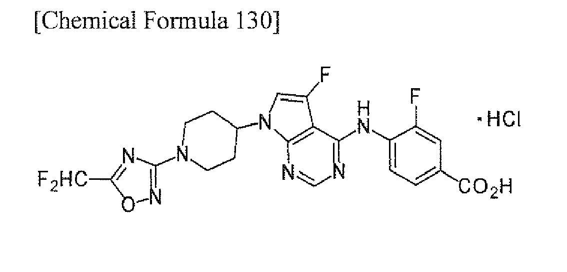 A corresponding starting compound was treated in the similar manner to Reference Example 75 to give the titled compound.
MS(APCI)m/z; 492[M+H]⁺.

### Reference Example 87

Preparation of 4-[7-[1-(5-ditrifluoromethoxypyrimidin-2-yl)piperidin-4-yl]-5-fluoro-7H-pyrrolo[2,3-d]pyrinmdin-4-ylamino]-3-fluorobenzoic acid dihydrochloride 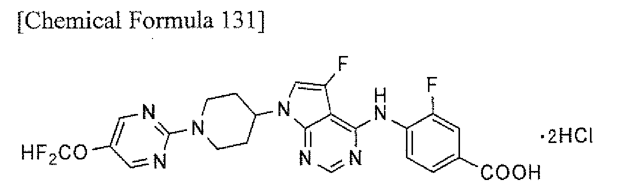 A corresponding starting compound was treated in the similar manner to Reference Example 24 to give the titled compound (yield: 62%).
MS(APCI)m/z; 518[M+H]⁺.

### Reference Example 88

Preparation of 3-fluoro-4-[5-fluoro-7-[1-(5-methyl-pyrimidin-2-yl)-piperidin-4-yl]-7H-pyrrolo[2,3-d]pyrimidin-4-ylamino]-benzoic acid dihydrochloride 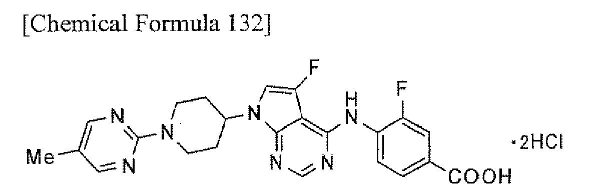 A corresponding starting compound was treated in the similar manner to Reference Example 24 to give the titled compound (yield: 25%).
MS(APCI)m/z; 466[M+H]⁺.

### Reference Example 89

Preparation of (3R,4R)-pyrrolidine-3,4-diol 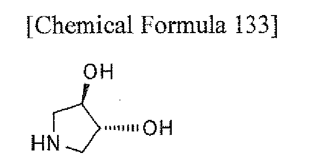 A corresponding starting compound was treated in the similar manner to Reference Example 85 to give the titled compound (yield: 100%).
MS(APCI)m/z; 104[M+H]⁺.

### Reference Example 90

Preparation of 4-chloro-5-fluoro-7-[1-(5-difluoromethyl-[1,2,4]oxadiazol-3-yl)piperidin-4-yl]-7H-pyrrolo[2,3-d]pyrimidine 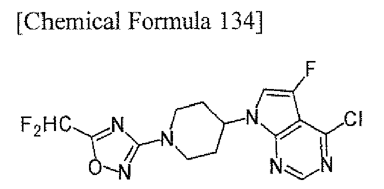 A corresponding starting compound was treated in the similar manner to Reference Example 18 to give the titled compound.
MS(APCI)m/z; 373/375[M+H]⁺,

### Reference Example 91 1

Preparation of 2-chloro-5-difluoromethoxypyrimidine 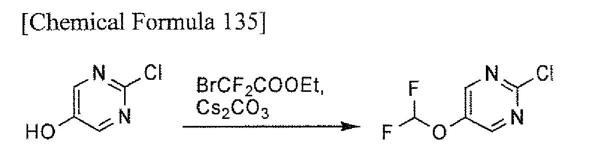 To a solution of 2-chloro-5-hydroxypyrimidine (4.13 g) in DMF (40 mL) were added ethyl 2-bromo-2,2-difluoroacetate (12.83 g) and cesium carbonate (20.59 g), and the mixture was reacted at 80°C overnight. The reaction solution was cooled to room temperature, and then poured into water. The mixture was extracted with ethyl acetate thrice. The organic layer was dried over magnesium sulfate, and then filtered. The filtrate was concentrated under reduced pressure. The resulting residue was purified by column chromatography on silica gel (hexane:ethyl acetate = 80:20 to 60:40) to give the titled compound (2.16 g) as a colorless liquid (yield 38%).
MS(APCI)m/z; Not detected.

### Reference Example 92

Preparation of 1-(5-difluoromethoxypyrimidin-2-yl)piperidin-4-ol 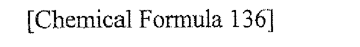 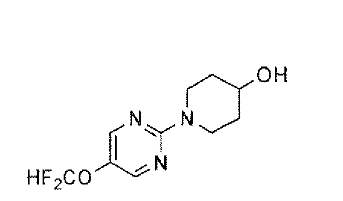 A corresponding starting compound was treated in the similar manner to Reference Example 17(1) to give the titled compound (yield: 100%).
MS(APCI)m/z:246[M+H]⁺.

### Reference Example 93

Preparation of 4-chloro-7-[1-(5-difluoromethoxypyrimidin-2-yl)piperidn-4-yl]-5-fluoro-7H-pyrrolo[2,3-d]pyrimidine 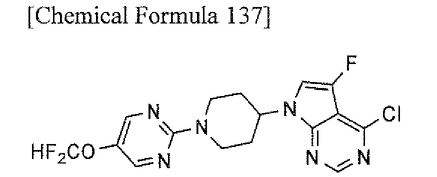 A corresponding starting compound was treated in the similar manner to Reference Example 1(2) to give the titled compound (yield: 63%).
MS(APCI)m/z:399/401 [M+H]⁺.

### Reference Example 94

Preparation of 1-(5-methylpyrimidin-2-yl)piperidin-4-ol 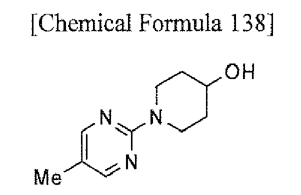 A corresponding starting compound was treated in the similar manner to Reference Example 17(1) to give the titled compound (yield: 100%).
MS(APCI)m/z: 194[M+H]⁺.

### Reference Example 95

Preparation of 4-chloro-5-fluoro-7-[1-(5-methylpyrimidin-2-yl)piperidin-4-yl]-7H-pyrrolo[2,3-d]pyrimidine 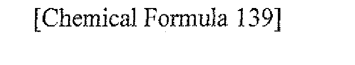 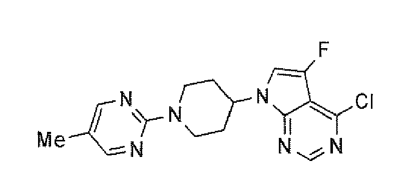 A corresponding starting compound was treated in the similar manner to Reference Example 1(2) to give the titled compound (yield: 29%).
MS(APCI)m/z:347/349[M+H]⁺.

### Experiment 1

### (Purpose for Experiment)

The present experiment is directed to evaluate GPR119 agonistic activity (in vitro) of test compounds by adding the compounds to human GPR119-expressed CHO cells to determine cAMP production of the cells.

### (Preparation of human GPR119-expressed CHO cells)

Human GPR119-expressed CHO cells (L8-18) were prepared by introducing an expression vector pMSF1-GPR119 (Geneticin-resistance) carrying human GPR119 genes into CHO cells (LM-3; Mock cells) wherein lusiferase expression vector pLG3-CRE6-CRE-VIP (Hygromycin B-resistance) were introduced according to the known method (The Journal of Biological Chemistry Vol. 274 (34), pp. 23940-23947).

### (Test Method)

Cryopreserved L8-18 cells were melted, and then suspended into 9-fold amounts of assay buffer and centrifuged (1000 rpm, 5 minutes) at room temperature. The supernatant was removed, and then the precipitated cells were resuspended into assay buffer (4 mL). Thereto was added assay buffer which contained IBMX (manufactured by Sigma, #17018-1G), and 7.5 x 10⁴cells/mL of cell suspension was prepared. The cell suspension was let stand at room temperature for 15 minutes, and then to each well of 96 half well white plate (manufactured by Corning Incorporated, #3693) were added the cell suspension (20 µL) and a solution of a test compound or AR231453 (5 µL; a total of 25 µL/well) (final concentration: 1500 cells/well, 500 µM IBMX, 1% dimethylsulfoxide). The mixture was incubated at 37°C for 30 minutes, and then to each well were added a 20-fold diluted solution (each 12.5 µL/well) of cAMP-d2 and Anti cAMP-Cryptate of HTRF cAMP kit (manufactured by Cisbio, #62AM4PEC). The mixture was stirred, and then was let stand for 1 hour under light shielding. A fluorescence intensity was measured by time-resolved fluorescence mode (Ex: 320 nm, Em: 665 nm, 620 nm) of Microplate Reader (ARVO or SpectraMax M5e). In ARVO, Ratios [Ratio = (665 nm/620 nm)×10⁴)] of cAMP-d2 to Anti cAMP-Cryptate were calculated from the resulting fluorescence intensity, and cAMP concentrations of each well were calculated from cAMP standard curve prepared by the Ratios and GraphPad Purism. In SpectraMax M5e, cAMP concentrations of each well were calculated on the basis of a standard curve prepared by the resulting fluorescence intensity and Softmax Pro.
EC₅₀ of test compounds were calculated by GraphPad Prism.

### (Results)

Results of the present experiment (EC₅₀ of each test compound) are shown in the following Table 35. "++" and "+++" in the table have the following meanings.
++: 3 µM > EC₅₀ ≥ 1 µM
+++: 1 µM > EC₅₀

Table 3 5

**[Table 35]**

| Test Compound | EC₅₀ |
|---|---|
| Example Compound 1 | +++ |
| Example Compound 2 | ++ |
| Example Compound 3 | +++ |
| Example Compound 13 | +++ |
| Example Compound 15 | +++ |
| Example Compound 17 | +++ |
| Example Compound 21 | +++ |
| Example Compound 29 | +++ |
| Example Compound 54 | +++ |
| Example Compound 56 | +++ |
| Example Compound 61 | +++ |
| Example Compound 62 | +++ |
| Example Compound 63 | +++ |
| Example Compound 77 | +++ |
| Example Compound 138 | +++ |
| Example Compound 153 | +++ |
| Example Compound 164 | +++ |
| Example Compound 184 | +++ |
| Example Compound 193 | +++ |

### Experiment 2

### (Inhibitory Effect on Increased Blood Glucose of Present Compounds)

### Test Method:

C57BL/6N male mice was fasted for 21 hours, and then a stratified randomization allocation was carried out by EXSUS Ver7.6 NP (Arm Systex Co., Ltd.) based on body weights (n = 8). Vehicle (solvent: 0.1% Tween 80/0.5% hydroxypropyl methylcellulose) (control group) or a suspended solution of a test compound in the vehicle (test compound group) was orally administered to the mice, and a glucose load (3 g/kg, p.o.) was carried out one hour after administration of a test compound. Blood samplings from the test mice were carried out at each time point of just before administration of drug (-60 min), immediately before glucose load (0 min), 30 minutes (30 min), 60 minutes (60 min) and 120 minutes (120 min) after glucose load. Blood glucose levels at each time point were measured by glucose CII-Test Wako (manufactured by Wako Pure Chemical Industries, Ltd.), AUC (0-120 min) was calculated in each administration group on the basis of the measured value, and evaluated by time-dependent variance analysis and Student's t-Test using EXSUS Ver7.6NP (Arm Systex Co., Ltd.).

### Results:

An inhibitory effect on increased blood glucose of each test compound (A ratio value of AUC (0-120 min) of the test compound group in case that AUC (0-120 min) of the control group is 100) is shown in the following Table 36.

Table 36

**[Table 36]**

| Test Compound | Doses (mg/kg) | Inhibitory Effect on Increased Blood Glucose | Evaluation |
|---|---|---|---|
| Example Compound 6 | 10 | 85 | ** |
| Example Compound 8 | 10 | 83 | ** |
| Example Compound 29 | 10 | 88 | * |
| Example Compound 61 | 10 | 85 | ** |
| Example Compound 62 | 10 | 86 | ** |
| Example Compound 63 | 10 | 89 | ** |
| Example Compound 115 | 3 | 92 | * |
| Example Compound 121 | 3 | 82 | ** |
| Example Compound 124 | 3 | 86 | * |
| Example Compound 128 | 3 | 83 | ** |
| Example Compound 129 | 3 | 88 | * |
| Example Compound 132 | 3 | 90 | * |
| Example Compound 134 | 3 | 82 | ** |
| Example Compound 153 | 3 | 84 | ** |
| Example Compound 154 | 3 | 86 | * |
| Example Compound 161 | 3 | 87 | * |
| Example Compound 163 | 3 | 86 | ** |
| Example Compound 178 | 1 | 88 | * |
| Example Compound 185 | 1 | 89 | * |

| | | | |
|---|---|---|---|
| *: p<0.05 (vs. Control Group), **: p<0.01 (vs. Control Group) | | | |

### INDUSTRIAL APPLICABILITY

The compound [I] of the present invention or a pharmacologically acceptable salt thereof shows a GPR119 receptor agonistic activity, and is useful for a medicament for preventing or treating various diseases or conditions which may be expected to be improved by controlling the receptor activity, e.g., metabolic diseases including obesity, hyperglycemia, diabetes and diabetes complication, metabolic syndrome, glucose intolerance, hyperinsulinemia, hyperlipidemia, hypercholesterolemia, hypertriglyceridemia and abnormal lipid metabolism, or cardiovascular diseases including arterial sclerosis, hypertension, coronary disease, cardiac infarction, etc.

## Claims

1. A compound of the following general formula I]: 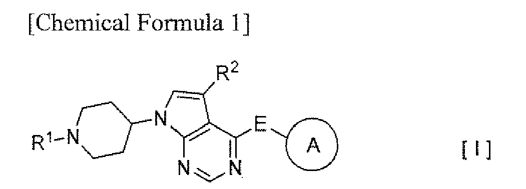 wherein E is a group of formula: -NH-, -O-, -C(=O)-, -CH(OH)- or -CF₂-,
Ring A is 6-membered aromatic ring optionally containing 1 to 2 nitrogen atoms as heteroatoms wherein the 6-membered aromatic ring may be optionally substituted by 1 to 3 groups selected from a) a halogen atom, b) cyano, c) alkylsulfonyl, d) alkyl optionally substituted by 1 to 3 halogen atoms, e) a group of formula: -CONR^{a}R^{b} and f) 5 to 6-membered heteroaryl containing the same or different 1 to 4 heteroatoms selected from the group consisting of nitrogen, oxygen and sulfur atoms,
R^{a} and R^{b} are the same or different and each hydrogen, alkyl, monohydroxyalkyl or alkoxyalkyl, or both combine each other together with the adjacent nitrogen atom to form 3 to 7-membered nitrogen-containing aliphatic heterocycle which may further contain heteroatoms selected from oxygen and sulfur atoms and may be optionally substituted by 1 to 2 hydroxyl,
R¹ is
a) acyl of R¹¹OCO- wherein R¹¹ is alkyl optionally substituted by 1 to 3 halogen atoms or cyanoalkyl,
b) 5 to 6-membered heteroaryl which contains the same or different 1 to 4 heteroatoms selected from the group consisting of nitrogen, oxygen and sulfur atoms wherein the heteroaryl may be optionally substituted by 1 to 3 groups selected from a halogen atom, alkyl optionally substituted by 1 to 3 halogen atoms, cycloalkyl, alkoxyalkyl, cycloalkylalkyl, alkoxy optionally substituted by 1 to 3 halogen atoms, alkoxycarbonyl and a group of formula: -CONR^{c}W^{d} wherein both R^{c} and R^{d} combine each other to form 3 to 7-membered nitrogen-containing aliphatic heterocycle optionally substituted by 1 to 2 halogen atoms, or
c) aryl (or nitrogen-containing heteroaryl)-alkyl,
R² is a halogen atom, cyano or alkoxycarbonyl; or a pharmaceutically acceptable salt thereof.

2. The compound as claimed in claim 1, wherein E is a group of formula: -NH-, Ring A is (i) a benzene ring substituted by 1 to 3 groups selected from (a) a halogen atom, (b) cyano, (c) alkylsulfonyl, (d) a group of formula: -CONR^{a}R^{b} and (e) 5-membered heteroaryl containing the same or different 1 to 3 heteroatoms selected from the group consisting of nitrogen, oxygen and sulfur atoms, or (ii) pyridine ring substituted by 1 to 2 groups selected from the group consisting of (a) a halogen atom and (b) a group of formula: -CONR^{a}R^{b}, R^{a} and R^{b} are the same or different and each hydrogen, alkyl, monohydroxyalkyl or alkoxyalkyl, or both R^{a} and R^{b} combine each other together with the adjacent nitrogen atom to form 4 to 6-membered nitrogen-containing aliphatic heterocycle which may further contain heteroatoms selected from oxygen and sulfur atoms and may be optionally substituted by 1 to 2 hydroxyl, R¹ is a) acyl group of R¹¹ OCO-, b) 5 to 6-membered heteroaryl which contains the same or different 1 to 4 heteroatoms selected from nitrogen, sulfur and oxygen atoms and is substituted by a halogen atom, alkyl, alkyl substituted by 1 to 3 halogen atoms, cyanoalkyl, cycloalkyl, alkoxy optionally substituted by 1 to 3 halogen atoms, alkoxycarbonyl or a group of formula: - CONR^{c}R^{d}, or c) nitrogen-containing 6-membered heteroaryl-alkyl.

3. The compound as claimed in claim 1, wherein E is a group of formula: -O-, Ring A is a benzene ring substituted by 1 to 3 groups selected from a) a halogen atom, b) cyano, c) alkylsulfonyl, d) a group of formula: -CONR^{a}R^{b} and e) 5 to 6-membered heteroaryl containing 1 to 4 nitrogen atoms, R^{a} and R^{b} are the same or different and each hydrogen or alkyl, R¹ is a) acyl group of R¹¹OCO- or b) 5 to 6-membered heteroaryl which contains 1 to 3 heteroatoms selected from nitrogen and oxygen atoms and is substituted by alkyl.

4. The compound as claimed in claim 1, wherein E is a group of formula: -C(=O)-, Ring A is a benzene ring substituted by 1 to 3 groups selected from a) a halogen atom and b) alkylsulfonyl, R¹ is acyl group of R¹¹OCO-.

5. The compound as claimed in claim 1, wherein E is a group of formula: -CH(OH)-, Ring A is a benzene ring substituted by 1 to 3 groups selected from a) a halogen atom and b) alkylsulfonyl, R¹ is acyl group of R¹¹OCO-.

6. The compound as claimed in claim 1, wherein E is a group of formula: -CF₂-, Ring A is a benzene ring substituted by 1 to 3 groups selected from a) a halogen atom and b) alkylsulfonyl, R¹ is acyl group of R¹¹OCO-.

7. The compound as claimed in claim 1, wherein E is a group of formula: -NH- or -O-, Ring A is a benzene ring substituted by 1 to 3 groups selected from a) a halogen atom, b) cyano, c) alkylsulfonyl, d) a group of formula: -CONR^{a}R^{b}, wherein R^{a} and R^{b} are the same or different and each hydrogen, alkyl or monohydroxyalkyl, or both R^{a} and R^{b} combine each other together with the adjacent nitrogen atom to form 5 to 6-membered aliphatic nitrogen-containing heterocycle in which the heterocycle may further contain sulfur atom as heteroatoms and may be optionally substituted by 1 to 2 hydroxyl, and e) 5 to 6-membered heteroaryl which contains the same or different 1 to 3 heteroatoms selected from the group consisting of nitrogen, oxygen and sulfur atoms, R¹ is a) alkoxycarbonyl or b) 5 to 6-membered heteroaryl which contains 1 to 3 heteroatoms selected from nitrogen and oxygen atoms and is substituted by a halogen atom, alkyl, dihalogenoalkyl, trihalogenoalkyl, cycloalkyl, alkoxy or dihalogenoalkoxy, and R² is a halogen atom.

8. The compound as claimed in claim 7, wherein E is a group of formula: -NH-.

9. A compound of the following formula [I-A]: 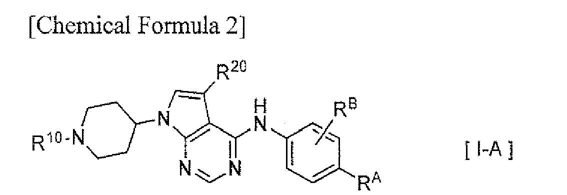 wherein R^{A} is a) a group of -CONR^{e}R^{f} wherein R^{e} and R^{f} are the same or different and each hydrogen, alkyl or monohydroxyalkyl or both combine each other together with the adjacent nitrogen atom to form 5 to 6-membered aliphatic nitrogen-containing heterocycle which may further contain sulfur atom as heteroatoms and may be optionally substituted by 1 to 2 hydroxyl, or b) 5-membered heteroaryl containing 1 to 3 nitrogen atoms as heteroatoms, R^{B} is a halogen atom, R¹⁰ is a) alkoxycarbonyl or b) 5 to 6-membered heteroaryl which contains 1 to 3 heteroatoms selected from nitrogen and oxygen atoms and is substituted by a halogen atom, alkyl, cycloalkyl, trihalogenoalkyl or alkoxy, R²⁰ is a halogen atom, or a pharmaceutically acceptable salt thereof.

10. A compound selected from the group consisting of:
4-[7-[1-(5-ethylpyrimidin-2-yl)piperidin-4-yl]-5-fluoro-7H-pyrrolo[2,3-d]pyrimnidin-4-ylamino]-3-fluoro-N,N-dimethylbenzamide;
4-[7-[1-(5-ethylpyrimidin-2-yl)piperidin-4-yl]-5-fluoro-7H-pyrrolo[2,3-d]pyrimidin-4-ylamino]-3-chloro-N,N-dimethylbenzamide;
4-[7-[1-(5-ethylpyrimidin-2-yl)piperidin-4-yl]-5-fluoro-7H-pyrrolo[2,3-d]pyrimidin-4-ylamino]-3-fluoro-N-(2-hydroxyethyl)-N-methylbenzamide;
[4-[7-[1-(5-ethylpyrimidin-2-yl)piperidin-4-yl]-5-fluoro-7H-pyrrolo[2,3-d]pyrimidn-4-ylamino]-3-fluorophenyl]((R)-3-hydroxypyrrolidin-1-yl)methanone;
[4-[7-[1-(5-ethylpyrimidin-2-yl)piperidin-4-yl]-5-fluoro-7H-pyrrolo[2,3-d]pyrimidin-4-ylamino]-3-fluorophenyl]((S)-3-hydroxypyrrolidin-1-yl)methanone;
4-[7-[1-(5-ethylpyrimidn-2-yl)piperidn-4-yl]-5-fluoro-7H-pyrrolo[2,3-d]pyrimidin-4-ylamino]-3-fluoro-N-(3-hydroxypropyl)-N-methylbenzamide;
3-fluoro-4-[5-fluoro-7-[1-(5-isopropyl-[1,2,4]oxadiazol-3-yl)piperidn-4-yl]-7H-pyrrolo[2,3-d]pyrimidin-4-ylamino]-N-(2-hydroxyethyl)-N-methylbenzamide;
[4-[7-[1-(5-propylpyrimidin-2-yl)piperidin-4-yl]-5-fluoro-7H-pyrrolo[2,3-d]pyrimidin-4-ylamino]-3-fluorophenyl]((R)-3-hydroxypyrrolidin-1-yl)methanone;
4-[7-[1-(5-isopropylpyrimidin-2-yl)piperidin-4-yl]-5-fluoro-7H-pyrrolo[2,3-d]pyrimidin-4-ylamino]-3-fluoro-N-(2-hydroxyethyl)-N-methylbenzamide;
[4-[7-[[1-(5-cyclopropylpyrimidin-2-yl)piperidn-4-yl]-5-fluoro-7H-pyrrolo[2,3-d]pyrimidin-4-ylam.ino]-3-fluorophenyl]((R)-3-hydroxypyrrolidin-1-yl)methanone;
4-[7-[1-(5-cyclopropylpyrimidin-2-yl)piperidin-4-yl]-5-fluoro-7H-pyrrolo[2,3-d]pyrimidin-4-ylamino]-3-fluoro-N-(2-hydroxyethyl)-N-methylbenzamide;
[4-[7-[1-(5-isopropylpyrimidin-2-yl)piperidin-4-yl]-5-fluoro-7H-pyrrolo[2,3-d]pyrimidin-4-ylamino]-3-fluorophenyl]((R)-3-hydroxypiperidin-1-yl)methanone;
4-[7-[1-(5-chloropyrimidin-2-yl)piperidin-4-yl]-5-fluoro-7H-pyrrolo[2,3-d]pyrimidin-4-ylamino]-3-fluoro-N-(2-hydroxyethyl)-N-methylbenzamide;
4-[7-[1-(5-isopropoxypyrimidin-2-yl)pipefidin-4-yl]-5-fluoro-7H-pyrrolo[2,3-d]pyrimidin-4-ylamino]-3-fluoro-N-(2-hydroxyethyl)-N-methylbenzamide;
4-[7-[1-(5-isopropoxypyrimidin-2-yl)piperidin-4-yl]-5-fluoro-7H-pyrrolo[2,3-d]pyrimidin-4-ylamino]-3-fluoro-N-(3-hydroxypropyl)-N-methylbenzamide;
3-fluoro-4-[5-fluoro-7-[1-(3-isopropyl-[1,2,4]oxadiazol-5-yl)piperidn-4-yl]-7H-pyrrolo[2,3-d]pyrimidin-4-ylamino]-phenyl]-pyrrolidin-1-yl-methanone;
isopropyl 4-[5-fluoro-4-[2-fluoro-4-(pyrrolidine-1-carbonyl)-phenylamino]-pyrrolo[2,3-d]pyrimidin-7-yl]piperidine-1-carboxylate;
[3-fluoro-4-[5-fluoro-7-[1-(5-trifluoromethyl-[1,2,4]oxadiazol-3-yl)piperidin-4-yl]-7H-pyrrolo[2,3-d]pyrimidin-4-ylamino]-phenyl]((R)-3-hydroxypyrrolidin-1-yl)methanone; and
[4-[7-[1-(5-ethylpyrimidin-2-yl)piperidin-4-yl]-5-fluoro-7H-pyrrolo[2,3-d]pyrimidin-4-ylamino]-3-chlorophenyl]((R)-3-hydroxypyrrolidn-1-yl)methanone, or a pharmaceutically acceptable salt thereof.

11. A pharmaceutical composition, comprising as the active ingredient the compound as claimed in any one of claims 1 to 10 or a pharmaceutically acceptable salt thereof.

12. The pharmaceutical composition as claimed in claim 11, for the prevention or treatment of metabolic disease or cardiovascular disease which can be treated by the activation of GPR119.

13. The pharmaceutical composition as claimed in claim 12, wherein the metabolic disease is obesity, hyperglycemia, diabetes and/or diabetes complication, metabolic syndrome, glucose intolerance, hyperinsulinemia, hyperlipidemia, hypercholesterolemia, hypertriglyceridemia or abnormal lipid metabolism.

14. The pharmaceutical composition as claimed in claim 12, wherein the cardiovascular disease is arterial sclerosis, hypertension, coronary disease or cardiac infarction.

15. A GPR119 activity modulator, comprising as the active ingredient the compound of any one of claims 1 to 10 or a pharmacologically acceptable salt thereof.
